(19)

Europäisches
Patentamt
European
Patent Office
Office européen
des brevets

(11) **EP 2 508 867 A1**

(12) **EUROPEAN PATENT APPLICATION**

(43) Date of publication:
**10.10.2012 Bulletin 2012/41**

(21) Application number: **12173753.0**

(22) Date of filing: **23.06.2006**

(51) Int Cl.:
**G01N 15/06** (2006.01)   **G01N 33/00** (2006.01)
**G01N 33/48** (2006.01)   **G01N 35/00** (2006.01)
**B01L 3/00** (2006.01)

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **24.06.2005 US 693613 P**
**10.11.2005 US 736082 P**

(62) Document number(s) of the earlier application(s) in
accordance with Art. 76 EPC:
**06785495.0 / 1 899 450**

(71) Applicant: **Board of Regents, The University of
Texas System
Austin, TX 78701 (US)**

(72) Inventors:
• **McDevitt, John T.
Austin, TX Texas 78725 (US)**

• **Ballard, Karri
Pflugerville, TX Texas 78660 (US)**
• **Christodoulides, Nicolaos J.
Austin, TX Texas 78749-4250 (US)**
• **Floriano, Pierre N.
Austin, TX Texas 78749 (US)**
• **Simmons, Glennon W.
Austin, TX Texas 78741 (US)**

(74) Representative: **Dehmel, Albrecht
Dehmel & Bettenhausen
Patentanwälte
Herzogspitalstrasse 11
80331 München (DE)**

Remarks:
This application was filed on 27-06-2012 as a
divisional application to the application mentioned
under INID code 62.

(54) **Systems and methods including self-contained cartridges with detection systems and fluid delivery systems**

(57)     The present invention relates to a method of obtaining a measurement of CD4+ cells, the method comprising the following steps:
(a) applying a fluid sample comprising a mixed population of cells to a microsieve such that at least some of the cells in the fluid sample are retained by the microsieve, wherein at least a portion of the mixed population of cells are white blood cells;
(b) applying a first detectable label to the cells retained on the microsieve, wherein the first detectable label is adapted to identify CD4+ cells, wherein the CD4+ cells are detectable at a first wavelength of light;
(c) applying one or more additional detectable labels to the cells retained on the microsieve, wherein the additional detectable labels are adapted to identify one or more of T cells, NK cells, B cells, or combinations thereof that are present on the microsieve, such that the cells identified by one or more of the additional detectable labels are detectable at one or more wavelengths of light that are different from the first wavelength of light;
(d) obtaining a first image of the retained cells at the first wavelength of light;
(e) obtaining a second image of the retained cells at one or more wavelengths of light that are different from the first wavelength of light; and
(f) obtaining a measurement of CD4+ cells.

EP 2 508 867 A1

FIG. 1

**EP 2 508 867 A1**

**Description**

1. Field of the Invention

The present invention relates to one or more systems, one or more methods, and one or more apparatuses for the detection of analytes. More particularly, the invention relates to the development of an analyte detection system capable of discriminating mixtures of analytes, toxins, cells and/or bacteria in medical, food/beverage, and/or environmental solutions.

2. Brief Description of the Related Art

The development of smart sensors capable of discriminating different analytes, toxins, and bacteria has become increasingly important for clinical, environmental, health and safety, remote sensing, military, food/beverage, and chemical processing applications. Some sensors have been fashioned for single analyte detection. Other sensors are capable of solution phase multi-analyte detection. Latex agglutination tests ("LATs") are used to detect many different types of analytes in clinical analyses. LATs employ colloidal polymer microspheres to determine the presence (or absence) of analytes. Commercially available LATs for more than 60 analytes are used routinely for the detection of infectious diseases, illegal drugs, and pregnancies. LATs generally operate on the principle of agglutination of latex particles (e.g., colloidal polymer microspheres). LATs are set up such that agglutination occurs when antibody-derivatized latex particles become effectively "cross-linked" by a foreign antigen, resulting in the attachment of the particle to, or the inability of the particle to pass through, a filter. The cross-linked latex particles are then detected colorimetrically upon removal of the antigen carrying solution.

More recently, "taste chip" sensors have been employed that are capable of discriminating mixtures of analytes, toxins, and/or bacteria in medical, food/beverage, and environmental solutions. Certain sensors of this type are described in US 20020197622 to McDevitt et al. The detection and analysis of white blood cells is widely used in medical diagnosis. For example, analysis of the number of CD4 lymphocytes in blood may be used to determine various diseases or conditions in a patient. Low CD4 counts have been associated with patients having a compromised immune system. Patients having a compromised immune system may be infected with HIV, parasitic infections, tuberculosis, and/or conditions resulting from physical or mental stress. Measuring white blood cells, subsets thereof, or other blood cell types in bodily fluids (e.g., blood from humans or other species) has proven to be beneficial for the diagnosis, treatment, and/or monitoring of various diseases such as malaria, syphilis, and mononucleosis.

New methods and systems capable of detecting and discriminating white blood cells (e.g., lymphocytes) and other components in blood for health and safety, environmental, homeland defense, military, diagnostic, food/beverage, and chemical processing applications are desired. Methods and systems that facilitate rapid screening of blood components, to be used as a trigger for more specific and confirmatory testing, are also desired.

**SUMMARY OF INVENTION**

In various embodiments, systems, methods, and apparatuses to analyze one or more samples containing one or more analytes are described. Samples may be fluid samples. In some embodiments, an analyte detection system is capable of detecting one or more analytes in solution. In some embodiments, the analytes include white blood cells. The analyte detection system may include a cartridge.

In some embodiments, the cartridge includes one or more collection regions, one or more fluid delivery systems, one or more channels, one or more reagent regions, one or more reservoirs, one or more detection regions, or combinations thereof. The detection region may include one or more detection systems. In some embodiments, one or more collection regions, one or more detection systems, one or more fluid delivery systems, one or more channels, one or more reagent regions, and one or more reservoirs are: coupled to or at least partially positioned in or on the cartridge. In some embodiments, one or more collection regions, one or more detection systems, one or more fluid delivery systems, one or more channels, one or more reagent regions, and one or more reservoirs are at least partially contained in a body of the cartridge. In some embodiments, a body of the cartridge includes a plurality of coupled layers.

In some embodiments, the body of the cartridge includes openings. The openings may be configured to receive one or more components used to facilitate analyte detection. One or more channels may couple the openings together. In some embodiments, one or more collection regions, one or more detection systems, one or more fluid packages, or combinations thereof are at least partially placed in one or more of the openings.

The collection region of a cartridge may receive a fluid and/or sample. In some embodiments, a collection region may include a cover.

Detection systems may include microsieve-based detection systems and/or particle-based detection systems. The detection systems are configured to interact with at least a portion of a sample to allow detection of an analyte.

In some embodiments, a microsieve of a microsieve-based detection system, when one or more samples are applied to the microsieve, at least partially retains desired matter in or on the microsieve. In some embodiments, one or more viewing windows are optically coupled to the microsieve, the viewing window being configured to allow one or more detectors to view at least a portion of the microsieve.

In some embodiments, an anti-reflective material is coupled to the microsieve. In some embodiments, the anti-reflective material is configured to inhibit reflection of light applied to the sample on the microsieve, such that an image of at least a portion of the sample in or on the microsieve is improved with respect to an image taken of the sample in the absence of the anti-reflective material.

One or more fluid delivery systems are configured to transport fluid from a first location to a second location in or on the cartridge. In some embodiments, a fluid delivery system includes one or more fluid packages and/or one or more syringes configured to facilitate transport of fluid. In some embodiments, at least one fluid delivery package is configured to create a partial vacuum, when opened, in one or more of the channels during use.

Fluid may be transported through one or more channels of the cartridge from a first location to a second location in or on the cartridge. Channels may couple one or more collection regions, one or more detection regions, and one or more fluid delivery systems. In some embodiments, one or more channels are part of a fluid delivery system. In some embodiments, a shape or elevation of at least a portion of one or more of the channels is configured such that fluids flowing in or through one or more channels during use are selectively directed through the one or more channels. In some embodiments, an inside material of or on at least a portion of one or more of the channels is configured to selectively direct fluids flowing in or through one or more of the channels during use.

Valves positioned in one or more of the channels and/or a cartridge may control fluid flow. In some embodiments, one or more pinch valves are coupled to one or more of the channels and/or the cartridge. In some embodiments, applying pressure to one or more pinch valves positioned in or on the cartridge controls fluid flow through one or more of the channels.

One or more vents may be coupled to one or more of the channels. In some embodiments, gas is released from the cartridge through vents as fluid flows through one or more of the channels.

One or more reagent regions may include a reagent pad, at least a portion of a channel, and at least a portion of a surface of a cartridge. At least one of the reagent regions may deliver one or more reagents from the reagent region to a fluid flowing through one or more of the reagent regions during use. In some embodiments, flowing fluid through one or more reagent regions allows at least one reagent from at least one of the reagent regions to be delivered to a sample.

In some embodiments, one or more reservoirs include an overflow reservoir, a waste reservoir, or a both an overflow reservoir and a waste reservoir. The overflow reservoir and/or waste reservoir may collect excess sample or fluid. In some embodiments, a portion of fluid or sample in a cartridge is directed to an overflow reservoir of the cartridge.

In some embodiments, an analyte detection system includes one or more cartridge control systems. The cartridge control systems include one or more control analytes. The cartridge control systems may be coupled to one or more of the detection systems. One or more of the detection systems are configured to interact with at least a portion of the control analytes to allow detection of the control analyte.

The present invention includes methods for evaluating a sample using the components, systems and embodiments discussed above. Moreover, it includes methods of detecting analytes in a sample, determining the number of analytes in a sample, measuring the number of analytes in sample, evaluating a sample to determine if it contains one or more analytes, evaluating a sample for the presence of one or more analytes, and/or evaluating a sample for the absence of one or more analytes.

In some embodiments, the analyte involved in methods of the invention includes one or more organic molecules, inorganic molecules, ions, cells, bacteria, viruses, fungi, and parasites. In certain embodiments, the organic molecule is a polypeptide, polynucleotide, lipid, and/or carbohydrate.

In further embodiments, the polypeptide is a cell surface polypeptide, while in other embodiments, it is a secreted polypeptide or a polypeptide located inside a cell, including inside its nucleus. In some cases, a cell surface polypeptide is a receptor or it may be a CD antigen.

Additional embodiments involve an analyte that is a polynucleotide, which may be deoxyribonucleic acid (DNA) or a ribonucleic acid (RNA). DNA may be genomic or mitochondrial. RNA may also be genomic (for instance, from a virus), mRNA, tRNA, rRNA, miRNA, or siRNA. A polynucleotide may be double-stranded, triple-stranded, or single stranded.

In certain embodiments, a polypeptide, polynucleotide may be chemically modified. RNA and polypeptides may be post-transcriptionally modified. In particular embodiments an analyte may be glycosylated, phosphorylated, amidated, acetylated, ubiquitinated, sentrinized, methylated, and/or modified by a lipid (e.g., farnesylated, etc.) or sugar molecule. Such modifications may also pertain to analytes that are lipids (e.g., glycolipids) or carbohydrates.

Methods of the invention may involve, in some aspects, a binding agent that binds to the analyte. In certain embodiments, the binding agent is all or part (binding portion) of an antibody, receptor, or ligand. Antibodies, in certain embodiments, are polyclonal, monoclonal, or single chain, Moreover, binding agents may be chimeric polypeptides, meaning they contain all or part of more than one polypeptide.

A method of detecting analytes in a sample may include applying a sample on or to a collection region of a cartridge. In some embodiments, a cover is positioned over the collection region.

In some embodiments, a sample flows from a collection region to one or more detection systems, and one or more images of at least a portion the detection system are provided. In some embodiments, fluid flows through channels to and from reagent regions with the assistance of one or more fluid delivery systems. Fluids from reagent regions may flow in and/or through one or more detection systems.

A method for detection of an analyte in a sample may include applying at least a portion of a sample to a detection system of a cartridge and allowing at least a portion of the sample to interact with the detection system.

A method of detecting analytes in a fluid includes applying one or more control analytes from one or more control analyte reservoirs in or on an analyte detection cartridge to one or more detection systems in or on the analyte detection cartridge and assessing a result from the detection system to determine whether the analyte detection cartridge is working within a selected range.

A method for detecting white blood cells in a sample includes applying a sample (e.g., allowing the sample to flow) to one or more microsieves in or on a cartridge and applying one or more detectable labels from one or more detectable label locations in or on a cartridge to a least a portion of the white blood cells retained in or on the one or more microsieves.

A method for assessing $CD4^+$ cells in a sample includes: applying a sample to a microsieve in or on a cartridge; applying a first detectable label to material retained on a microsieve to label any $CD4^+$ cells; applying one or more additional detectable labels to the material retained on the microsieve to label any T cells, NK cells, and B cells retained on the microsieve; providing a first image of the $CD4^+$ cells; providing a second image of the retained material; and assessing a number of $CD4^+$ cells by assessing the number of labeled cells in the first image that are also depicted as labeled cells in the second image. In some embodiments, a ratio of $CD4^+$ cells is assessed by comparing the number of labeled cells that are depicted in both the first image and the second image to the number of labeled cells that are depicted in the second image.

A method of analyzing a blood sample includes introducing the blood sample into an analyte detection system, assessing a number of at least a portion of the cellular components collected by a microsieve, and assessing an amount and/or identity of proteins that interact with the particle-based detection system.

An apparatus for analyzing a blood sample includes a microsieve-based detection system and a particle-based detection system. The microsieve-based detection system includes a microsieve. The microsieve collects at least a portion of a first analyte in the blood sample as the blood sample passes through the microsieve during use. The particle-based detection system includes one or more particles. At least some of the particles are configured to interact with a second analyte in the blood sample during use.

Any embodiment discussed with respect to one aspect of the invention applies to other aspects of the invention as well. For instance, any embodiment discussed in the context of a system of the invention may be implemented in the context of any method of the invention, and vice versa. Moreover, any embodiment discussed in the context of a sub-genus or species may be applied in the context of any other sub-genus or species discussed herein. For instance, any embodiment discussing certain CD antigens may be implemented in the context of any other CD antigen discussed herein.

The embodiments in the Example section are understood to be embodiments of the invention that are applicable to all aspects of the invention.

The use of the term "or" in the emb.s is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard deviation of error for the device or method being employed to determine the value. Following long-standing patent law, the words "a" and "an," when used in conjunction with the word "comprising" in the emb.s or specification, denotes one or more, unless specifically noted.

**BRIEF DESCRIPTION OF THE DRAWINGS**

FIG. 1 depicts a perspective view of an embodiment of a cartridge.
FIG. 2 depicts an exploded view of an embodiment of a cartridge.
FIG. 3 depicts an embodiment of a cartridge with channels.
FIG. 4 depicts an embodiment of a cartridge with fluid delivery systems with fluid packages.
FIG. 5 depicts an alternate embodiment of a cartridge.
FIG. 6 depicts a cross-sectional view of a valve.

FIG. 7 depicts a top view of an actuation system coupled to a cartridge.

FIG. 8 depicts a cross-sectional side view of an embodiment of a fluid package.

FIG. 9 depicts a top view of an embodiment of the fluid package depicted in FIG. 8.

FIG. 10 depicts a cross-sectional side view of an embodiment of a fluid package positioned in a cartridge.

FIG. 11 depicts a cross-sectional side view of rupturing the fluid package depicted in FIG. 10.

FIG. 12 depicts a cross-sectional side view of an embodiment of a fluid package in a cartridge.

FIG. 13 depicts a perspective view of a fluid delivery system that includes a fluid package and a reservoir.

FIG. 14 depicts an exploded view of the fluid delivery system depicted in FIG. 13.

FIG. 15 depicts a perspective cut-away view of the fluid delivery system depicted in FIG. 13.

FIG. 16 depicts a cut-away perspective view of the bottom of the fluid delivery system depicted in FIG. 13.

FIG. 17 depicts a top view of a seal offset from a top layer opening of the fluid delivery system depicted in FIG. 13.

FIG. 18 depicts a perspective view of an alternate embodiment of a fluid delivery system.

FIG. 19 depicts an exploded view of the fluid delivery system depicted in FIG. 18.

FIG. 20 depicts an embodiment of a fluid package used in the fluid delivery system depicted in FIGS. 18 and 19.

FIG. 21 depicts an exploded view of an alternate embodiment of a fluid delivery system.

FIGS. 22A and 22B depict embodiments of fluid packages.

FIG. 23 depicts an embodiment of a fluid bulb for fluid delivery.

FIG. 24 depicts an alternate embodiment of fluid bulb for fluid delivery.

FIGS. 25A- 25H depict embodiments of syringes.

FIG. 26A depicts an embodiment of syringes coupled to a cartridge.

FIG. 26B depicts a magnified view of a portion of the cartridge depicted in FIG. 26A.

FIG. 27 depicts an embodiment of a cartridge that includes more than one detection system.

FIG. 28 depicts a top view of an embodiment of a multi-functional cartridge.

FIG. 29 depicts an exploded view of the multi-functional cartridge depicted in FIG. 28.

FIG. 30 depicts an exploded view of a microsieve-based detection system.

FIG. 31 depicts an exploded view of a microsieve-based detection system with directed fluid flow.

FIG. 32 depicts a top view of a support with a parallelogram shape.

FIG. 33 depicts a top view of a support with a euclidian shape.

FIG. 34 depicts a cross-sectional view of an embodiment of an open area of a support.

FIG. 35 depicts a cross-sectional view of an alternate embodiment of an open area of a support.

FIG. 36 depicts a schematic diagram of a cartridge positioned in an optical platform with two light sources.

FIG. 37 depicts a schematic diagram of a cartridge positioned in an alternate optical platform with two light sources.

FIG. 38 depicts a schematic diagram of a cartridge positioned in an optical platform with a single light source.

FIGS. 39A and 39B depict schematic diagrams of a cartridge positioned in an optical platform that includes movable filters.

FIGS. 40A- 40C depict representations of images of cells obtained using an analyte detection system.

FIGS. 41A-41D depict representations of images of cells obtained using an analyte detection system.

## DETAILED DESCRIPTION

In various embodiments, an analyte detection system may be used to analyze a sample containing one or more analytes. Samples may be fluid samples, e.g., a liquid sample or a gaseous sample. The analyte detection system may, in some embodiments, generate patterns that are diagnostic for both the individual analytes and mixtures of the analytes. In some embodiments, the analyte detection system includes a microsieve capable of retaining a portion of the sample. The analyte detection system, in certain embodiments, may include a plurality of chemically sensitive particles, formed in an ordered array, capable of simultaneously detecting different analytes. In some embodiments, the analyte detection system may be formed using a microfabrication process, thus allowing the analyte detection system to be economically manufactured.

Terms used herein are as follows:

"Analyte" refers one or more substances undergoing analysis. Examples of analytes include, but are not limited to, organic molecules, inorganic molecules, ions, cells, bacteria, viruses, fungi, and parasites.

"Anti-reflective" refers to inhibiting the reflection of light at predetermined wavelengths.

"Binding agent" refers to a compound capable of binding an analyte. In certain embodiments, the binding agent is also coupled with a detectable label.

"Cartridge" refers to a removable unit designed to be placed in a larger unit.

"Couple" refers to either a direct connection or an indirect connection (e.g., one or more intervening connections) between one or more objects or components.

"CRP" refers to C-reactive protein.

"Detectable label" refers to one or more compounds capable of detection. A detectable label may be coupled directly or indirectly to a substance, which consequently allows for detection of the substance through detection of the label. In certain embodiments, a detectable label is directly and noncovalently coupled to a substance.

"Detection system" refers to one or more systems designed to interact with one or more analytes during use.

"Detector" refers to one or more devices capable of detecting the presence of one or more analytes, one or more signals produced by one or more of the analytes, one or more signals produced by the interaction of one or more analytes with a detection system, or combinations thereof. Signals produced by analytes include, but are not limited to, spectroscopic signals. Spectroscopic signals include, but are not limited to, signals produced at wavelengths detectable in an ultraviolet ("UV") region, a visible region, and/or an infrared ("IR") region of the electromagnetic spectrum. Spectroscopic signals also include signals produced by fluorescence of an analyte or a component of a detection system. The detector may be, but is not limited to an optical digital camera, a charge-coupled device ("CCD"), a complementary-metal-oxide-semiconductor ("CMOS") detector, or a spectrophotometer capable of detecting UV, visible and/or IR wavelengths of light.

"Fluid" refers to a substance in a gas phase or a liquid phase.

"Fluid delivery system" refers to one or more systems or devices capable of causing a fluid to flow. A fluid delivery system may include a plurality of components. Components that may be part of a fluid delivery system include, but are not limited to, reservoirs containing fluids, flexible chambers containing fluids, channels, reagent reservoirs, buffer reservoirs, fluid packages, syringes, fluid bulbs, and/or pipettes.

"Fluid package" refers to a pouch, a container, or a chamber configured to contain one or more fluids.

"Fluorophore" refers to one or more fluorescent molecules or compounds.

"Hydrophilic material" refers to one or more materials having the ability to hydrogen bond with water. Hydrophilic materials may have an affinity for aqueous solutions.

"Hydrophobic material" refers to one or more materials ineffective at hydrogen bonding with water. Hydrophobic materials may lack an affinity for water.

To "label" refers to coupling one or more detectable labels to a substance to allow detection of the substance, for example, by altering the absorbance and/or fluorescence of the substance. In some embodiments, a substance is labelled via a binding agent that is coupled to a detectable label.

"LED" refers to light emitting diode.

"Microsieve" refers to one or more sheets or layers capable of retaining matter from a fluid and/or a sample including, but not limited to, membranes, microchips, and various monoliths.

"Positioned in" or "positioned on" refers to placing one or more substances, elements, or members at least partially or fully in or on an opening or a surface of a substrate.

"RBCs" refer to red blood cells.

"WBCs" refer to white blood cells including granulocytes (eosinophils and basophils), lymphocytes (B cells, T Cell, natural killer cells), monocytes, and macrophages.

Analytes in a sample may be analyzed using an analyte detection system. In some embodiments, a sample is a biological sample. A biological sample is a sample that contains biological material such as all or part of an organ, tissue, cells, nucleic acids, proteins, or other such macromolecules and substances. The sample may include sputum, serum, blood, plasma, spinal fluid, semen, lymphatic fluid, urine, stool, pleural effusion, ascites, a tissue sample, tissue biopsy, cell swab, or a combination thereof. In other embodiments of the invention, a sample may include cells that are from lung, skin, muscle, liver, renal, colon, prostate, breast, brain, bladder, small intestine, large intestine, cervix, stomach, pancreas, testes, ovaries, bone, marrow, or spine. In some embodiments, the sample is a whole blood, plasma or serum sample.

In certain embodiments, the biological sample is described as a bodily fluid (e.g., saliva, urine, and/or blood or any fluid discussed above). The blood sample may be human blood or blood from another species. A blood sample may be obtained from any species. Collection of a sample may be accomplished by making an incision (e.g., a prick or cut) in a part of (e.g., a finger) a human body to allow collection of the sample (e.g., blood) or it may be obtained by lavage, smear, or swab of an area on or in a subject or patient.

The sample may be collected with a tube, a fluid bulb, a syringe, or a pipette. The sample may be directly transferred to a cartridge of the analyte detection system (e.g., transferred to a collection region of the cartridge) using the fluid bulb, the syringe, or the pipette. For example, a sample is collected in a tube or a vacuum tube and transferred to a collection region of the cartridge. In some embodiments, a cartridge may include a conduit coupled to a disposable tip. The disposable tip may puncture a portion of a human body and draw a sample into the cartridge. In some embodiments, a sample is reacted with one or more reagents and/or one or more detectable labels in a sample collection device prior to being transferred to the cartridge. In some embodiments, a sample may be applied directly to the cartridge from the sample source (e.g., from the human body).

The sample may be diluted before it is applied to a cartridge or after it is applied to the cartridge. For example, a sample of whole blood may be diluted before applying it to a collection region of a cartridge. It may be diluted with a solution that is pharmacologically acceptable and/or that preserves the integrity of one or more components of the sample. In certain embodiments, the solution is buffered and/or contains one or more preservatives.

The use of a sample collection device may limit health and safety risks associated with exposure to pathogens present in a sample. Using a sample collection device may allow a sample to be directly transported from the source to the instrument without further handling.

It is further contemplated that a sample after it has been collected or solution it is exposed to or diluted with may be chilled or frozen. Such methods may be implemented to help preserve the biological sample and are well known to those of skill in the art.

Sample collection devices are described by McDevitt et al. in US Nos. 11/022,176 entitled "INTEGRATION OF FLUIDS AND REAGENTS INTO SELF-CONTAINED CARTRIDGES CONTAINING SENSOR ELEMENTS"; 11/020,443 entitled "INTEGRATION OF FLUIDS AND REAGENTS INTO SELF-CONTAINED CARTRIDGES CONTAINING SENSOR ELEMENTS"; 11/020,442 entitled "INTEGRATION OF FLUIDS AND REAGENTS INTO SELF-CONTAINED CARTRIDGES CONTAINING SENSOR ELEMENTS"; 11/022,365 "INTEGRATION OF FLUIDS AND REAGENTS INTO SELF-CONTAINED CARTRIDGES CONTAINING SENSOR ELEMENTS"; 11/021,123 entitled "PARTICLE ON MEMBRANE ASSAY SYSTEM"; and 11/022,219 entitled "MEMBRANE ASSAY SYSTEM INCLUDING PRELOADED PARTICLES".

The analyte detection system may include, but is not limited to, one or more apparatuses (e.g., cartridges), an optical platform, one or more detectors, an analyzer, or combinations thereof. The cartridge may include, but is not limited to, one or more sample collection devices, one or more collection regions, one or more fluid delivery systems, one or more reagent regions, one or more detection regions, or combinations thereof. The detection regions may include one or more detection systems. The optical platform may include, but is not limited to, one or more detectors, one or more light sources, one or more lenses, one or more filters, one or more dichroic mirrors, one or more shutters, one or more actuators, or combinations thereof. The analyzer may include one or more computer systems and/or one or more microscopes. In some embodiments, the analyte detection system includes a housing. The housing may include the optical platform and accept one or more cartridges.

In some embodiments, a cartridge is self-contained and/or disposable. The cartridge may include all reagents and/or fluids necessary for the detection of one or more analytes in a sample. Use of a self-contained and/or disposable cartridge may limit environmental and health risks associated with handling of fluids and/or samples.

In some embodiments, one or more barcodes or other readable indicia are positioned on a cartridge. A detector and/or an analyzer of the analyte detection system may read the barcode to determine hardware and/or software specifications for the assay. Using barcodes or other readable indicia may allow a user to analyze a plurality of cartridges using the same analyte detection system. When the cartridge is positioned in an analyte detection system, a reader in the analyte detection system may read the indicia on the cartridge and set the system specifications for the indicated test. A barcode or indicia may represent information such as, but not limited to, the type of analyte to be detected, light sources to be used, process time, sample number or code, detector settings, or combinations thereof. System specifications include, but are not limited to: light sources, filters, or lenses; detector settings; fluid delivery system activation order and/or times; actuator activation sequence; actuator positions; exposure times; sample incubation time; and/or which detectable labels used in the cartridge.

A cartridge may include indicia that tell a user which direction to insert the cartridge into the analyte detection system. For example, a body of a cartridge may include a notch, arrow and/or a barcode to indicate the proper placement of the cartridge.

In some embodiments, a cartridge includes a viability indicator (e.g., a temperature indicator). A viability indicator may indicate if the cartridge has been exposed to conditions that could damage the cartridge and/or one or more chemical components of the cartridge. For example, a temperature-based indicator indicates if the cartridge has been exposed to temperatures that are above or below a temperature that would cause decomposition of one or more chemical components in the cartridge. An analyte detection system may read the viability indicator to determine if the cartridge is viable prior to initiating any detection operations with the cartridge.

The cartridge may be formed of an inert or biodegradable material. The cartridge may be sized to allow the cartridge to be hand-held and/or portable. A cartridge has dimensions which allows insertion of the cartridge into a housing of an analyte detection system.

In some embodiments, a cartridge body is substantially planar. A width (w) of the cartridge may range from: about 30 mm to about 100 mm, about 40 mm to about 90 mm, about 50 mm to about 80 mm, or about 60 mm to about 70 mm. A length (1) of the cartridge may range from: about 50 mm to about 300 mm, about 60 mm to about 200 mm, about 70 mm to about 150 mm, or about 80 mm to about 100 mm. A height (h) of the cartridge may range from: about 1 mm to about 30 mm, about 5 mm to 20 mm, or about 10 mm to 15 mm. In some embodiments, a cartridge is about 35 mm wide and 125 mm long, about 35 mm wide and about 75 mm long, or about 50 mm wide and about 75 mm long.

A cartridge body may include one or more openings designed to receive one or more components used to facilitate analyte detection. Components include, but are not limited to, a collection region (e.g., a sample collection pad), a fluid delivery system (e.g., a fluid package, a fluid bulb, a syringe, and/or a fluid reservoir), reservoirs, a microsieve-based detection system, a particle-based detection system, or combinations thereof. Components may be positioned in one or more cartridge body openings. Adhesive may be used to secure the components to the cartridge body and/or within the openings formed in the cartridge body. Openings may be designed to receive a specific component. For example, an opening designed for a collection region may have a specific shape that is different than an opening designed for a fluid delivery system component. In some embodiments, openings for components have the same dimensions and/or shape. In some embodiments, a cartridge body includes channels coupling one or more of the openings in or on the cartridge. The ability to customize the cartridge body may allow many different configurations of a cartridge to be produced.

In some embodiments, collection regions, fluid delivery systems, reagent regions, and/or detection systems may be coupled to the cartridge, directly attached to the cartridge, positioned in the cartridge, or positioned on the cartridge. Collection regions, reagent regions, fluid delivery systems, and/or detection systems may be incorporated in a cartridge body. Collection regions, reagent regions, fluid delivery systems, and detection systems may be at least partially contained in a cartridge body.

In some embodiments, components are at least partially positioned in different layers of a body of the cartridge. For example, the collection region may be positioned in a different layer of the cartridge than the detection system. In some embodiments, reservoirs (e.g., sample collection reservoir, overflow reservoir, and/or waste reservoir) are positioned in the same layer or in more than one layer. For example, a waste reservoir is positioned in a different layer of the cartridge than the detection system and/or the collection region. Fluid delivery systems may be positioned in one or more of the same layers of the cartridge body. The cartridge body may include one or more layers that retain fluid in at least a portion of the cartridge. In some embodiments, a top layer includes an opening coupled to the sample collection region to allow application of the sample to the sample collection region, while retaining fluid in other portions of the cartridge.

A cartridge with one or more openings may have a variety of configurations. For example, a cartridge includes a detection region and one or more openings. A collection region, one or more fluid delivery systems and/or one or more reservoirs may be positioned in the openings of the cartridge. Alternatively, a cartridge includes a sample collection region and one or more openings. A detection system and/or at least one fluid package may be positioned in the openings. In another example, a cartridge includes one or more fluid delivery systems and one or more openings. Components (e.g., a sample collection region and/or detection system) may be inserted the openings.

The collection region of a cartridge may be coupled to, positioned in, or positioned on the cartridge. The collection region may collect sample from a sample collection device. In some embodiments, fluids other than sample are collected in the collection region.

The collection region may include a channel positioned at a predetermined height with respect to the region. When a sample is deposited in the collection region, excess sample may flow through the channel into an overflow reservoir and/or waste reservoir of the cartridge. The height at which the channel is positioned with respect to the region may determine the amount of sample collected in the collection region. Inclusion of the channel may inhibit sample from spilling out of a collection region. Inhibiting a sample from overflowing from the collection region may lessen exposure to potentially hazardous material. In some embodiments, a collection region of a cartridge includes a sample collection reservoir and/or a collection pad.

One or more fluid delivery systems may be coupled to, positioned in, positioned on, or embedded in a cartridge. In some embodiments, fluid delivery systems containing appropriate reagents, buffers, and/or detectable labels are positioned in openings in the cartridge body. Some fluid delivery systems are described in US 5,096,660 to Lauks et al. ; US 5,837,199 to Dumschat; and US 6,010,463 to Lauks et al.. In some embodiments, gravity, elevation changes within the cartridge and/or channel, capillary forces, or combinations thereof, promote and/or facilitate the transport of fluids in the cartridge. In certain embodiments, pumps and/or vacuum sources are coupled to the cartridge, in addition to fluid delivery systems, to assist fluid flow.

A cartridge may include one or more reagent regions. One or more reagent regions may be at least partially coupled to or positioned in or on the cartridge. In some embodiments, a reagent region includes one or more reagents, detectable labels, and/or buffers that are disposed on one or more reagent pads, one or more surfaces of a channel, one or more surfaces of a cartridge, or a combination of these locations.

The reagents, detectable labels, and/or buffers may be in solid, liquid, or gaseous state. In some embodiments, a reagent region includes one or more reagents, detectable labels, and/or buffers entrained in a dissolvable material. When a fluid contacts (e.g., passes over) the dissolvable material, at least a portion of the reagents, detectable labels, and/or buffers entrained in the dissolvable material may be released. For example, dried reagents may be positioned in or on a dissolvable material. Fluid passing over the dissolvable material may at least partially dissolve the dissolvable material and partially reconstitute the dried reagents.

A reagent pad of a reagent region may be, but is not limited to, a filter, absorbent pad, or container. Reagents including, but not limited to, detectable labels, anti-coagulants, and/or particles may be positioned in the reagent pad and/or on a surface of the reagent pad such that fluid passing over and/or through the reagent pad may at least partially reconstitute the reagents contained in or on the pad. In some embodiments, a reagent pad performs as a filter to remove large particles from a fluid flowing through the reagent pad.

In certain embodiments, dried reagents, lyophilized reagents, and/or solid reagents are positioned in or coated on a surface of a reagent region (e.g., surfaces of a channel or a cartridge).

As fluid passes through the channel, reagents and/or detectable labels may be reconstituted. Dried, lyophilized, or solid reagents may be more stable. Using reagents that are dried, lyophilized, or are in a solid state may increase the shelf life of a cartridge. Using dried, lyophilized, or solid reagents may allow a cartridge to be stored at ambient temperatures rather than in a controlled temperature storage unit (e.g., a refrigerator).

In some embodiments, one or more reservoirs (e.g., one or more overflow reservoirs and/or one or more waste reservoirs) are coupled to or positioned in or on a cartridge. The overflow reservoir and/or waste reservoir may collect excess fluid (e.g., excess sample, excess detectable label, and/or excess reagents).

The overflow reservoir is, in some embodiments, coupled to a collection region, a detection region, a detection system, and/or one or more reagent regions. The overflow reservoir may be coupled to the collection region to allow an excess amount of sample (e.g., an amount of sample greater than a predetermined amount of sample) applied to the collection region to flow to the overflow reservoir. Coupling the overflow reservoir to the collection region may allow a predetermined amount of sample to be collected. Coupling the overflow reservoir to the collection region may inhibit overfilling the collection region. Inhibiting overfilling of the collection region may inhibit release of potentially hazardous material.

In some embodiments, the overflow reservoir is coupled to the detection region and/or detection system to inhibit excess fluid from entering the detection region and/or detection system. If excess fluid enters the detection region and/or detection system, matter and/or particles retained in or on the detection region and/or detection system may be disturbed. Disturbance of retained matter and/or particles may cause the matter and/or the particles to leave the detection region and/or detection system. For example, if too much fluid flows onto a microsieve positioned in or on a detection region and/or a detection system, matter retained on a surface of the microsieve may be disturbed and a portion of the retained matter may flow into proximate channels or regions before analysis.

One or more detection regions of a cartridge include areas of the cartridge where one or more detection systems are located. Detection systems may be coupled to, positioned in, or positioned on, a cartridge. It should be understood, that various combinations of detection systems in, on, or coupled to the cartridge are possible. For example, one detection system may be positioned in an opening of the cartridge, while another detection system is positioned on the cartridge. A detection system may be coupled to the cartridge, while another detection system is positioned in the cartridge. Detection systems may include, but are not limited to, a microsieve-based detection system and/or a particle-based detection system. A detection system is selected based on the analyte of interest. For example, a microsieve-based detection system may be selected to assess cells or bacteria in a fluid and/or sample.

Detection systems and methods of using the detection systems are described herein and in US Nos. 11/020,442; 11/022,365; 11/021,123; and 11/022,219, and in the following US patents, patent applications: US patents 6,908,770; 6,680,206; 6,602,702; 6,589,779; 6,649,403; and 6,713,298; US applications 20020160363; 20040029259; 20030064422; 20030186228; 20040053322; 20050136548; 20050164320; 20050214863; 09/616,731 entitled "METHOD AND APPARATUS FOR THE DELIVERY OF SAMPLES TO A CHEMICAL SENSOR ARRAY" filed July 14, 2000; 10/522,499 entitled "CAPTURE AND DETECTION OF MICROBES BY MEMBRANE METHODS" filed January 24, 2005; 10/470,646 entitled "CAPTURE AND DETECTION OF MICROBES BY MEMBRANE METHODS" filed January 24, 2005; 10/522,926 entitled "CAPTURE AND DETECTION OF MICROBES BY MEMBRANE METHODS" filed January 24, 2005; 10/544,864 entitled "MICROCHIP-BASED SYSTEM FOR HIV DIAGNOSTICS" filed August 5, 2005; and 10/544,954 entitled "MULTI-SHELL MICROSPHERES WITH INTEGRATED CHROMATOGRAPHIC AND DETECTION LAYERS FOR USE IN ARRAY SENSORS" filed August 8, 2005.

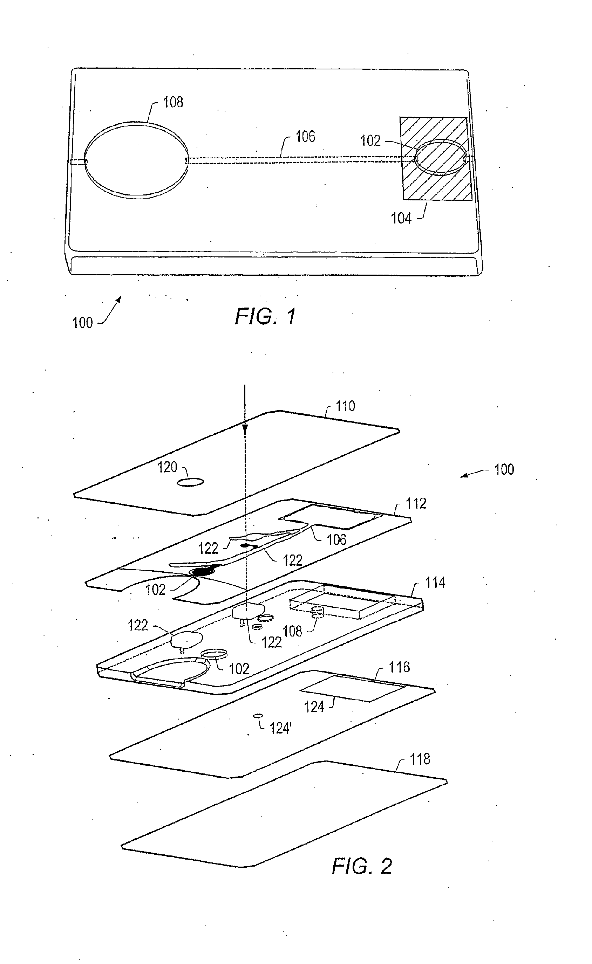

FIG. 1 depicts a perspective top view of an embodiment of a cartridge. Cartridge 100 includes collection region 102, cover 104, fluid channel 106, and detection region 108. A sample may be placed in collection region 102. In some embodiments, other fluids (e.g., reagents and/or buffer solutions) may be added to the collection region and mixed with the sample. The sample may flow from collection region 102 through channel 106 to detection region 108.

Collection region 102 may include, but is not limited to, a reservoir, a pad, a channel, a capillary, a tube, a vacuum collection tube (e.g., a Vacutainer® commercially available from Becton Dickinson Company Franklin Parks, New Jersey, USA), an opening in the cartridge, or combinations thereof. In some embodiments, collection region 102 is a portion of the detection system on which sample is applied. In certain embodiments, collection region 102 is a microsieve.

In some embodiments, cover 104 is removable. Cover 104 may cover a portion or all of collection region 102.

The use of cover 104 is optional. Cover 104 may be positioned manually or automatically. In some embodiments, an analyte detection system automatically positions the cover over the collection region after the cartridge is positioned in the system. Cover 104 may be a flap coupled to the cartridge that may be moved to uncover or cover the collection region, as desired. Cover 104 may be moved in a sliding motion to cover or uncover the sample collection region. Cover 104 may seal the sample collection region and inhibit contaminants from entering the sample collection region. In some embodiments, the cover may include an opening. Cover 104 may at least partially contain biological waste and/or hazardous materials in the cartridge. In some embodiments, the cover may substantially contain biological waste and/or hazardous materials in the cartridge. In some embodiments, the cover may include an adhesive strip, an absorbent pad, a non-removable plug, a swinging window, a film, a nylon filter, or combinations thereof.

In some embodiments, it may be desirable to inhibit sample from flowing towards a detection region. For example, after a predetermined amount of sample flows towards the detection region, it may be desirable to inhibit more of the sample from flowing towards the detection region. Cover 104 may inhibit undesired additional sample from flowing towards a detection region by absorbing sample from the collection region.

In some embodiments, a cartridge and/or a body of the cartridge are formed of one or more layers. In certain embodiments, one or more layers seal one or more components in the cartridge. Layers may be coupled, sealed, welded or bonded together to form the cartridge. The cartridge body may include more than three layers or more than four layers coupled together.

FIG. 2 depicts an exploded view of an embodiment of a cartridge formed of layers. Cartridge 100 may include top layer 110, channel layer, 112, sample layer 114, reservoir layer 116, and support layer 118.

Top layer 110 may include opening 120. Samples may be deposited on sample layer 114 through opening 120. Top layer 110 and support layer 118 may seal cartridge 100. In some embodiments, each of the layers may include more than one layer coupled together.

In some embodiments, sample layer 114 may be positioned between one or more channel layers 112 and reservoir layer 116. Sample layer 114 may include collection region 102 and/or one or more reagent regions 122. Collection region 102, one or more fluid channels 106, and/or reagent regions 122 may be at least partially contained in more than one layer of a body of cartridge 100.

Reservoir layer 116 may be positioned proximate sample layer 114. Reservoir layer 116 may collect sample and/or one or more fluids passing through the cartridge during use. Reservoir layer 116 may include one or more reservoirs 124, 124' that collect sample and/or fluid passing through the cartridge (e.g., an overflow reservoir and/or a waste reservoir). In some embodiments, reservoirs may extend through more than one layer. For example, reservoir 124 may extend through channel layer 112 and sample layer 114. Reservoir 124 may be accessible from one or more selected layers.

Channel layer 112 may be positioned above sample layer 114. In some embodiments, an additional channel layer may be positioned below a reservoir layer. In certain embodiments, one or more channel layers may be positioned above or below one or more sample layers and/or one or more reservoir layers. Channel layer 112 may include a plurality of channels coupling various components of cartridge 100. One or more channels 106 may allow fluid to flow within a layer and/or from one layer to another layer.

In some embodiments, channels are positioned in more than one layer of a cartridge. Positioning a channel in more than one layer may change an elevation of the channel enough to enhance sample and/or fluid to flow in and/or through the cartridge. Channels may be coupled to two or more locations in or on a cartridge. In some embodiments, one or more channels are a part of one or more fluid delivery systems.

In some embodiments, one or more channels couple a collection region to a detection region, one or more detection systems, and/or one or more overflow reservoirs. Channels may couple one or more fluid delivery systems to a collection region, a detection region, one or more detection systems, and/or one or more reservoirs (e.g., overflow reservoirs and/or one or more waste reservoirs). Two or more channels may be coupled such that they intersect and fluid may optionally flow through more than one channel; however, the size, the elevation, and/or the inside material of the intersecting channel may affect through which channel a fluid flows and/or selectively direct fluid flow. Channels or a portion of a channel may promote and/or inhibit fluid flow in or on the cartridge.

The size and/or the elevation of a channel may selectively direct fluid flow through the channel. Fluid may flow preferentially through a channel that is wider before flowing through narrower channels, thus the fluid may be inhibited from flowing in channels narrower than other proximate channels. In some embodiments, a portion of the fluid may flow into a narrower channel, while another portion of the fluid flows into a channel wider than the narrow channel. In some embodiments, some channels may have a cross-sectional area larger than a cross-sectional area of other channels of a cartridge. Fluid may flow through the channel with the largest cross-sectional areas prior to flowing through channels with smaller cross-sectional areas. Fluid may be inhibited from flowing into a channel with a smaller cross-sectional area than proximate channels.

In some embodiments, channels include changes in elevation. A portion of a channel may be positioned in a first layer of a cartridge while another portion may be positioned in a second and/or third layer of a cartridge. A channel may have an elevation gradient along an axis parallel to fluid flow. Changes in elevation of a channel may promote, facilitate, and/or increase fluid flow in or on a channel. Elevation changes may inhibit fluid from flowing into a channel.

In some embodiments, channel properties may affect fluid flow in the channels. At least a portion of a channel may selectively direct fluid flow in one or more channels. A channel may be formed of a material, coated with a material, or have material deposited on a surface of a portion of the channel that selectively directs fluid flow in one or more channels. For example, a channel may be at least partially formed of a hydrophilic material to promote aqueous fluid flow in the channel. A channel may be at least partially formed of a hydrophobic material to inhibit aqueous fluid flow in the channel. In some embodiments, portions of a channel may be coated with a hydrophilic and/or hydrophobic material. A material that defines at least a part of the channel may be hydrophilic. A channel coupled to a collection region may be partially made of a hydrophilic material to allow an aqueous sample to be drawn from the collection region. In some embodiments, channels partially made of a hydrophobic material may inhibit aqueous fluid flow, thus a waste region may not be needed.

Channels may be formed of or coated with a hydrophilic material and/or the elevation of the channel may promote fluid flow towards the detection region. In some embodiments, a channel releasing fluid into the detection regions and/or a detection system is at least partially formed of a hydrophilic material to promote laminar flow in the channel. Laminar flow of fluid in the channel may cause matter (e.g., particles, cells, or other matter) in the sample to be evenly distributed across a surface of a portion of a detection system (e.g., a microsieve of a microsieve-based detection system).

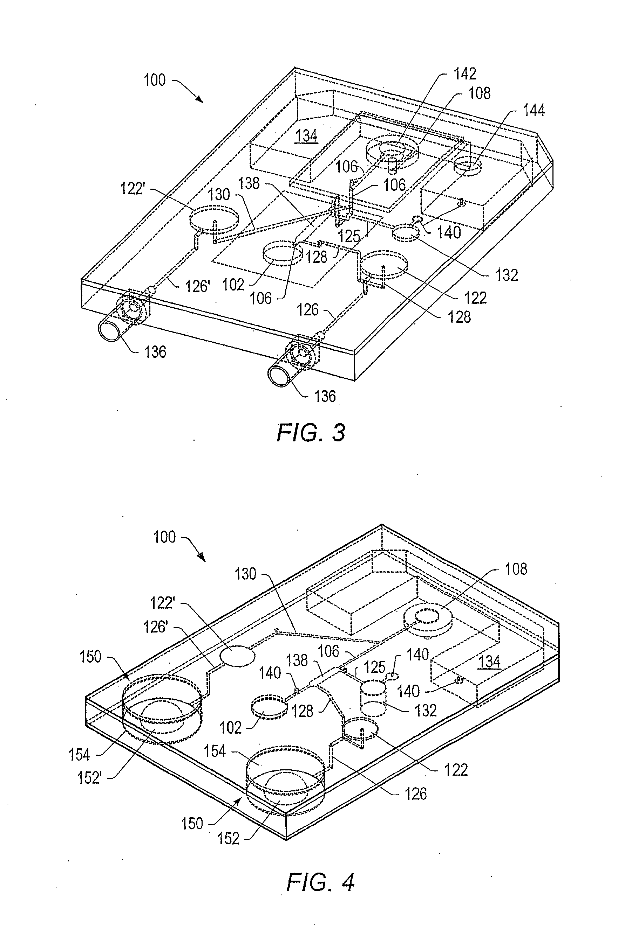

FIG. 3 depicts an embodiment of a cartridge that includes channels having different elevations. Cartridge 100 may include channels 106, 125, 126, 126', 128, 130, collection region 102, reagent regions 122, 122', detection region 108, overflow reservoir 132, waste reservoir 134, and connectors 136.

Sample deposited in collection region 102 may flow through channel 106 toward detection region 108. Channel 106 includes metered volume portion 138. Metered volume portion 138 may be a part of the channel. In some embodiments, the metered volume portion is coupled to the channel and/or the collection region. Metered volume portion 138 may have a diameter greater than diameters of proximate channels. After metered volume portion 138 fills with a predetermined amount of fluid (e.g. sample), fluid may flow towards overflow reservoir 132 through channel 125. In some embodiments, substantially all of an introduced sample flows out of collection region 102 and into metered volume portion 138. Excess introduced sample will enter overflow reservoir 132 if the metered volume portion is filled. In some embodiments, overflow region 132 is coupled a waste region. Overflow reservoir 132 may include vent 140 to promote fluid flow.

Vents 140 may be positioned proximate one or more collection regions, metered volume portions, waste reservoirs, overflow reservoirs, and/or in channels coupled to fluid delivery systems. Vents 140 may allow gas to escape from cartridge 100 as fluids pass through or on one or more channels or layers of the cartridge. Vents 140 may inhibit pressure in the channels of the cartridge from becoming greater than ambient pressure. Vents 140 may promote fluid flow in cartridge 100 by releasing pressure associated with the passage of pressurized fluids through the channels. Vents 140 may facilitate laminar flow of fluids in cartridge 100. In some embodiments, vents 140 are designed to inhibit release of fluids through the vent. It may be desirable to limit release of liquids while allowing gas to escape from the cartridge to contain fluids (hazardous reagents and/or biological samples) in the cartridge.

Elevation of channel 106 may vary. Different elevations in the channel may inhibit fluid from flowing into detection region 108. A sample may be pushed towards a detection system rather than allowing a sample to flow towards a detection system without applied pressure. The sample may be allowed to mix and interact with reagents prior to entering the detection region. Channel 106 may promote fluid flow towards the overflow region. In certain embodiments, channel 106 may have a negative pressure so that fluids are drawn into the channel. In some embodiments, a channel coupled to a collection region may have a negative pressure to draw the sample into the channel.

Fluid may be delivered to cartridge 100 from one or more fluid delivery systems connected to the cartridge by connectors 136. Connectors may include, but are not limited to, tubing, quick-disconnect connections, and/or locking connectors. It should be understood that any of the various embodiments of fluid delivery systems described herein and/or other fluid delivery systems known in the art may be incorporated with or coupled to cartridge 100.

Fluid enters channel 126, 126' and passes through and/or over reagent regions 122, 122'. In some embodiments, the reagent region may be a pad, a channel, a depression, and/or a reservoir. In some embodiments, the reagent regions may be a part of the fluid delivery system. In some embodiments, the reagent regions are channels, which are a part of a fluid delivery system. Reagent regions 122, 122' may include dried reagents, anti-coagulants, and/or detectable labels. In some embodiments, reagents, buffers, binding agents and/or detectable labels are dried on or in a pad positioned in or on reagent regions 122, 122'. In some embodiments, reagents and/or detectable labels on and/or in the reagent regions 122, 122' may be reconstituted by fluid passing over and/or the through reagent region.

Channels 128, 130 may allow fluid to flow from the bottom surface of reagent regions 122, 122' to other components of cartridge 100. In some embodiments, inlet and outlet channels to the reagent regions may be positioned such that fluid is forced to pass through, on, and/or over reagent regions 122, 122'. In some embodiments, additional fluid delivery systems are positioned proximate the reagent regions.

The fluid delivery system may be controlled to allow fluid to pass across reagent regions 122, 122' enter metered volume portion 138, and then enter detection region 108. Reagents and/or detectable labels in reagent region 122 may be reconstituted by the fluid from the fluid delivery system and may react with the sample. The fluid delivery system may be controlled to allow a predetermined volume of fluid to pass through detection region 108. In some embodiments, fluid from a fluid delivery system may enter detection system of the cartridge while the sample incubates on the detection system and/or a microsieve of the detection system.

Channels 128, 130 intersect channel 106, and fluid and/or sample from these channels enters detection region 108 via channel 106. Detection region 108 may include viewing window 142. Viewing window 142 may be optically coupled to a detection system. Viewing window 142 may be positioned in or on the cartridge. Viewing window 142 may be a portion of a detection system. For example, viewing window 142 may be a portion of a top member of a microsieve-based detection system located in the detection region. Viewing window 142 may be made of a material transparent to light (e.g., visible or ultraviolet). Viewing window 142 may include or be composed of a material that allows certain wavelengths of light to pass or filters out certain wavelengths of light. Viewing window 142 may include a lens that assists in focusing light onto a portion of a detection system and/or onto one or more detectors. A detector may capture an image or light from a detection system through viewing window 142.

Detection region 108 and/or a detection system in the detection region may be coupled to waste reservoir 134 to allow fluids flowing through the detection system to pass into the waste region. Waste reservoir 134 may be, but is not limited to, a container, a depression, or an opening. Waste reservoir 134 may be coupled to, positioned in, or positioned on the cartridge. By allowing fluids to flow towards a waste reservoir after use, all fluids in the cartridge may be contained within the cartridge. A contained waste reservoir may minimize health and safety hazards due to handling of and/or exposure to the sample and/or fluid.

Waste reservoir 134 may include cap 144. Cap 144 allows a user to remove fluids from the waste region and/or release pressure from the waste region. All or a portion of cap 144 may be removable. Cap 144 may have a variety of shapes and/or configurations (e.g., round, oval, threaded, and/or tapered). A cap on a waste reservoir may allow the waste reservoir to be pressurized so that fluids may be drawn towards the detection system and/or waste reservoir. A waste reservoir may include vent 140 to inhibit a build up of pressure in the waste reservoir.

In some embodiments, a fluid delivery system facilitates transport of fluid or sample from one location to another location in or on the cartridge (e.g., from a first location in or on the cartridge to a second and/or third location in or on the cartridge). In certain embodiments, a fluid delivery system delivers reagents, buffer, and/or detectable labels to the detection system. The fluid delivery system may facilitate transport of at least a portion of the sample from the sample collection region to the detection system. The fluid delivery system may couple and/or include channels that couple different regions of the cartridge. For example, the fluid delivery system couples the collection region to the detection system. The fluid delivery system may couple the collection region to the detection system and/or to one or more waste reservoirs. In some embodiments, the fluid delivery system includes channels that couple components of the analyte detection system to each other.

FIG. 4 depicts an embodiment of cartridge 100 with two fluid delivery systems. Cartridge 100 may include channels 106, 125, 126, 126', 128, 130, collection region 102, reagent regions 122, 122', detection region 108, overflow reservoir 132, waste reservoir 134, fluid delivery systems 150, and vents 140. Fluid delivery systems 150 may include fluid packages 152, 152' and reservoirs 154. During use, sample may be released from collection region 102, flow through channel 106 and enter detection region 108. Channel 106 may include metered volume portion 138.

Fluid packages 152, 152' may be opened at predetermined times (e.g., simultaneously or one at a time) to allow fluid (e.g., a buffer, reagent solution or detectable labels) in the fluid package to be released into channels 126, 126'. The released fluids may pass over reagent regions 122, 122' before a portion of sample in channel 106 reaches detection system 108. For example, a portion of a sample is placed in collection region 102 and released into channel 106 after fluid from one of fluid packages 152 flows over and/or through reagent region 122. Alternatively, a portion of sample is placed in collection region 102 and released into channel 106 prior to and/or simultaneously as fluid from one of fluid packages 152, 152' flows over and/or through reagent regions 122, 122'. In some embodiments, substantially the entire excess introduced sample flows out of collection region 102 and into overflow reservoir 132 via channel 125. A size of overflow reservoir 132 may allow fluid from more than one assay to be collected during use.

Fluid from reagent region 122 flows through channel 128, enters into channel 106, and then enters detection region 108. In some embodiments, channel 128 and channel 106 are the same channel. Channel 126' delivers and/or directs fluid flow from fluid delivery system 150, across and/or through the reagent region 122', and into channel 130. Channel 130, which intersects channel 106, directs fluid from reagent region 122' to a position in channel 106 such that the reagents from reagent region 122' mix with a portion of the sample and/or fluid in channel 106 prior to entering detection region 108. In some embodiments, channel 130 is a part of channel 106.

Vents 140 may be positioned in or on cartridge 100. Vents 140 may be a part of waste reservoir 134 or a part of one or more channels (e.g., channel 106).

In some embodiments, valves are used to control fluid flow through the cartridge. Valves may be positioned on or in the cartridge. Valves may direct, control, and/or restrict fluid flow. Active or passive valves may be positioned in channels. Valves may include, but are not limited to, pinch valves, pressure valves, electromagnetic valves, and/or temperature valves.

In some embodiments, a temperature-controlled valve may be used. A temperature-controlled valve may include a fluid such as, but not limited to, water that is at least partially frozen in a channel to inhibit further fluid from passing through the channel. To open the valve, heat is applied to the frozen fluid to melt the fluid. A temperature-controlled valve includes, in some embodiments, a material that is a solid at room temperature (e.g., paraffin or wax). To open a channel, heat may be applied to the solid in the channel to melt the solid material.

In certain embodiments, a valve is hydraulically activated. In some embodiments, pressurized fluid (e.g., air or water) is used to open or close a valve. Pressure may be transferred via a gas or liquid in a channel to another location in the cartridge. The gas or liquid may be used to compress a drum and/or close a valve. In some embodiments, valves surrounding a portion of a channel having negative pressure inhibit equalizing the negative pressure until desired.

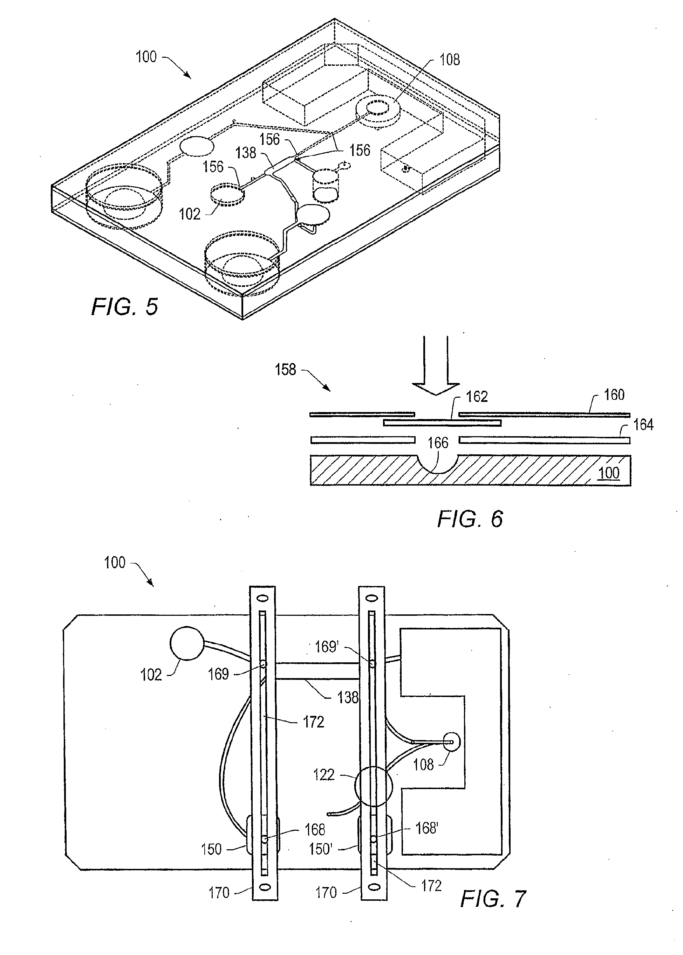

FIG. 5 depicts cartridge 100 (shown in FIG. 4) with valves 156. Valves 156 are positioned after collection region 102 and after metered volume portion 138. Valves 156 may be used to direct fluid flow from collection region 102 to detection region 108. Valves 156 may be positioned at various other locations in or on cartridge 100.

FIG. 6 depicts an embodiment of a pinch valve. Pinch valve 158 may include one or more layers 160, 162, 164 and channel 166. Layers 160, 162, 164 may be positioned over a surface of cartridge 100. In some embodiments, the layers are incorporated into the cartridge. Channel 166 may be an opening in cartridge 100.

Layer 162 may be coupled to layer 160 and layer 164. Surfaces of layers 160, 164 may be composed of materials including, but not limited to, thermal bond film, pressure sensitive adhesive, or other adhesive materials. Layer 162 may be adhered to at least one of layers 160, 164 (e.g., using a heat sealing process). In some embodiments, layer 164 forms a wall of channel 166. Layer 162 may be designed so that pressure applied to a surface of layer 162 causes the layer to deform (e.g., flex). Deformation of at least a portion of layer 162 may at least partially obstruct channel 166 as layer 162 is forced into channel 166 by the applied pressure. Layer 162 may be formed of any material that exhibits flexibility when pressure is applied to the layer (e.g., an elastomer).

Valves may be activated manually or automatically. In some embodiments, an analyte detection system automatically opens or closes the valves. Actuators may be coupled to the analyte detection system to open and/or close the valves. In some embodiments, an actuator is positioned above the cartridge to apply pressure to a valve through an opening in the cartridge. In some embodiments, an actuator is positioned below the cartridge to apply pressure to a valve through an opening in the cartridge. In some embodiments, actuators are designed to open fluid delivery systems or fluid packages. In some embodiments, a metered volume of a sample including particulate components (e.g., cellular components) may be defined within a cartridge by actuation of one or more valves (e.g., pinch valves).

In some embodiments, actuation is used to release liquids or gas from a fluid delivery system. Liquids and/or gas may be pressurized into or in the fluid delivery system. An actuated fluid delivery system may be actuated from a top surface, a bottom surface, and/or a side surface of the cartridge. For example, a cartridge may be loaded in a housing of an analyte detection system with actuators. Actuators are then automatically, semi-automatically, or manually aligned with actuation points of the cartridge. A cartridge positioning system may facilitate cartridge placement into a position such that actuation points are aligned with actuators. Actuation points may be positioned on top, bottom, and/or side surfaces of a cartridge. For example, when a cartridge is positioned in the housing of an analyte detection system, actuators may be positioned below the cartridge.

FIG. 7 depicts a perspective top view of a cartridge 100 with an actuator system. The actuator system may include actuators 168, 168', 169, 169' and structure 170. Structure 170 may be designed to move from one side of a cartridge 100 to another side of the cartridge along a surface of the cartridge to facilitate actuation of various valves and/or fluid delivery systems. Structures 170 may be positioned at various points on cartridge 100. As shown, structures 170 are positioned between collection region 102 and detection region 108. Structures 170 may include opening 172. In some embodiments, opening 172 is a track. Actuators 168, 168', 169, 169' may be positioned at various points on or in structure 170 or opening 172. Actuators 168, 168', 169, 169' may move along opening 172 in structure 170 as needed.

Actuators 168, 168' are positioned over fluid delivery systems 150, 150'. Actuation of fluid delivery system 150 by actuators 168 may force fluid to flow towards metered volume portion 138, e.g., by bursting a fluid package. Actuation of fluid delivery system 150' by actuator 168' may allow fluid to flow towards reagent region 122.

Actuators 169, 169' may be positioned over valves proximate metered volume portion 138. Actuation of one or more of the valves proximate meter volume portion 138 may allow a metered volume of sample to flow into and/ out of metered volume portion 138. For example, actuator 169 may open the valve between collection region 102 and metered volume portion 138 to allow a portion of a sample to flow into the metered volume portion. Actuator 169' may at least partially open the valve between metered volume portion 138 and detection region 108 to allow a portion of the sample to flow towards the detection region. Structure 170 may then be moved to a different location, as desired. In some embodiments, sample in a channel may be inhibited from flowing back towards a collection region by actuating a valve. In some embodiments, one or more actuators may be moved along an opening or a track of the structure until the actuator aligns with a valve. The actuator may then actuate the valve.

In some embodiments, fluid delivery systems include one or more fluid packages. A fluid package is a package that contains a fluid used by a fluid delivery system. Fluid packages may include liquids or gas under pressure. Fluid packages contain a fluid until the package is opened. Upon opening of the package, fluid in the fluid package may be at least partially released. A fluid package may contain a fluid until an activation pressure is applied to the fluid package. An activation pressure may be the pressure required to release at least a portion of the fluid from the fluid package. An activation pressure may be the pressure required to rupture the package of the fluid package. Upon application of an activation pressure to the fluid package, at least a portion of the fluid contained in the fluid package will be released. In some embodiments, a fluid package is activated (e.g., opened) by heat or by an electromagnetic signal.

In some embodiments, fluid packages contain liquids, such as one or more buffers (e.g., phosphate buffers), one or more solvents (e.g., water, methanol, ethanol, and/or THF), one or more reagents, one or more binding agents (e.g., antibodies), and/or one or more detectable labels. One or more detectable labels may be coupled to one or more binding agent. Positioning one or more liquids required for analysis in or on a cartridge may make the fluids more accessible during use and enhance usage of the cartridge. Pre-packaged liquids may limit exposure to the liquids resulting from selection and/or mixing of solutions during use. Pre-packaged liquids may reduce time of analysis from sample collection to analysis of the sample. Placing the liquids required for analysis in fluid packages may increase stability and/or shelf life of a cartridge that includes an actuated fluid delivery system. Additionally, fluid packages may allow the cartridge to be stored at room temperature rather than requiring refrigeration.

In some embodiments, a fluid package includes a solvent. The solvent in a fluid package may be released from the fluid package and flow over one or more reagent pads that include buffer chemicals, reagents, binding agents, and/or detectable labels. A cartridge including solvent-filled fluid packages and dried buffers, reagents, and/or detectable labels may increase the stability of the cartridge since dried buffers, reagents, and detectable labels may be more stable and/or may have a greater shelf life than aqueous solutions.

In some embodiments, a fluid package delivers air or another gas to the cartridge. Gas released from a fluid package may assist in transporting a fluid and/or a sample through and/or in the components and/or channels of the cartridge.

In certain embodiments, a fluid package is designed to be filled with fluid with substantially few or no air bubbles. A fluid package may be designed to inhibit release of air bubbles or gas within a fluid package into a cartridge channel or component during partial or full compression and/or actuation of the fluid delivery system.

In some embodiments, a fluid package is designed to release at least 80 percent of liquid or gas contained in the fluid package. A fluid package may include up to about 1 mL of fluid. In certain embodiments, a fluid package has a shelf life of at least 2 years and/or has a volume loss of less than 5 percent of the original volume during a 2-year period.

A fluid package may be, but is not limited to, a pouch, container, and/or chamber. The fluid package may be formed from plastic materials. Plastic material may allow the fluid package to deform and release fluid. Once the fluid is released, a plastic fluid package may not reform, thus inhibiting formation of at least a partial vacuum. Creation of at least a partial vacuum may draw fluids and/or gas back into the fluid package.

In some embodiments, a fluid package may be deformable in a controlled manner. The fluid package may be formed of a material that allows the fluid package to be deformed and/or compressed (e.g., elastomeric material). A deformable/compressible material may allow a fluid package to be transported, stored, and/or positioned without breakage.

A fluid package may be made of materials including, but not limited to, polyvinyl chloride (PVC), polyvinylidene chloride (PVDC), polyethylene (PE), rubber, polypropylene (PP), polyacrylonitrile (PAN), cyclic olefin copolymer (COC), polydimethylsiloxane (PDMS), fluoropolymer films, foil (e.g., aluminum foil or plastic foil), adhesive tapes, or combinations thereof.

In some embodiments, a fluid package may be formed of a first material and a second material, where a second material is designed to rupture or break before the first material when pressure is applied to the fluid package. In some embodiments, a wall of the fluid package may be formed of layers of polypropylene and cyclic olefin copolymer.

A fluid package may be formed of a material compatible with the fluid it is designed to contain. A fluid package may be formed of a material that will not leach into the fluid contained within the fluid package. In certain embodiments, a fluid package includes a layer that couples the fluid package to the cartridge. The layer may be formed of a material capable of bonding (e.g., adhesive material) to acrylic, plastics, and/or other materials used to form a cartridge body.

A wall of a fluid package may be designed to have a weak portion (e.g., a burst point). The weak wall portion may rupture when a predetermined amount of pressure is applied to the fluid package. Fluid may be released from a fluid package by applying sufficient pressure to the package to cause the weak wall portion to rupture. The location of the weakened wall portion may be aligned with or coupled to a channel and/or component opening. A fluid package may be designed with a burst point or point at which fluid is released of about 3 psi to about 7 psi (absolute pressures).

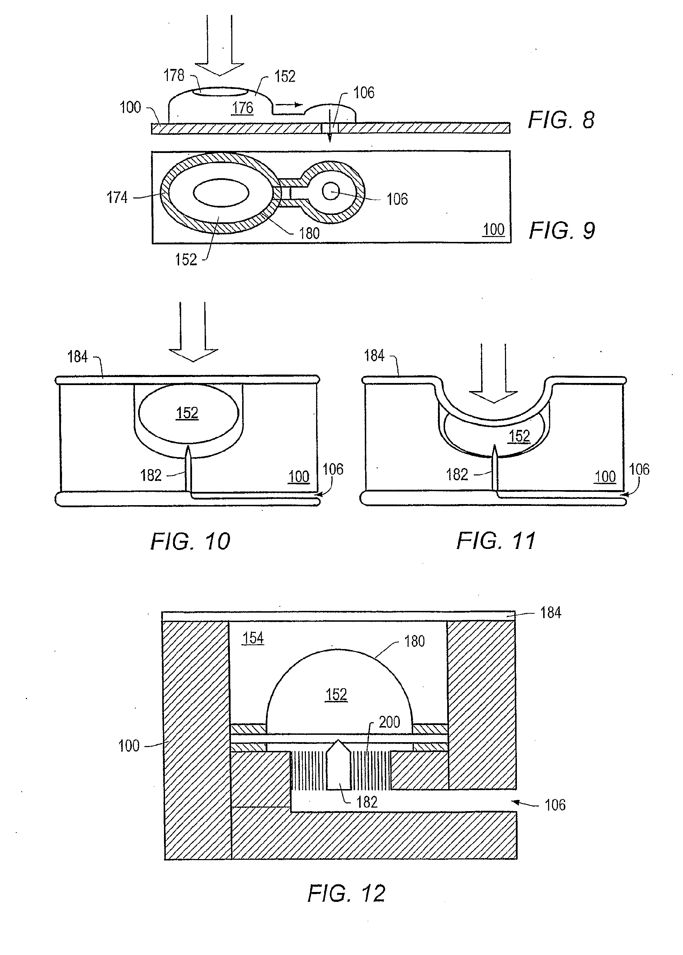

FIG. 8 depicts a side view of an embodiment of a fluid package. FIG. 9 depicts a top view of the embodiment of the fluid package depicted in FIG. 8. Fluid package 152 may be coupled to, at least partially positioned in, or at least partially positioned on cartridge 100. As depicted in FIG. 9, fluid package 152 may include layer 174. Layer 174 may be made of material (e.g., adhesive) that allows fluid package 152 to couple to cartridge 100. Fluid package 152 may be at least partially filled with liquid. Fluid package 152 may include liquid 176 and gas 178.

Examples of gas 178 are air, nitrogen, and/or argon. A portion of a wall of fluid package 152 may include a burst point. As pressure is applied to the fluid package, wall 180 of fluid package 152 may rupture at the burst point. Once fluid package 152 ruptures, fluid may be released from the fluid package into channel 106. The rigidity of fluid package 152 may be modified to accommodate various applications and/or storage or transport conditions. In some embodiments, fluid and/or air may be contained in the fluid package by a removable adhesive strip. Removal of the adhesive strip may allow fluid and/or air from the fluid package to be released from the fluid package.

In some embodiments, a cartridge includes a projection to rupture a portion of the fluid package. The projection may be needle shaped or any other shape capable of perforating a fluid package. The projection may be formed from any suitable material such as metal, plastic, and/or silicon. FIG. 10 depicts a side view of an embodiment of a fluid package positioned in a cartridge with a projection. Projection 182 may be positioned proximate to a surface of fluid package 152 and/or cartridge 100. Cover 184 may be positioned over fluid package 152. FIG. 11 depicts an embodiment of rupturing the fluid package depicted in FIG. 10. When pressure is applied to cover 184, the cover contacts the fluid package 152 causing the fluid package to contact projection 182. Projection 182 may rupture a portion of fluid package 152 causing fluids to be released in channel 106.

FIG. 12 depicts cross-sectional view of a fluid package positioned in cartridge 100. Fluid package 152 is positioned in opening 154 of cartridge 100. In some embodiments, fluid package is positioned on the cartridge. In some embodiments, one or more walls of the opening are capable of being deformed (e.g., the walls flex). Cover 184 may be positioned above opening 154. Cover 184 may be formed of an adhesive so that fluid package 152 is retained in opening 154. Projection 182 may be coupled to cartridge 100. Pressure applied to cover 184 may cause wall 180 of fluid package 152 to contact projection 182 and rupture. Fluid from fluid package 152 may be released into channel 106. Baffles 200 positioned proximate the bottom of opening 154 may assist in controlling flow rate of the fluid from fluid package 152.

In some embodiments, a fluid delivery system includes one or more fluid packages and a reservoir. The one or more fluid packages may be sealed and/or positioned in the reservoir. The reservoir may be coupled to, positioned in, or positioned on the cartridge.

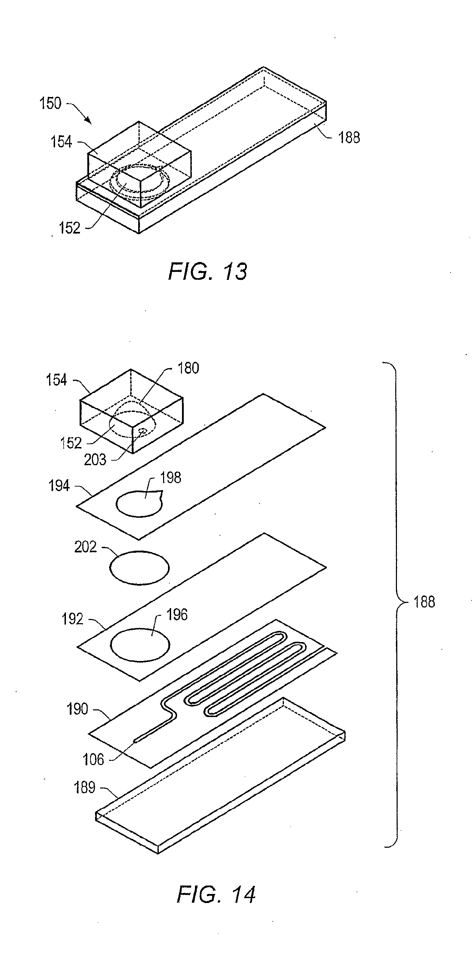

FIG. 13 is a perspective view of a fluid delivery system with a fluid package and a reservoir. Fluid delivery system 150 may include fluid package 152, reservoir 154, and support 188. In some embodiments, support 188 is part of a cartridge body. Portions of the fluid delivery system may be formed of several layers. In some embodiments, portions of the fluid delivery system may be formed of silicon resin, double-sided adhesive, thermo-bond film, and/or metal foil.

FIG. 14 depicts an exploded view of fluid delivery system 150 depicted in FIG. 13. Support 188 may include support layer 189, channel layer 190, middle layer 192, and top layer 194. Support layer 189 and/or middle layer 192 may assist in retaining fluids in channel layer 190. Support layer 189 may be a portion of a cartridge. Support layer 189 may be formed of metal, plastic, and/or glass. Channel layer 190 may be coupled to, or be a part of, support layer 189. Channel 106 of channel layer 190 directs fluid flow to a collection region and/or a detection region of the cartridge. Channel layer 190 may include reagent regions and/or have properties described herein. In some embodiments, the layers of fluid delivery system 150 may be the same as the layers in cartridge 100.

Middle layer 192 may be coupled to or be a part of channel layer 190. Portions of middle layer 192 may include coupling agents (e.g., adhesive or adhesive film) that couple the middle layer to channel layer 190. Middle layer 192 may include opening 196. Opening 196 may direct fluid into channel 106. Middle layer 192 may be coupled to top layer 194 using generally known coupling techniques (e.g., adhesive, pins, and/or screws).

Top layer 194 may seal or contain fluids in fluid package 152 and/or reservoir 154. Top layer 194 may include opening 198. Opening 198 may direct fluid from fluid package 152 and/or reservoir 154 to channel layer 190. Top layer 194 may include seal 202. Seal 202 may be positioned between middle layer 192 and top layer 194. Seal 202 may cover opening 198 of top layer 194. Seal 202 may seal fluid and/or gas in fluid package 152 and/or reservoir 154. Seal 202 may be formed from a variety of materials (e.g., thermo-bond film, and/or foil). Seal 202 may rupture when pressure is applied to fluid package 152 and/or reservoir 154. In some embodiments, seal 202 may be a part of top layer 194.

Top layer 194 may be coupled to or be a part of reservoir 154 using generally known coupling techniques. Reservoir 154 may include opening 203. Reservoir opening 203 may be aligned with top layer opening 198. Top layer 194 may coupled to or be a part of reservoir 154 and/or fluid package wall 180.

Fluid package 152 may be positioned in reservoir 154. A wall of fluid package 152 may be aligned with reservoir opening 203 and top layer opening 196. A portion of a wall of fluid package 152 includes a burst point to allow the fluid package to rupture when a predetermined amount of pressure is applied to the fluid package and/or reservoir 154. In some embodiment, the fluid package and the reservoir are one unit. In some embodiments, the reservoir does not include the fluid package.

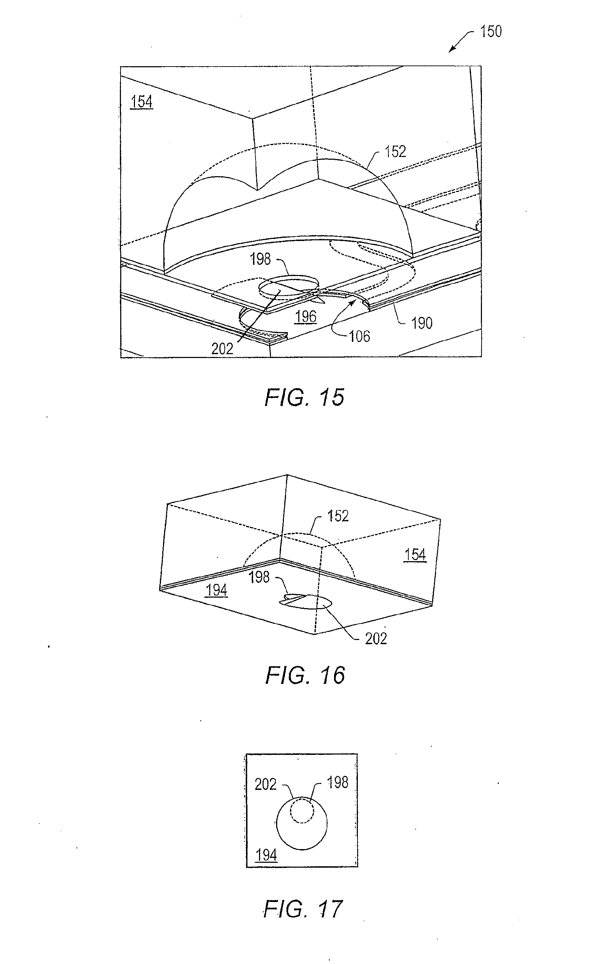

FIG. 15 depicts a perspective cut-away view of the reservoir of fluid delivery system 150 depicted in FIG. 13.

A diameter of top layer opening 198 and/or the reservoir opening may be less than, equal to, or greater than a diameter of middle layer opening 196. As depicted, seal 202 has been torn to allow fluid to flow to channel 106 in channel layer 190. A center of seal 202 may be directly aligned or offset with a center of top layer opening 198.

FIG. 16 depicts a cut-away perspective view of top layer 194 and reservoir 154 containing fluid package 152 as depicted in FIG. 13. FIG. 17 depicts a top view of fluid reservoir 154. As seen in FIG. 17, seal 202 is offset from top layer opening 198 in top layer 194. Offsetting seal 202 may facilitate the rupturing of the seal when a predetermined amount of pressure is applied to the fluid package and/or reservoir by creating a weak point in the seal.

A center of the seal may be offset from the center of the top layer opening by a distance ranging from about 0.2 mm to about 2 mm, about 0.3 mm to about 1.5 mm, or about 0.4 mm to about 1 mm. When the center of the seal is offset from the center of the top cover opening by about 0.25 mm, a burst point of the seal may rupture at a pressure of about 1 psi to at most 10 psi, from about 3 psi to about 8 psi, or from about 5 psi to about 7 psi. In contrast, the burst point of the seal may rupture at a pressure of greater than 10 psi when a center of the seal is aligned with the center of the top cover opening.

In some embodiments, the pressure required to rupture a fluid package is lowered by varying the materials used to create the seal, decreasing the surface area of the seal in a strategic location, decreasing the bonding temperature of the seal, and/or decreasing the time of heat sealing the seal to the top layer and/or the reservoir. Application of force to the reservoir and/or the fluid package may change the internal pressure in the reservoir and/or the fluid package enough to cause the seal to rupture or separate from the top layer. Rupturing or separating the seal from the top layer allows fluids in the reservoir to pass through the reservoir opening, the top layer opening, and/or the cover layer opening and into the channel layer.

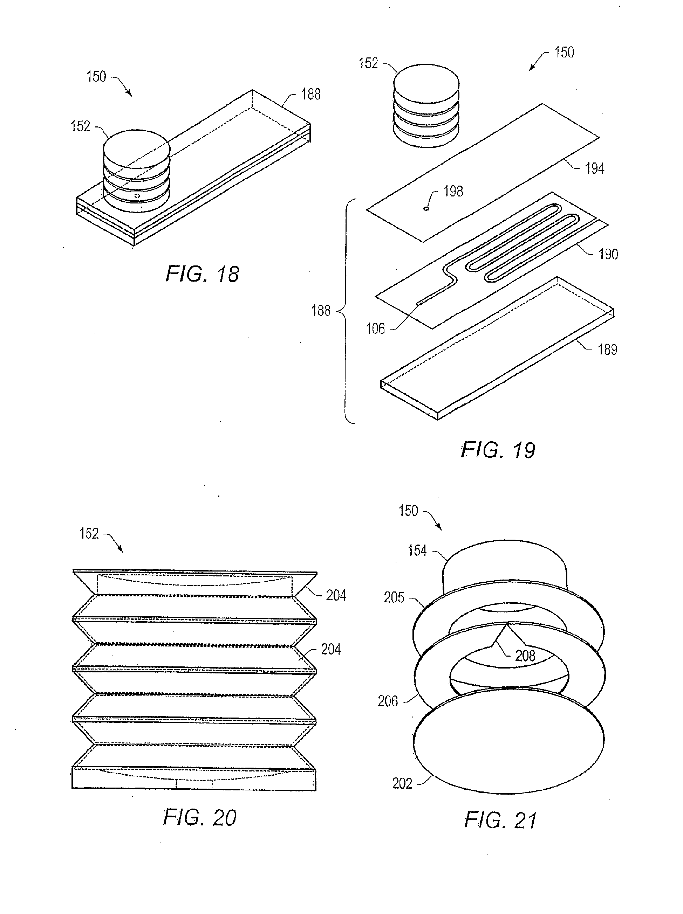

In some embodiments, a fluid package is coupled to a structure (e.g., a planar support or a cartridge). The structure may provide support for the fluid package. FIG. 18 depicts an embodiment of fluid delivery system 150 that includes fluid package 152 coupled to support 188 (e.g., a cartridge). FIG. 19 depicts an exploded view of fluid delivery system 150 depicted in FIG. 18. Support 188 may include support layer 189, channel layer 190 and top layer 194. Channel layer 190 may be coupled to support layer 189 and top layer 110. Channel layer 190 may be at least partially formed from double-sided adhesive. Channel layer may include channel 106.

Top layer 194 and support layer 189 may seal fluids in channel layer 190. Top layer 194 may include opening 198. Top layer opening 198 may direct fluid from fluid package 152 to channel layer 190. Top layer 194 or a portion of the top layer may include a material capable of coupling the top layer to fluid package 152 (e.g., vinyl adhesive or other types of adhesive). In some embodiments, top layer 194 and fluid package 152 are formed as one unit.

FIG. 20 depicts an embodiment of the fluid package depicted in FIG. 18 and FIG. 19. Fluid package 152 may include walls 204. Walls 204 may be formed of a material that allows the walls to be rigid while being able to collapse. Walls 204 may be corrugated and designed to fold. For example, walls 204 may form a shape similar to an accordion. Walls 204 may have limited outward flexibility under pressure. A corrugated fold may maximize the efficiency of the fluid package to deliver fluid. Walls 204 may be designed such that compression (full or partial) of the fluid package will not cause the base of the fluid package to flex upwards and/or cause the walls of the fluid package to flex outwards. In some embodiments, a diameter of the fluid package base is larger than a diameter of the fluid package opening and the top layer opening. The larger base may enhance bonding of the fluid package to the top layer. In some embodiments, fluid package 152 may have a rigid and/or ridged top surface. The rigid and/or ridged top surface may allow an actuator to contact the fluid package without puncturing the fluid package. The actuator may apply pressure to the top surface to force fluid from the fluid package.

FIG. 21 depicts an exploded view of a fluid delivery system that may be coupled to a support. Fluid delivery system 150 may include reservoir 154, gasket 206, and seal 202. Reservoir 154 includes one closed end and one open end. In some embodiments, the reservoir is formed from a mold made from Delrin® (DuPont, Wilmington, DE), an inflexible polymer, brass, stainless steel, and/or aluminum. For example, reservoir 154 may be molded from polydimethylsiloxane. The open end of reservoir 154 may include flange 205. Gasket 206 may couple flange 205 to seal 202. Seal 202 may be coupled to an opening in a top layer. Gasket 206 may include burst point 208. When a predetermined pressure is applied to reservoir 154, gasket 206 may rupture at burst point 208 causing seal 202 to rupture and/or tear. Rupturing of seal 202 allows fluid from reservoir 154 to flow through the opening in the top layer to a channel layer of the cartridge. In some embodiments, gasket 206 is a double-sided adhesive layer.

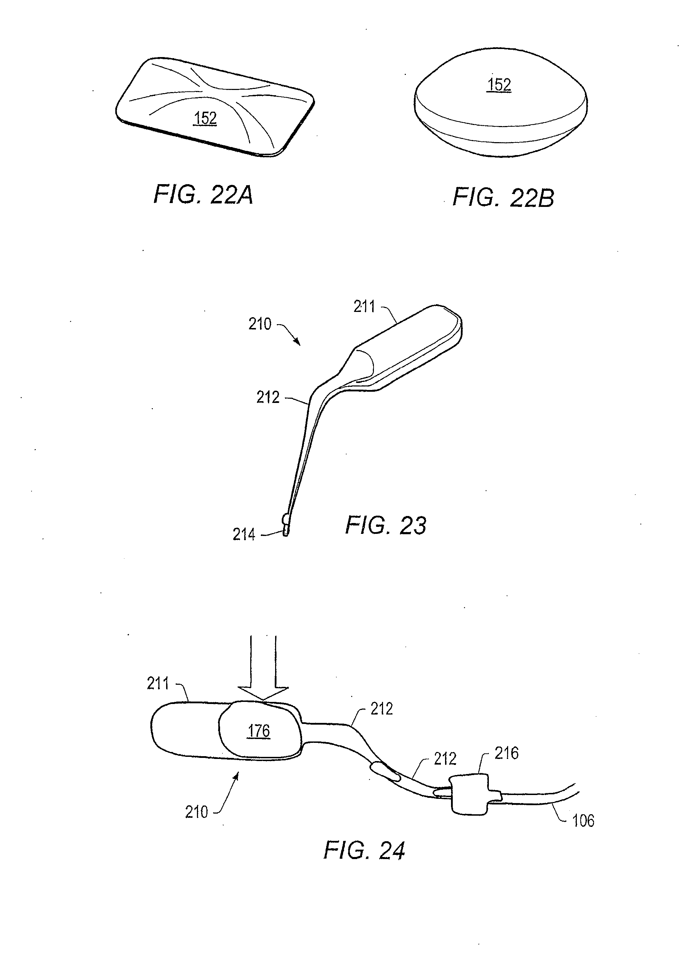

In some embodiments, a fluid delivery system includes a flexible conduit with a negative pressure source. The negative pressure source may be a fluid package. The negative pressure source may have a pressure less than ambient pressure. FIG. 22A depicts fluid package 152 as a negative pressure source before actuation. FIG. 22B depicts fluid package 152 as a negative pressure after actuation. When a negative pressure source is actuated (e.g., a seal is removed, a seal is ruptured, or a conduit is inserted in a wall or seal of the negative pressure source), air and/or fluid are drawn towards the negative pressure source until the pressure equalizes (the negative pressure source inflates). Actuating or opening a negative pressure source may create at least a partial vacuum in one or more channels.

A fluid delivery system may include a fluid bulb coupled, integrated, or embedded into the cartridge. A cartridge may be designed to incorporate commercially available fluid bulbs or custom designed fluid bulbs. Fluid bulbs may have various dimensions depending on dispensing volumes required and/or cartridge specifications.

FIG. 23 depicts an embodiment of a fluid bulb. Fluid bulb 210 may include body 211 and conduit 212. Conduit 212 may be straight, angled, and/or tapered. Conduit 212 may include tip 214. In some embodiments, tip 214 may be a breakaway sealed tip. Tip may be angled 214. Tip 214 may couple or removably couple to a cartridge.

FIG. 24 depicts an embodiment of fluid bulb 210 coupled or removably coupled to a channel in the cartridge. Body 211 may release liquid 176 upon actuation. Body 211 may be coupled, via conduit 212, to connector 216. Connector 216 may connect fluid bulb 210 to channel 106 of the cartridge. In some embodiments, tip 214 may be positioned in connector 216. In certain embodiments, the connector may include one or more openings to allow more than one fluid delivery system to be attached to the connector. Connector 216 may be permanently affixed to conduit 212. In some embodiments, connector 216 may be removably coupled to conduit 212 and/or channel 106.

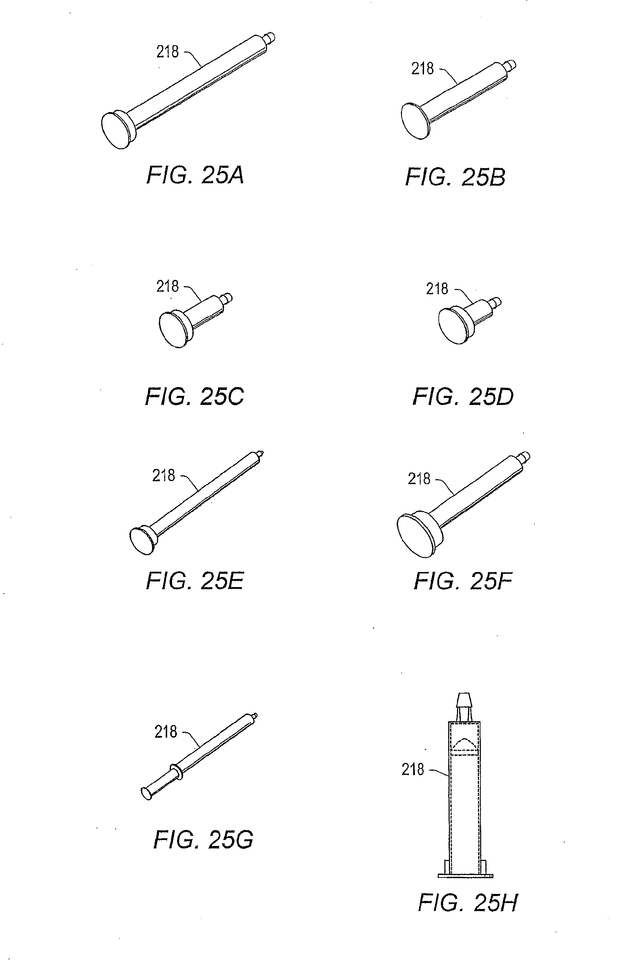

In some embodiments, a fluid delivery system may include one or more mini syringes coupled, embedded, or integrated into the cartridge. Syringes may be used to provide fluid delivery control, volume control, and/or a secure fluid seal to a cartridge. A syringe may be formed from a biocompatible material. Syringes may have a variety of designs including, but not limited to, the embodiments depicted in FIGS. 25A- 25H. The dimensions of syringes 218 may vary depending on dispensing volumes required and/or cartridge specifications. A mini syringe may hold a volume of about 50 microliters to 5000 microliters. Use of a syringe in a fluid delivery system may offer accurate and/or precise fluid delivery. In some embodiments, prefilled syringes may be positionable in a cartridge prior to use. Mini syringes may be disposable.

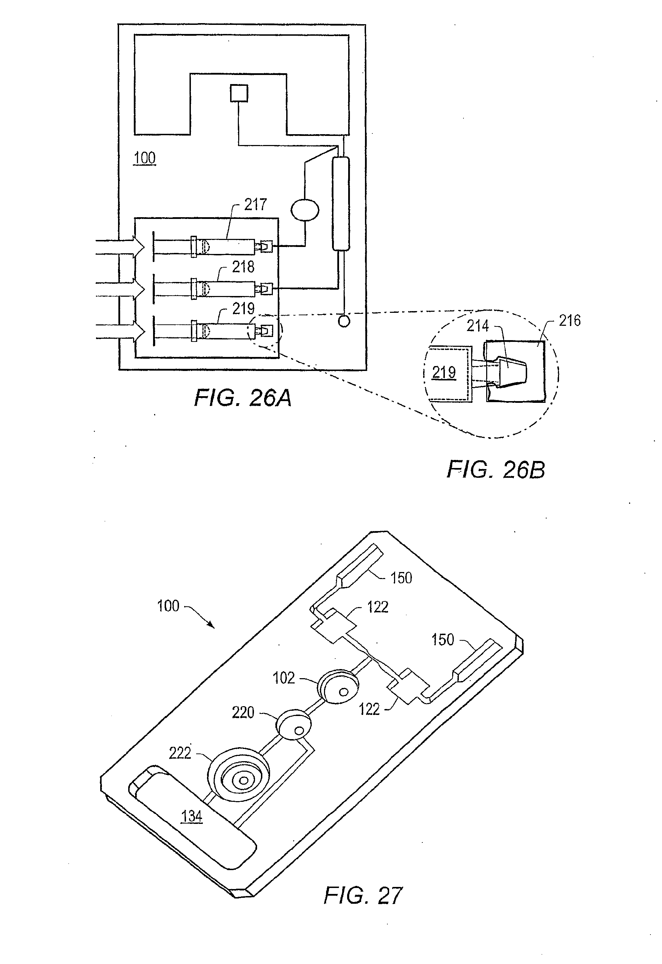

FIG. 26A depicts an embodiment of a cartridge that includes syringes 217, 218, 219. Syringes 217, 218, 219 may be linearly activated simultaneously or sequentially. Syringes 217, 218, 219 may be actuated when a prong contacts the fluid delivery system. In some embodiments, an actuator with three prongs of different lengths may be actuated to release fluid from the syringes. Using an actuator with prongs of different lengths may allow actuation of different syringes at different times using a single actuation of the prongs. Since the prongs are of different lengths, the actuation system may be set up such that each prong contacts a syringe at a different, predetermined, time. As each prong of the actuator depresses a syringe, fluid may be released. Syringes 217, 218, 219 may deliver fluid to various portions of the cartridge. For example, syringe 217 may deliver a fluid toward reagent region 122, while syringe 218 delivers fluid towards metered volume portion 138.

An expanded view of one end of syringe 219 is depicted in FIG. 26B. Syringe 219 includes tip 214 positionable in connector 216. In some embodiments, connector 216 is coupled to the cartridge. Tip 214 may be designed to mate with connector 216. In some embodiments, a tip may include adhesive and/or a gasket to seal the syringe to the connector. A cartridge may include a spring mechanism that holds the syringes in position.

In some embodiments, a metered syringe pump is used to push and pull fluids through the system. During use, a capillary containing sample may be inserted into the cartridge coupled to a fluid bus. The system may then be filled with buffer through two lines. Using a third line, sample may be pushed into a trap that releases air trapped in the sample. A line may then be used to draw a predetermined amount of sample into the detection system. After sample analysis, the system may be washed with a buffer solution and waste may be transferred to a waste reservoir positioned in the cartridge or coupled to the cartridge.

In some embodiments, an analyte detection system may be used to detect multiple analytes. The analyte detection system may include a multi-functional cartridge. The multi-functional cartridge may include two or more detection systems. In some embodiments, a single cartridge or system may include a microsieve-based detection system and a particle-based detection system. The microsieve-based detection system may be positioned upstream from the particle-based detection system. A sample may be introduced into the cartridge or system and passed through the microsieve-based detection system where a portion of the sample is retained by the microsieve. The material passing through the microsieve may be passed to the particle-based detection system. Particles in the particle-based detection system may interact with one or more analytes in the fluid passed over the particles. In alternate embodiments, a particle-based detection system may be positioned upstream from a microsieve-based detection system. In certain embodiments, particles may be coupled to (e.g., at least partially embedded in) at least a portion of a microsieve of a microsieve-based detection system. In combination, the two detection systems allow the presence of at least two analytes to be assessed in a single sample at about the same time.

FIG. 27 depicts perspective top view of an embodiment of a cartridge that includes two detection systems. Cartridge 100 may include fluid delivery systems 150, reagent regions 122, collection region 102, microsieve-based detection system 220, particle-based detection system 222, and waste reservoir 134.

Sample may be deposited in and/or delivered to collection region 102. In some embodiments, a filter may be positioned proximate the collection region to allow removal of large particles and/or coagulated matter from the sample. In some embodiments, fluid may be released from fluid delivery systems 150 directly into channel 106. In some embodiments, fluid from the fluid delivery system may flow directly to one of the detection systems (e.g., flow directly to the microsieve-based detection system).

Fluid may be released from fluid delivery systems 150 and pass through reagent region 122. Reagent region 122 may include dried reagents, anti-coagulants, and/or detectable labels. In some embodiments, reagents and/or detectable labels on and/or in the reagent pad may be reconstituted by fluid passing over and/or through reagent region 122. In some embodiments, reagent region 122 includes reagent pads that contain dried reagents, anti-coagulants, and/or detectable labels. A reagent pad acts, in some embodiments, as a filter and removes large particles and/or coagulated matter from the sample.

In some embodiments, a reagent region may be positioned proximate the collection region so that sample from the collection region may pass over the reagent pad and reconstitute reagents and/or detectable labels in the reagent region. Directly flowing sample over and/or through a reagent region may reduce the time required for reaction between sample and reagents and/or detectable labels.

After fluid flows through and/or over reagent region 122, fluid may flow over and/or through collection region 102. A combined fluid and sample flows toward the microsieve-based detection system 220 and particle-based detection system 222. In some embodiments, a combined fluid and sample passes through the particle-based detection system first. In certain embodiments, a combined fluid and sample may first pass through a first detection system for a first test and only pass through the second detection system based on the results of the first test.

Microsieve-based detection system 220 and/or particle-based detection system 222 may be coupled to waste region 134. Fluid may flow from microsieve-based detection system 220 to particle-based detection system 222 and then to waste region 134.

In some embodiments, a cartridge of an analyte detection system may be multi-functional (e.g., used to analyze two or more analytes in a sample). In some embodiments, the analysis may be done simultaneously, or substantially simultaneously. For example, a cartridge may be used to assess WBC count and CRP levels in a whole blood sample.

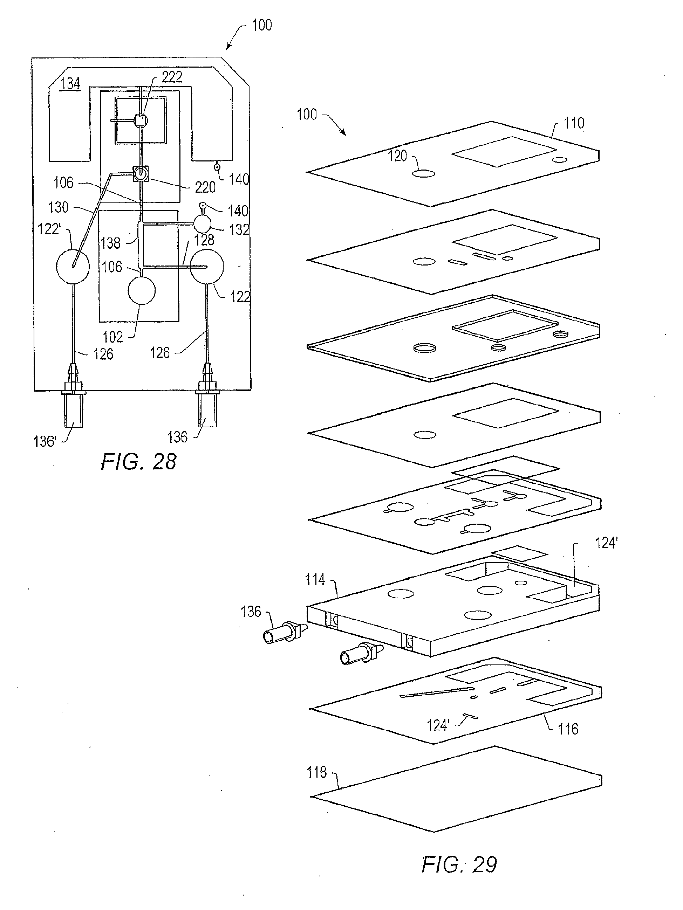

FIG. 28 depicts a top view of an embodiment of multi-functional cartridge 100. Cartridge 100 may include connectors 136, 136', channels 106, 126, 128, 130, metered volume portion 138, collection region 102, reagent regions 122, 122', overflow reservoir 132, microsieve-based detection system 220, particle-based detection system 222, waste reservoir 134, and vents 140.

Sample may be deposited in collection region 102. Sample flows from collection region 102 through channel 106 and enters metered volume portion 138. Sample may then be delivered to microsieve-based detection system 220 from metered volume portion 138. Excess sample may be collected in overflow reservoir 132.

Connectors 136, 136' may connect one or more fluid delivery systems to the cartridges. Fluid from the fluid delivery systems flows through channels 126 to reagent regions 122, 122', respectively. Fluid may be delivered at different time intervals or substantially simultaneously to the reagent regions from separate fluid delivery systems. In some embodiments, fluid from the fluid delivery system may flow directly to one of the detection systems (e.g., flow directly to the microsieve-based detection system).

Fluid may pass through or over reagent region 122, through channel 128, and enter metered volume portion 138. Fluid may be delivered to microsieve-based detection system 220 from metered volume portion 138. Excess fluid and/or sample may be collected in overflow reservoir 132.

A fluid may pass through or over reagent region 122 and through or over reagent region 122'. Fluid from reagent region 122' flows toward microsieve-based detection system 220 through channel 130. In some embodiments, an additional amount of sample is delivered from metered volume portion 138 to microsieve-based detection system 220 before fluid from reagent region 122' reaches the microsieve-based detection system. In some embodiments, fluid from reagent region 122' may flow directly to particle-based detection system 222.

Sample and/or fluid that passes through or over microsieve-based detection system 220 is transported to particle-based detection system 222. The detection systems may be optically coupled to a detector and the analytes in the sample may be analyzed. In some embodiments, the analytes in the sample retained in microsieve-based detection system 220 may be analyzed prior to sending the remainder of the sample to the particle-based detection system 222. In some embodiments, the sample may be transported to the particle-based detection system 222 before being delivered to the microsieve-based detection system 220.

Microsieve-based detection system 220 and/or particle-based detection system 222 may be coupled to waste region 134. Fluid may flow from microsieve-based detection system 220, to particle-based detection system 222, and then to waste region 134.

FIG. 29 depicts an exploded view of the embodiment of cartridge 100 depicted in FIG. 28. Cartridge 100 includes top layer 110, top layer opening 120, sample layer 114, reservoir layer 116, reservoirs 124, support layer 118, and connectors 136 designed to couple to fluid delivery systems. In certain embodiments, one or more additional fluid delivery systems (e.g., fluid packages) may be coupled to, positioned on or positioned in cartridge 100 to provide fluid for sample processing during use.

Cartridges described herein may include a microsieve-based detection system. A microsieve-based detection system may include a microsieve and, optionally, a support. The microsieve may retain at least a portion of matter in the sample, while allowing other portions of the sample to pass through the microsieve. For example, with blood samples, a microsieve may be selected that will allow red blood cells and plasma to pass through the microsieve, while the microsieve retains white blood cells.

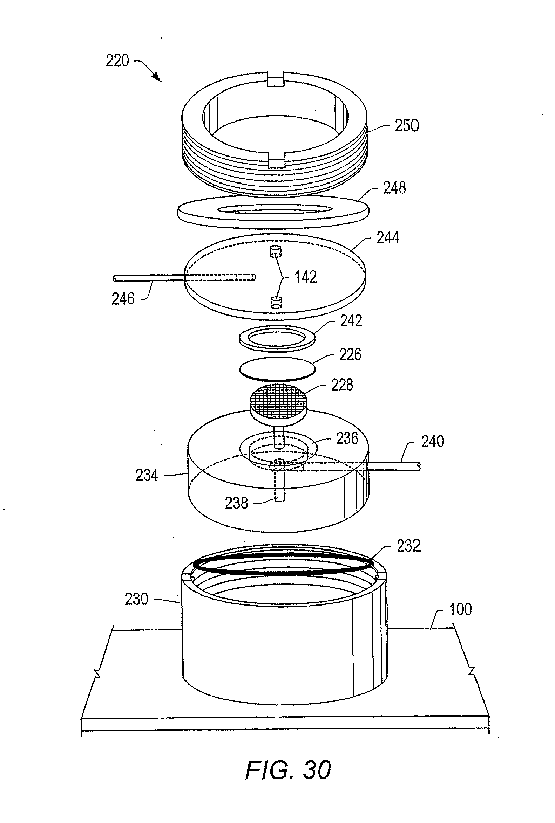

FIG. 30 depicts an embodiment of a microsieve-based detection system. The microsieve-based detection system may be coupled to, positioned in, or positioned on cartridge 100. The microsieve-based detection system may be integrated within a cartridge.

Microsieve-based detection system 220 includes microsieve 226 and support 228. In some embodiments, a microsieve may be designed such that a support is not necessary. For example, a thickness of a microsieve may be selected so that a microsieve remains substantially planar. In some embodiments, the microsieve is porous.

The microsieve-based detection system 220 may include housing 230 positioned in and/or on cartridge 100. Bottom spacer 232 may position bottom member 234 in housing 230. Bottom member 234 may include indentation 236 to receive microsieve 226 and support 228. Channel 238 in bottom member 234 may receive fluids flowing through microsieve 226 and conduct the fluids to outlet 240. In some embodiments, the outlet is coupled to a waste reservoir of the cartridge. Gasket 242 may be positioned between top member 244 and microsieve 226. Gasket 242 may reduce leaks from the microsieve-based detection system. Inlet 246 coupled to top member 244 may allow fluids to enter the microsieve-based detection system. Top spacer 248 may be positioned between top member 244 and fastening member 250. Top member 244 may include viewing windows 142. Viewing windows 142 may be transparent to visible light and/or ultraviolet light. Fastening member 250 may keep the components of the microsieve-based detection system coupled during use. Fastening member 250 may be machined (e.g., threaded and/or tapered) to mate with housing 230.

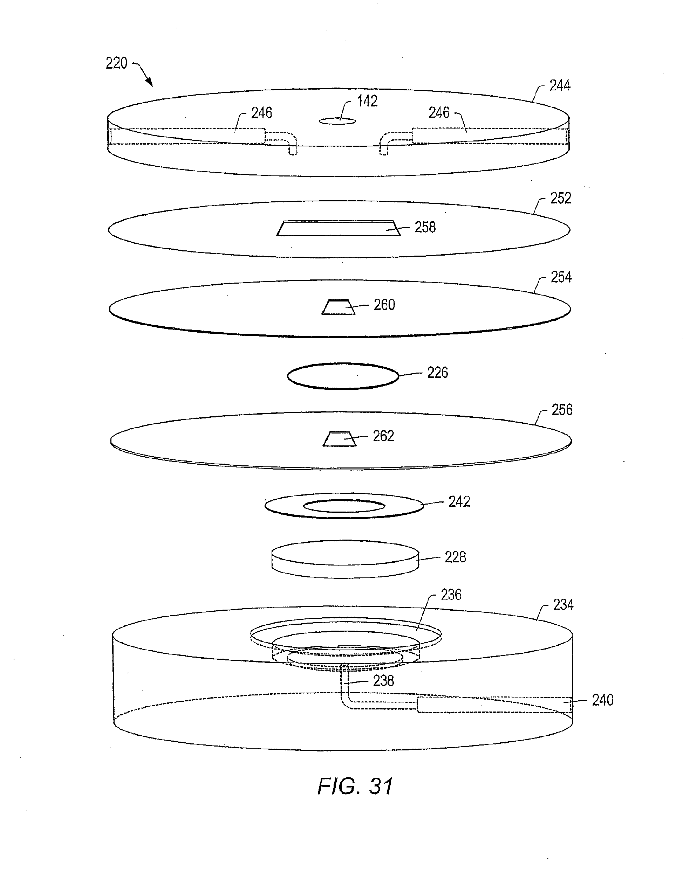

In some embodiments, a microsieve-based detection system may include layers to direct fluid flow. FIG. 31 depicts an exploded view of an embodiment of a microsieve-based detection system with directed fluid flow. The microsieve-based detection system may include a plurality of layers positioned in the cartridge or on a surface of the cartridge. Microsieve-based detection system 220 includes top member 244, top layer 252, middle layer 254, microsieve 226, bottom layer 256, and support 228. Layers of the microsieve-based detection system may be coupled to each other. Top layer 252, middle layer 254, and bottom layer 256 may include openings 258, 260, and 262, respectively. Fluid may flow from inlet 246 through openings 258 and 260 to and/or through microsieve 226. A portion of analytes in the fluid flowing to the microsieve 226 may be retained on the microsieve. Light may be directed to a portion of the microsieve to detect analytes in the fluid. Fluid may flow through microsieve 226, through opening 262 and through outlet 240 to one or more reservoirs.

In some embodiments, a cavity is formed between top member 244 and microsieve 226. Top member 244 may be spaced at a distance above microsieve 226 to form the cavity and/or the top member may have a shape such that a cavity is formed between the top member and the microsieve.

Top member 244 may be at least partially transparent to visible light and/or ultraviolet light. Top member 244 is, in some embodiments, formed of PMMA, polycarbonate, cyclic olefin copolymers, and/or glass. Top member 244 may include viewing window 142. In some embodiments, a portion of top member 244 may be opaque or translucent to visible light and/or ultraviolet light while viewing window 142 may be substantially transparent to visible light and/or ultraviolet light.

Fluid may be directed towards microsieve 226 through top layer 252 positioned below top member 244. A portion of top layer 252 may be formed of a material or materials (e.g., vinyl material and/or an adhesive) capable of coupling the top layer to middle layer 254. Top layer 252 may direct flow of fluid from top member 244 through opening 258 and towards microsieve 226.

Middle layer 254 may be positioned below top layer 252. Middle layer 254 may be formed of a vinyl material and/or adhesive. A portion of middle layer 254 may be formed of a material or materials (e.g., vinyl material and/or an adhesive) capable of coupling the middle layer to top layer 252 and/or bottom layer 256. Middle layer 254 may be opaque or translucent to visible light and/or ultraviolet light. Middle layer 254 may direct fluid to flow through opening 260 toward microsieve 226.

Fluid that flows through microsieve 226 passes through opening 262 in bottom layer 256. Bottom layer 256 may direct fluid flow through opening 262. A portion of bottom layer 256 may be formed of a material or materials (e.g., vinyl material and/or an adhesive) capable of coupling the bottom layer to middle layer 254. In some embodiments, opening 262 in bottom layer 256 has a size similar to the size of opening 260. Openings with similar sizes may allow fluid to be retained in the area of microsieve 226 between the middle layer 254 and bottom layer 256.

Gasket 242 may be positioned below bottom layer 256 to inhibit leaks from the microsieve-based detection device. Support 228 may be positioned below gasket 242. In some embodiments, support 228 may inhibit sagging of microsieve 226. Support 228 may be positioned in bottom member 234 and/or an opening of the cartridge. Bottom member 234 may include indentation 236 to receive microsieve 226 and/or support 228. Channel 238 in bottom member 234 may receive fluids flowing through microsieve 226 and conduct the fluids to outlet 240.

In some embodiments, a microsieve is selected depending on the analyte of interest. The microsieve may capture or retain matter in the sample (e.g., particles, cells, or other matter). Matter may be retained on a surface of the microsieve and/or in the microsieve. The microsieve may include a thin film or layer capable of separating one or more components from a liquid passing through the film or layer. The surface of a microsieve may be hydrophilic to promote cell proliferation across the surface of the microsieve. A microsieve may have a variety of shapes including, but not limited to, square, rectangular, circular, oval, and/or irregularly shaped. In some embodiments, a microsieve includes openings (e.g., pores) that inhibit an analyte of interest from passing through the microsieve. A microsieve designed to capture substantially all of an analyte of interest may be selected depending on the analyte of interest.

In some embodiments, a microsieve is a monolithic microchip with a plurality of high-density holes. The monolithic microchip microsieve may be formed from materials including, but not limited to, glass, silica/germanium oxide doped silica, inorganic polymers, organic polymers, titanium, silicon, silicon nitride, and/or mixtures thereof. Organic polymers include, but are not limited to, PMMA, polycarbonate (PC) (e.g., NUCLEPORE® membranes, Whatman; Florham Park, NJ), and resins (e.g., DELRIN®). A microsieve formed of polymeric material may include pores of a selected range of dimensions. In certain embodiments, a microsieve is an acrylic frit. In some embodiments, a microsieve is formed of multiple layers (e.g., at least 2 layers, at least 3 layers, at least 4 layers, or at least 5 layers) of etchable and/or non-etchable glass. In some embodiments, a microsieve is formed from an anti-reflective material and/or a material that does not reflect light in the ultraviolet-visible light range. In some embodiments, a microsieve includes one or more locking mechanisms to assist in securing placement of the microsieve in or on the cartridge or support.

In some embodiments, microsieves may be manufactured from silicon materials and/or plastic materials. In some embodiments, a microsieve is a layered plastic microsieve. In certain embodiments, a microsieve is a membrane (e.g., polycarbonate track-etched membrane).

Microsieves may have a thickness from about 0.001 mm to about 25 mm, from about 1 mm to about 20 mm, or from about 5 mm to 10 mm. In some embodiments, a thickness of the microsieve ranges from about 0.001 mm to about 2 mm. Microsieves may have a diameter from about 1 mm to 500 mm, from about 5 mm to about 100 mm, or from about 10 mm to about 50 mm.

Pores of a microsieve may have various dimensions (e.g., diameter and/or volume). In some embodiments, pores of the microsieve may have approximately the same dimensions. In some embodiments, microsieve pores have a pore diameter ranging from about 0.0001 mm to about 1 mm; from about 0.0002 mm to about 0.5 mm; from about 0.002 mm to about 0.1 mm. The microsieve pores have, in some embodiments, a pore diameter of at most 0.005 mm or at most 0.01 mm. Microsieves with different pore sizes may be chosen for systems including two or more microsieve-based detection systems in an analyte detection system.

Pores of the microsieve may be randomly arranged or arranged in a pattern (e.g., a hexagonal close-packed arrangement. Pores of the microsieve may occupy at least 10 percent, at least 30 percent, at least 50 percent, or at least 90 percent of the surface area of a microsieve. The pores may assist in selectively retaining matter in a sample and/or a fluid. The pores may be positioned to achieve a desired flow pattern or back pressure. In some embodiments, a thickness of a microsieve may exceed a pore diameter of a microsieve.

In some embodiments, a microsieve is positioned from about 0.3 mm to about 0.5 mm below a top surface of the cartridge. In some embodiments, the microsieve includes a support. In some embodiments, a microsieve is designed such that a support is not needed (e.g., utilizing a microsieve having a thickness of at least 5 mm). In some embodiments, one or more layers separate the microsieve and the support. The support may facilitate positioning of the microsieve in or on the cartridge.

A support may be coupled to the cartridge or integrated within a cartridge. In some embodiments, a support is used to maintain a microsieve in a substantially planar orientation. In certain embodiments, a support is integrated with one or more microsieves. The support may be formed of the same material as the microsieve. The support may be formed of materials including, but not limited to, glass, polymers, metal, silicon, PC, cyclic olefin copolymer (COC), nylon, and/or nitrocellulose. The support may be, but is not limited to, a stainless steel filter or a plastic mesh.

A support assembly may be coupled to the support to allow the microsieve and support to withstand backpressures of at least 10 psi. The support may be selected to produce a predetermined backpressure. When backpressure is controlled, cells may be more uniformly distributed across a surface of a microsieve. Uniform distribution of cells across a microsieve surface may facilitate imaging of a region containing cells and/or analyte detection.

In some embodiments, a support includes open areas (e.g., pores or holes). Open areas in the support may have any shape, such as substantially square and/or substantially circular. The shape of the open areas in the support may be different than the shape of pores in the microsieve. Open areas of the support may be equal to or greater than the diameter of the pores of the microsieve. In some embodiments, a support has open areas with diameters ranging from about 0.0001 mm to about 1 mm, from about 0.0002 mm to about 0.5 mm, or from about 0.002 mm to about 0.1 mm. The open areas have, in some embodiments, diameters of at most 0.005 mm or at most 0.01 mm.

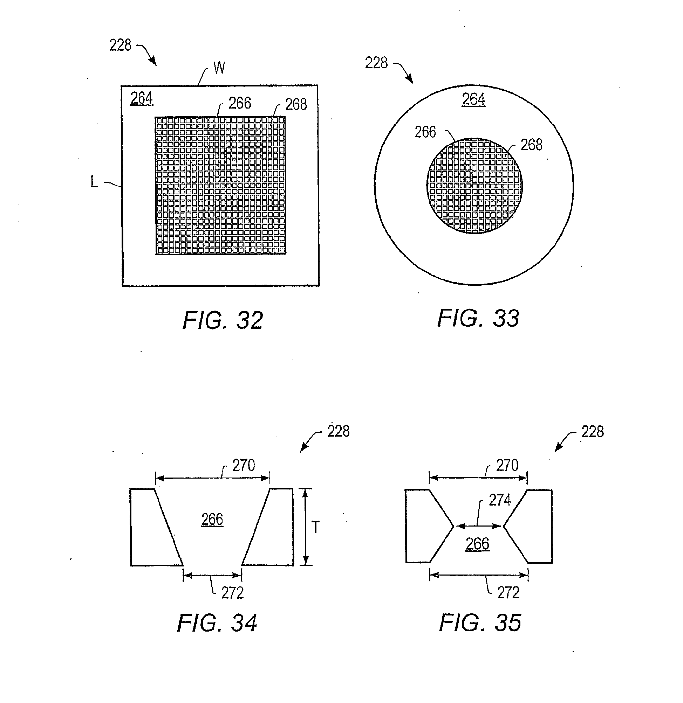

FIG. 32 depicts a top view of an embodiment of a support having a parallelogram shape. Support 228 may include outer area 264 and open area 266. Open area 266 may include openings 268. Support 228 may be machined and/or fabricated out of, for example, plastic or stainless steel) such that open area 266 has various shapes. Length (L) of outer area 264 may be greater than or about equal to width (W) of the outer area (e.g., outer area 264 may have a substantially square, rectangular, oval, or round shape). A length of open area 266 may be greater than, or about equal to a width of the open area (e.g., open area 266 may have a substantially square shape or a substantially rectangular shape). Open area 266 may have dimensions that are less than the dimensions of outer area 264. In some embodiments, an outer area of a support may have a length of about 4 mm to about 6 mm and a width of about 4 mm to about 6 mm. An open area of a support may have a length from about 2.5 mm to about 4 mm and a width from about 2.5 mm to about 4 mm. FIG. 33 depicts a top view of an embodiment of support 228 having a euclidian shape (e.g., substantially oval or circular). Open area 266 may have dimensions that are less than the dimensions of outer area 264. Open area 266 may be customized to include a desired number of openings of selected sizes and hole density in a chosen pattern

FIG. 34 depicts a perspective cross-sectional view of open area 266 of support 228. Open area 266 includes top portion 270 and bottom portion 272. Bottom portion 272 may be equal to or less than the top portion 270. In some embodiments, a support may include a top portion formed from a film and a bottom portion formed from silicon. A support may be formed from a hydrophilic and/or anti-reflective material. Forming a support from a hydrophilic material may reduce the formation of air bubbles across the microsieve and support. Use of a hydrophilic material may also inhibit nonspecific binding of analytes. Using a support made at least partially of anti-reflective material may enhance analyte detection.

In embodiments where the support is formed from silicon (e.g., silicon etching), a bottom portion of the support has a thickness (T) ranging from about 10 um to about 5 mm. For silicon supports, a thickness of the support is related to a length (Lt) of top portion 270 and a length (Lb) of bottom portion 272 as represented by the equation:

$$T = \tan(54.7) \times (Lt-Lb)/2.$$

FIG. 35 depicts a perspective cross-sectional view of open area 266 of support 228. Open area 266 includes top portion 270, middle portion 274, and bottom portion 272. A length of middle portion 274 may be less than a length of top portion 270 and a length of bottom portion 272. Thus, an hourglass shaped opening is formed. Support 228 may reduce back pressure compared to, for example, embodiments depictged in FIGS. 5 and 6.

In a microsieve-based detection system, a fluid and/or sample in the detection region of the cartridge may be treated with a light. Interaction of the light with the fluid and/or sample may allow the analyte to be detected. Light from one or more light sources may shine on or in at least the detection region of a cartridge, such as the portion of the microsieve where the fluid and/or sample is retained. The light may allow a signal from the retained fluid and/or sample to be detected. When light shines on a microsieve surface, some of the light may be reflected. Areas proximate the detection region may also reflect some of the light that shines on a sample. Light reflecting from the microsieve surface and/or support may interfere with obtaining an accurate reading from the detector. In some embodiments, an anti-reflective material may be optically coupled to the microsieve and/or the support.

In some embodiments, an anti-reflective material is optically coupled to the microsieve and/or the support. Alternatively, an anti-reflective material may be a coating on a surface of the microsieve and/or support. For example a black coating on a surface of the microsieve and/or support may act as an anti-reflective coating.

In certain embodiments, a portion of the microsieve and/or support may be made of an anti-reflective material. The anti-reflective material may be positioned above or below a microsieve. An anti-reflective material may inhibit the reflection of light applied to analytes retained in or on the microsieve. The anti-reflective material may absorb one or more wavelengths of light that are emitted by an analyte of interest. The anti-reflective material may improve the contrast of an image of at least a portion of the analyte retained in or on the microsieve by inhibiting reflection of light.

In some embodiments, materials that form the components of the cartridge control flow of fluids through the cartridge. In some embodiments, hydrophilic material is coupled to the microsieve and/or support. Alternatively, hydrophilic material may be a coating on a surface of a microsieve and/or support. In certain embodiments, a portion of the microsieve and/or support is made from hydrophilic material. Hydrophilic material may enhance flow of a fluid through the microsieve. Hydrophilic material may reduce the formation of air bubbles across the microsieve and support and/or inhibit nonspecific binding of analytes. Hydrophilic material may attract or have an affinity for aqueous fluids flowing through the microsieve. Hydrophilic material may be positioned downstream of the microsieve.

In some embodiments, hydrophobic material is positioned in or on the cartridge. Hydrophobic material may repel aqueous fluid away from surfaces of the cartridge and cause the fluid to flow towards the microsieve. For example, positioning a top member above the microsieve forms a cavity between the top member and the microsieve. Hydrophobic material may be coupled to the top member. The hydrophobic material may be a coating on a surface of the top member, and/or the hydrophobic material may form a portion of the top member. As an aqueous sample or fluid enters the cavity, it is repelled away from the hydrophobic top member and flows towards the microsieve.

One or more microsieve-based detection system may be used alone or in combination with one or more particle-based detection systems. In some embodiments, a particle-based detection system includes a supporting member with one or more cavities. One or more particles may be positioned in the cavities of the supporting member. In some embodiments, a particle-based detection system detects one or more analytes simultaneously using reactive particles that interact with the analytes.

In a particle-based detection system, a particle may produce a signal in the presence of an analyte. Particles may produce optical (e.g., absorbance or reflectance) or fluorescent/phosphorescent signals upon exposure to the analyte. Particles include, but are not limited to, functionalized polymeric beads, agarose beads, dextrose beads, polyacrylamide beads, control pore glass beads, metal oxides particles (e.g., silicon dioxide ($SiO_2$) or aluminum oxides ($Al_2O_3$)), polymer thin films, metal quantum particles (e.g., silver, gold, and/or platinum), and semiconductor quantum particles (e.g., Si, Ge, and/or GaAs).

The particles may include a binding agent coupled to a polymeric bead. The binding agents, in some embodiments, are chosen for interacting with analytes. This interaction may take the form of a binding/association of the binding agents with the analytes. A particle, in some embodiments, possesses both the ability to bind the analyte of interest and to create a modulated signal. The particle may include binding agents, which possess the ability to bind the analyte of interest and to create a modulated signal. Alternatively, the particle may include binding agents and indicators. The binding agent may posses the ability to bind to an analyte of interest. Upon binding the analyte of interest, the binding agent may cause the indicator molecule to produce the modulated signal. The binding agents may be naturally occurring or synthetic binding agents formed by rational design or combinatorial methods. Natural binding agents include, but are not limited to, DNA, RNA, proteins, enzymes, oligopeptides, antigens, and antibodies. Either natural or synthetic binding agents may be chosen for their ability to bind to the analyte molecules in a specific manner.

Some particle-based detection systems and particles for use in particle-based detection systems are described in US applications 09/616,731; 20020160363; 20020064422; 20040053322; 20030186228; 20020197622; 20040029259; 20050136548; and 20050214863; and US patents 6,680,206; 6,602,702; 6,589,779; 6,649,403; 6,713,298; and 6,908,770.

In some embodiments, components necessary to obtain and assist in the analysis of a fluid and/or sample are included in a single package as a kit. In some embodiments, a package includes a cartridge, a sample collection device (e.g., a lancet, a syringe, or a needle), and one or more disinfectant wipes. Disinfectant wipes may be used prior to using the sample collection device to draw a sample from a person. A disinfectant wipe may also be used by a user to wipe portions of the analyte detection system before or after sample analysis. Packaging a cartridge and a sample collection device together may make collection and analysis of samples easier for an operator. Packaging a cartridge and a sample collection device together may inhibit contaminants from entering the cartridge and the sample collection device.

A package may be sealed to inhibit entrance of air (e.g. vacuum sealed). A package may be formed from a material that is waterproof and/or water resistant, controls static electricity, kills microbes that enter the package, blocks sunlight, and blocks UV light. Materials that have these properties include polymeric materials and metal foils. A package may have a positive pressure to protect items in the package. Insulating materials, such as polyurethane or bubble wrap, may be placed inside a package to protect items in the package. A package may contain inert gas or dessicant.

A cartridge and/or system may include a control to ensure that the cartridge and/or system are operating correctly. Long storage times and/or less than ideal storage facilities may damage and/or affect the quality of the cartridge and/or components of the cartridge.

In some embodiments, it is desirable to check the fluids and/or reagents stored in the cartridge. A particle larger than cells to be detected or other particles in the sensor array may be placed in a detection system as a control analyte. For example, a control analyte includes any type of particle previously described, including quantum particles or dots. Control analytes may allow assessment of a cartridge and/or equipment used in conjunction with the cartridge, such as, but not limited to, light sources, detectors, analyzers, and/or computer systems. The control analyte may produce a result within a selected range and/or produce a result substantially similar to an expected result from a selected analyte.

In some embodiments, a control analyte is a control particle. A control particle may be produced by coupling a known analyte to a particle. Reagents passing over the detection system may interact with the sample and the control particle. When an image of the detection system is captured the control particle is used to determine if the cartridge is functioning properly. For example, if a control particle is not detected, the quality of the reagents may be determined to be poor and the cartridge may be discarded. In some embodiments, a control particle is distinguishable from other matter in the detection system due to the size of the control particle.

In some embodiments, a control analyte is stored in or on the cartridge. For example, a bead containing a known analyte may be designed to produce a predetermined signal. A weak or non-existent signal from the control analyte may indicate an improperly functioning cartridge.

In certain embodiments, a cartridge control system may be coupled to, positioned in, positioned on, or integrated in the cartridge. The cartridge control system may include, but is not limited to, one or more control analytes, one or more buffer solutions, and one or more reagent pads containing a dried predetermined analyte. In some embodiments, the cartridge control system includes one or more fluid packages. The fluid packages may include one or more control analytes, one or more control solutions, and/or other reagents. Prior to analyzing a sample, a control solution may be released from the fluid packages and pass over detection system.

In some embodiments, the detection system includes a control-detection system and an analyte detection system. The known or control analyte may be applied to the control-detection system and the sample may be applied to the analyte detection system. If the known analyte is captured by the control-detection system and a predetermined signal is produced, the cartridge is considered to be operating properly. If the known analyte passes through the control-detection system but does not produce an appropriate signal, it may indicate that the cartridge is not working properly (e.g., due to improper storage and/or age of the cartridge). Improperly working cartridges may be discarded prior to deposition of a sample on the cartridge. Once the quality of the cartridge has been confirmed, a sample is analyzed for analytes.

In some embodiments, an analyte detection system is used with different cartridges to detect a plurality of analytes. The analyte detection system may include a housing. The housing may include a slot for receiving a cartridge. In some embodiments, the housing includes an optical platform.

In some embodiments, an analyte detection system may include an analyzer (e.g., a computer system). The analyzer may analyze images and/or control the one or more components of the analyte detection system. The analyzer may be coupled to the housing and/or an optical platform of the analyte detection system. The analyzer and/or analyte detection system may include a display to show images produced by the detector. The analyzer and/or analyte detection system may include a temperature controller. A temperature controller may control temperatures of or around the housing or components of the analyte detection system.

The analyte detection system may include a cartridge positioning system. In some embodiments, the cartridge positioning system is included in a housing of the analyte detection system. The cartridge positioning system may automatically position the cartridge so that it is optically coupled to one or more light sources and/or one or more detectors. In some embodiments, one or more detectors and/or one or more light sources are coupled or directly attached to an optical platform.

One or more detectors may include, but are not limited to, a CCD detector, a CMOS detector, a camera, a microscope, or a digital detector. One or more detectors may detect one or more signals from an analyte. For example, a CMOS detector may be used for detection in microsieve-based detection systems or for quantitative measurements while a CCD camera detector may be used for detection in particle-based detection systems. A signal may be represented by one or more wavelengths of light absorbed by: the analyte; matter retained on a microsieve; a fluorophore; a particle, or combinations thereof. A signal may be represented by the fluorescence of: the analyte; matter retained on a microsieve; a fluorophore; a particle; or combinations thereof. The detector may transform the signal to one or more images. The images may be of: one or more analytes in one or more fluids; samples retained on or in one or more microsieves; one or more particles of a detection system; or combinations thereof.

In certain embodiments, a monochromatic detector may be used. When a monochromatic detector is used with multiple fluorophores and excitation sources, one or more filters may be used to isolate light emitted in a selected spectrum. For example, a green filter may be used to isolate light emitted from a green fluorophore, and thus an image of the detection system may include material that emits green light. A red filter may be used to isolate light emitted from a red fluorophore.

In some embodiments, one or more light sources may emit light of different wavelengths. For example, a light source may be capable of emitting two or more different wavelengths of light. Different wavelengths of lights may enhance detection of various types of analytes. In certain embodiments, different assays require different exposure times when images of the detection systems are obtained. An exposure time from approximately 1-5 seconds may be used.

In some embodiments, two light sources (e.g., green and red LED light sources) and one or more detectors may be used to assist in detection of an analyte in a fluid and/or sample. Each light source may emit light at a different wavelength. For example, two light sources may be included in an optical platform and different combinations of light sources may be used to detect different analytes. Green and red light sources may be used for CD4 cell assays, *E. coli* assays, ß-galactosidase (BG) assays, and cellular assays. A green light source may be used for CRP, tumor necrosis factor-$\alpha$ (TNF-$\alpha$), and BG assays. A red light source may be used for interleukin-6 (IL-6) assays.

In some embodiments, an analyte detection system includes several different lenses for the detection of different analytes. More than one lens may be used in the detection of some analytes. The lenses may be included in an optical platform and/or as part of a detector. Lenses of different magnification levels may be used in the analysis of one or more analytes. Lens magnification levels may include, but are not limited to, 4x, 10x, and/or 20x. For example, a 10x lens may be used for CD4 assays, while a 4x lens may be used for CRP, TNF-$\alpha$, and IL-6 assays. Alternatively, a 4x lens and a 10x lens may be used in the detection of *E. coli* and/or BG assays.

In some embodiments, fiber optic cables are coupled to a detection system to facilitate image capturing. In certain embodiments, fiber optic cables are coupled to a particle-based detection system to facilitate analyte detection and reduce the need to adjust magnification between detection regions.

In some embodiments, an analyte detection system includes a motor coupled to a lens and/or a detector. The motor may be coupled to the housing, the optical platform and/or a detector of the analyte detection system. A motor may move the lens and/or the detector in a direction perpendicular to the plane in which the cartridge is positioned (e.g.,

the z-axis). Moving the lens and/or the detector along the z-axis may focus the image of the detection region.

In some embodiments, a cartridge is coupled to a motor, actuator, or a cartridge positioning system designed to move the cartridge along the z-axis to focus an image of the detection region. A cartridge may be moved to allow more than one image of analytes to be captured in more than one detection system. For example, a cartridge contains more than one detection region. The area of interest in the detection systems may be too large to be captured with one image, thus the cartridge may be moved horizontally or in any direction along the x-y plane to obtain images of the desired areas. In some embodiments, two or more images may be captured along the z-axis and combined to form one image. For example, 5 or 10 images may be captured in 20 micron steps along the z-axis, and the images may be combined ("stitched together") to form a final image that includes analytes imaged in a series of x-y planes along the z-axis.

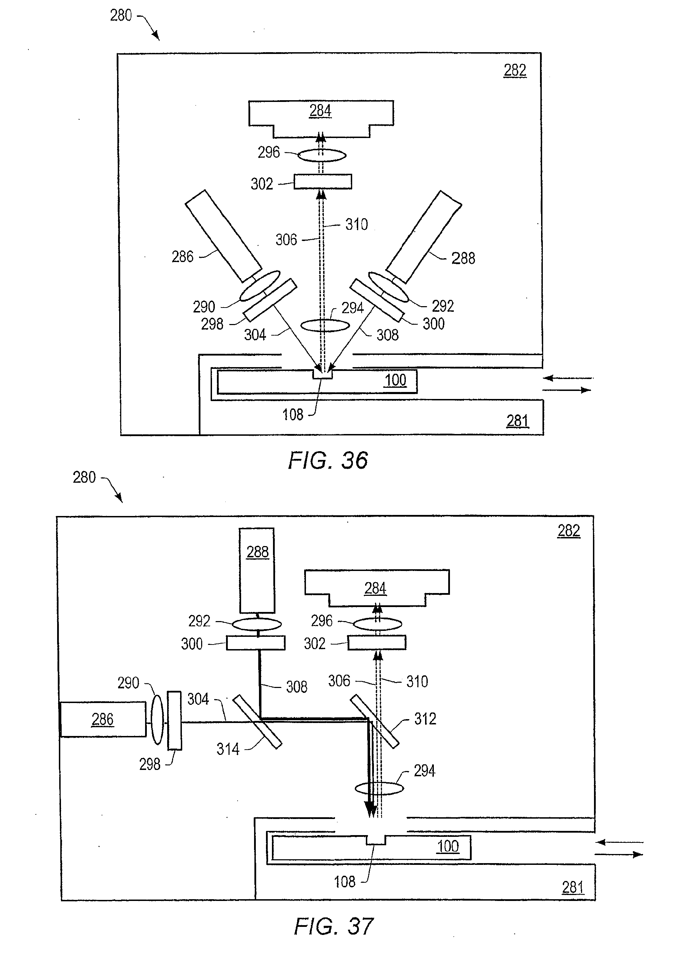

FIG. 36 depicts a cartridge positioned in an analyte detection system. Analyte detection system 280 includes cartridge 100, housing 281 and optical platform 282. Optical platform 282 includes detector 284, light sources 286, 288, lenses 290, 292, 294, 296 and filters 298, 300, 302. Cartridge 100 may be positioned automatically and/or manually in housing 281. Light 304 (e.g., white light) from light source 286 may be collimated with lens 290, filtered to a desired wavelength using filter 298 (e.g., filtered to a wavelength in a green portion of visible light), and directed toward a detection system positioned in detection region 108 of cartridge 100. In some embodiments, light from a light source may enter the cartridge at an angle. For example, the light source may be positioned at a 45° angle with respect to the detector and/or the cartridge. Filter 298 (e.g., excitation filters and/or emission filters) may be used to narrow excitations from light emitting diodes and/or other light sources. For example, filter 298 may be a D467/20x filter capable of filtering light to a wavelength ranging from about 450 nm to about 480 nm (e.g., 457 nm to about 477 nm). Filter 300 may be a 635/20x filter capable of filtering light to a wavelength ranging from about 625 nm to about 645 nm.

After light 304 is directed toward detection region 108, light 306 (e.g., signal) produced from interaction of the analyte with the light may then be obtained using detector 284. The signal may be transformed into an image representing the desired analyte. In some embodiments, the image represents a microsieve of the detection system and/or one or more analytes in the fluid and/or sample. Detector 284 may include, but is not limited to, a digital detector, a CMOS camera, or a CCD device. In some embodiments, moving the optical platform along the axis perpendicular to the cartridge while the cartridge is held in one place allows images of the cartridge to be brought into focus for the detector. Emission filter 302 may be used with detector 284. For example, light 306 reflected from the detection region 108 may pass through lens 294 and/or an emission filter 302. Lens 296 is used to collimate light 306 from detection region 108 and/or focus the light from the detection region to detector 284. Emission filter 302 may be a dual band emission filter that allows transmission between about 504 nm and about 569 nm and between about 670 nm and about 822 nm.

Next, light 308 from light source 288 is collimated with a lens 292, filtered to a desired wavelength with filter 300, and focused on a sample. Emitted light 310 produced by interaction of the analyte with the sample and emitted from detection region 108 passes through lens 294 and/or emission filter 302 and is collimated with lens 296 to detector 284. Detector 284 captures the signal from illumination of detection region 108 with light source 288. Emitted light 310 is transformed into an image representing an image of the detection region. It should be understood that additional light sources (e.g., a third light source, a fourth light source, a fifth light source, etc.) may also be used. Signals produced from the detection region may then be processed to produce images of a portion of the detection region (e.g., a portion of a microsieve) and/or of analytes present in the sample. In some embodiments, an analyzer determines the identity and/or presence of the analytes.

FIG. 37 depicts an alternative arrangement for analyte detection system 280. Optical platform 282 includes light sources 286, 288. Light sources 286, 288 emit light in a range from about 460 nm to about 480 nm, from about 465 nm to about 475 nm, or from about 460 nm to about 470 nm. During use, detection region 108 of cartridge 100 may be positioned automatically or manually in housing 281. Detection region 108 contains one or more detection systems (e.g., a microsieve-based detection system and/or a particle-based detection system). The detection system includes at least one sample and at least one detectable label. Light 304 from first light source 286 is collimated with lens 290, filtered to a desired wavelength using filter 298, reflected 90 degrees by dichroic mirror 312, and focused on a detection system in detection region 108 with lens 294. In some embodiments, the dichroic mirror is a combination of dichroic mirrors. The dichroic mirror may include one or more reflection bands and/or one or more transmission bands. For example, dichroic mirror 312 may be a Z502RDC long pass dichroic mirror, which is a dual band dichroic mirror having 2 reflection bands and 2 transmission bands. One reflection band of a dichroic mirror may reflect light at a wavelength ranging from about 463 nm to about 483 nm and transmit light ranging from about 502 nm to about 587 nm. A second reflection band of the dichroic mirror may reflect light at a wavelength ranging from 603 nm to about 637 nm and transmit light at a wavelength ranging from about 656 nm to about 827 nm.

Light 306 reflected and/or emitted from detection region 108 passes through lens 294 and is filtered to selected wavelengths with filter 302 (e.g., a dual band emission filter), collimated with lens 296, and processed by detector 284 to produce an image of the detected analytes.

Light 308 from second light source 288 is collimated with lens 292, filtered to a desired wavelength with filter 300. Filter 300 is a different filter than filter 298, thus light 308 has a different wavelength than light 304. Filtered light 308 is reflected 90 degrees by dichroic mirror 314, reflected 90 degrees by dichroic mirror 312, and focused on or in detection region 108 using lens 294. Light 310 reflected and/or emitted from detection system 108 passes through lens 294 and dichroic mirror 312, is filtered to predetermined wavelengths with filter 302, is collimated by lens 296, and processed by detector 284 to produce an image of the detected analytes. Filter 302 may be a dual band emission filter capable of filtering light at two different ranges of wavelengths (e.g., a first wavelength from about 504 nm to about 569 nm and a second wavelength from about 607 nm to 822 nm).

The signal obtained by detector 284 may then be analyzed to determine the presence and/or identity of analytes in the detection region. Any number of light sources may be used in a similar manner as described above. It may be desirable to use a plurality of light sources to substantially simultaneously detect a plurality of analytes.

In some embodiments, a single light source with a beam splitter is used instead of multiple light sources. Using one excitation source may reduce costs. The single light source may excite two or more detectable labels applied to matter captured on a microsieve of a detection system of a cartridge. The emission of light from the detection system may be separated using one or more dichroic mirrors and one or more detectors.

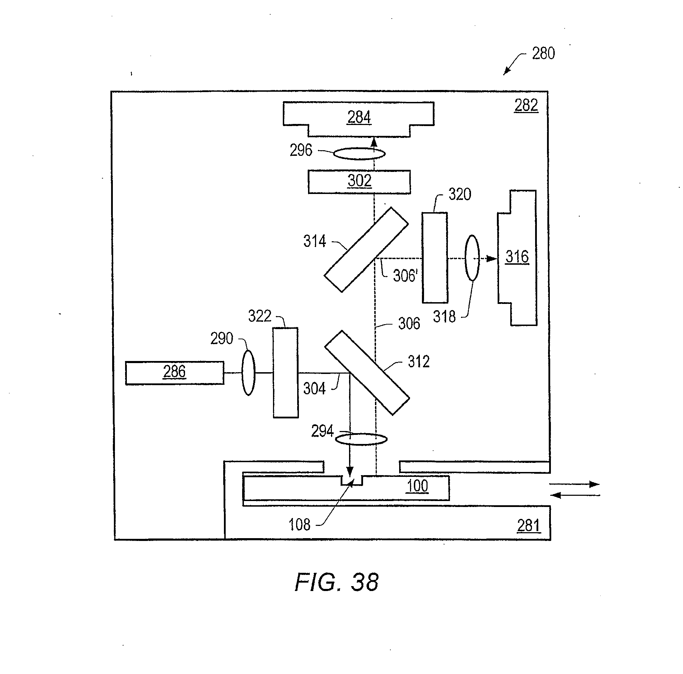

FIG. 38 is a schematic of a cartridge positioned in an analyte detection system with an optical platform that includes a single light source. Analyte detection system 280 includes cartridge 100, housing 281, and optical platform 282. Optical platform 282 includes detectors 284, 316, light source 286, lenses 290, 294, 296, 318, filters 302, 320, dichroic mirrors 312, 314 and shutter 322.

Light 304 from single source 286 is collimated with lens 290, passed through shutter 322, reflected 90 degrees by dichroic mirror 312, and focused on detection region 108 of cartridge 100 with lens 294. Shutter 322 is positioned between lens 290 and dichroic mirror 312. Shutter 322 may block light from shining on detection region 108 and/on cartridge 100. Light 306 reflected and/or emitted from a detection system of detection region 108 may pass through lens 294, dichroic mirrors 312, 314, filter 302, and lens 296 where light 306 is collimated onto detector 284. A portion of light 306, depicted as light 306′, may be reflected using dichroic mirror 314, pass through filter 320 (e.g., a dual band emission filter), and lens 318 where light 306′ is collimated onto detector 316.

In some embodiments, an actuator is used to move a series of different emission filters into the path of light entering a detector. The ability to use different emission filters allows more than one signal from the detection region of the cartridge to be analyzed by one detector. The use of one detector and more than one filter may enhance the sensitivity of a test process, allowing less sample to be used for an analysis of multiple analytes. Determination of the appropriate emission filters to position in front of the detection system may be based on data obtained from a barcode located on the cartridge.

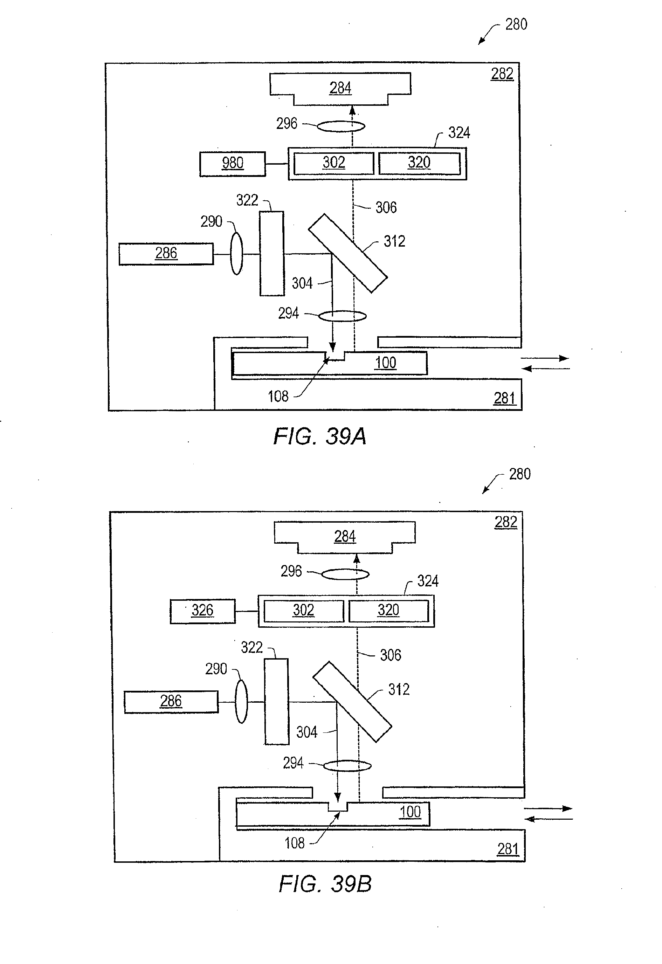

FIG. 39A is a schematic diagram of a cartridge positioned in an analyte detection system that includes an optical platform equipped with an actuator. The actuator is designed to position a series of filters in front of a detector. Analyte detection system 280 includes cartridge 100, housing 281, and optical platform 282. Optical platform 282 includes detector 284, light source 286, lenses 290, 294, 296, dichroic mirror 312, shutter 322, filter holder 324, filters 302, 320, and actuator 326. Light 304 from light source 286 is collimated with lens 290, passed through shutter 322, reflected 90 degrees by dichroic mirror 312, and focused onto detection region 108 of cartridge 100 with lens 294. Light 306 reflected and/or emitted from a detection region 108 may pass through lens 294, dichroic mirror 312, filter 302 or filter 320 positioned in filter holder 324, and lens 296 where light 306 is collimated onto detector 284. Filter holder 324 may include additional emission filters depending on the analyte to be analyzed. Filter holder 324 is coupled to actuator 326, which is designed to move filter holder 324. Actuator 326 may move filter holder 324 based on a signal from detector 284 and/or an analyzer of analyte detection system 280. Filter holder 324 may be positioned between cartridge 100 and detector 284. In some embodiments, actuator 326 may move filter holder 324 such that filter 320 may be positioned between detector 284 and detection region 108 such that light 306 may pass filter 320 and into detector 284, as shown in FIG. 39B, allowing analysis of the detection region using a different wavelength of light. The filter light (e.g., filtered signal) may then be analyzed in the detector to produce an image and/or data of analytes in the fluid and/or sample. A plurality of images and/or data from the fluid and/or sample may be obtained using a plurality of emission filters placed sequentially in front of the detector.

Analyte detection systems described herein may be used to identify the presence of a plurality of analytes in a sample. Analyte detection systems may be designed for detection of one or more specific analytes (e.g., cellular components, proteins, or pathogens such as viruses, bacteria, fungi or parasites, or combinations thereof) typically associated with various infections, diseases, illnesses, and/or syndromes. Examples of diseases, illnesses, viruses and syndromes include, but are not limited to, HIV/AIDS, malaria, cardiovascular disease, atherosclerosis, cancer, influenza, hepatitis, tuberculosis, mononucleosis, syphilis, sickle-cell anemia, herpes virus, HIV, Good's syndrome, or Sjogren's syndrome. Examples of viruses include, but are not limited to, Epstein-Barr virus (EBV), cytomegalovirus (CMV), herpes simplex viruses 1 and 2 (HSV1, HSV2), varicella-zoster virus (VZV), Kaposi's sarcoma-related virus (HHV8), HTLV, herpes lymphotropic virus (HHV6), and human herpes virus 7 (HHV7).

Analysis of human blood samples may allow for early detection of various diseases, illness, viruses and/or syndromes. For example, WBCs and RBCs may be separated and analyzed to determine specific diseases, illnesses, viruses, and/or syndromes. In some embodiments, WBCs are separated from RBCs and immunotyped to determine the total number of various cell types in a sample and/or their ratio relative to other cell types. A three- or five-part WBC differential, which is part of a typical complete blood count, may be used for general illness assessment. A three- or five-part WBC differential may sort out results based on counts of various white blood cells in various classes of diseases and may be used to diagnose viral, bacterial, allergic, and immune diseases.

Samples may be analyzed by characterizing one or more components of a blood sample, including the fluid component of whole blood, such as serum or plasma. Samples may also be analyzed by characterizing one or more solid components of a blood sample. Solid components of a blood sample may include, but are not limited to, blood cells, platelets, or pathogenic organisms (e.g., bacteria, viruses, fungi, or blood-borne parasites).

In some embodiments, the cellular components of a sample may be characterized by detecting the presence and/or expression levels of one more molecular groups (e.g., polypeptides, polynucleotides, carbohydrates, lipids) typically known to be associated or correlated with a specific trait for which the test is being performed. For example, a blood sample may be collected to measure the number of cells of one or more specific cell types present in the sample (commonly referred to in the art as "cell counts"), and/or the ratio thereof with respect to one or more different cell types or sub-types also present in the sample. Examples of the types of blood cells that may be detected in a blood sample include, but are not limited to, erythrocytes, lymphocytes (e.g., T cells and B cells), Natural Killer (NK) cells, monocytes/ macrophages, megakaryocytes, platelets, eosinophils, neutrophils, basophils or mast cells. In some embodiments, various sub-populations of specific cell types within a fluid sample are distinguished. For example, the T cells present in a blood sample may be further categorized into helper (CD4$^+$), cytotoxic (CD8$^+$), memory (CD4$^+$/CD8$^+$ and/or CD45RO) or suppressor/regulatory (CD4$^+$CD25$^+$/FOXP3$^+$) T cells. Alternatively, B cells present in a blood sample may be further categorized into populations of immature, mature, activated, memory, or plasma cells, based on the immunoglobulin isotype expressed on the cell surface, and presence or absence of various additional proteins.

Table I summarizes the surface expression profile of a selection of non-limiting protein markers that may be used to classify the stage of B cell differentiation, where filled circles denote expression, open circles denote lack of expression, and partially filled circles denote partial or limited expression of the indicated surface marker. The presently described systems and methods are not limited to detecting the cell types disclosed in Table I. It should be understood, that the presently disclosed systems and methods may be suitably adapted to analyze most cell types and/or macromolecules present in a biological sample without departing from the spirit and scope of the presently described embodiments.

**Table I.**

| B cell stage | Surface Immunoglobulin isotype | | | Marker protein | | | | | |
|---|---|---|---|---|---|---|---|---|---|
| | IgM | IgG or IgA | IgD | CD23 | PCA-1 | CD38 | CD25 | CD10 | CD19 |
| Pre B | O | O | O | O | O | ● | O | ◉ | ● |
| Immature | ● | O | O | ◉ | O | O | ◉ | O | ● |
| Mature | ● | O | ● | ● | O | O | ● | O | ● |
| Activated | ● | ● | O | ● | O | O | ● | ● | ● |
| Memory | O | ● | O | O | O | O | O | O | ? |
| Plasma cell | O | O | O | O | ● | ● | O | O | O |

Analysis of a cellular composition of a sample may include detecting the presence of one or more "surface markers" known to be expressed on the surface of the population of cells of interest. Certain surface markers useful in the differential identification of cells in a sample (e.g., in particular cells involved in immune responses) and/or diseases are commonly referred to as "cluster of differentiation" (CD) antigens or CD markers, of which over 250 have been characterized. Many of the CD antigens may also be referred to by one or more alternative terms. Table II lists several examples of CD antigens, and the cells in which they are expressed, that may be referred to using one or more alternative terms. It is contemplated that embodiments of the invention may involve one or more different CD antigens, Some embodiments involve at least, but are not limited to, 1, 2, 3, 4, 5, 6, 7, 8, 9, 10 or more of the CD markers discussed herein. The system of CD marker nomenclature is widely recognized by ordinary practitioners of the art. General guidance in the system of CD marker nomenclature, and the CD expression profiles of various cells is found in most general immunology reference textbooks such as, for example, in IMMUNOLOGY, 4th Edition Ed. Roitt, Brostoff and Male, Chapter 28 and Appendix II (Mosby/Times Mirror International Publication 1998), or in IMMUNOBIOLOGY: THE IMMUNE SYSTEM IN HEALTH AND DISEASE, 5th Edition, Eds. Janeway et al. Appendices I-IV (Garland Publishing, Inc. 2001).

**Table II.**

| CD Antigen | Identity/function | Expression |
|---|---|---|
| CD2 | T-cell adhesion molecules | T cells, NK cells |
| CD3 | T cell binding agent ($\gamma,\delta,\varepsilon,\zeta,\eta$) | Thymocytes, T cells |
| CD4 | MHC class II binding agent | Thymocyte subsets, T helper cells, monocytes, macrophages |
| CD8 | MHC class I binding agent | Thymocytes subsets, cytotoxic T cells |
| CD10 | Neutral endopeptidase/CAALA | T and B cell precursors, activated B cells, granulocytes |
| CD11a | Integrin $\alpha$ | Lymphocytes, granulocytes, monocytes and macrophages |
| CD11b | Integrin $\alpha$ | Myeloid and NK cells |
| CD13 | Aminopeptidase N | Monocytes, granulocytes |
| CD16 | Fc$\gamma$RIIIA/ B | Neutrophils, NK cells, macrophages |
| CD19 | B cell function/activation | B cells |
| CD20 | Ca$^{2+}$ ion channel | B cells |
| CD21 | C3d and EBV binding agent | Mature B cells |
| CD35 | Complement binding agent 1 | Erythrocytes, B cells, monocytes, neutrophils, eosinophils |
| CD41 | $\alpha$IIb integrin | Platelets, megakaryocytes |
| CD45RO | Fibronectin type II | T-cell subsets, B cell subsets, monocytes, macrophages |
| CD45RA | Fibronectin type II | B cells, T-cell subsets (naive T cells), monocytes |
| CD45RB | Fibronectin type II | T-cell subsets, B cells, monocytes, macrophages, granulocytes |
| CD56 | NKH-1 | NK cells |

In some embodiments, the presently described analyte detection systems and methods may be used to analyze blood samples on the basis of the expression profile or presence of one or more macromolecules (e.g., proteins, phosphoproteins, glycoproteins, polynucleotides, or variants or isoforms thereof) that are indicative or prognostic of certain pathological states. Types of analytes that may be useful diagnostic or prognostic indicators and whose plasma or cellular expression levels are correlated with various diseases, illnesses, viruses, and/or syndromes include, but are not limited to, chemokine binding agent 5 (CCR5), viral DNA or RNA sequences, certain species of plasma RNA, interferon-gamma (IFN-$\gamma$), virus particles, early secreted antigenic target protein-6 (ESAT-6), culture filtered protein-10 (CFP-10), C-reactive protein (CRP), troponin-I, and TNF-$\alpha$.

In some embodiments, an analyte detection system may be used for prognostic tests for HIV seropositive patients. HIV infects CD4$^{+}$ cells (e.g., certain populations of T helper cells, monocytes, and macrophages) by binding to a co-binding agent CCR5. The expression level of certain CCR5 variants in CD4$^{+}$ cells has been shown to correlate with viral load and progression to AIDS. The presently described analyte detection systems and methods may be used to, for example, monitor CCR5 expression in CD4$^{+}$ cells in patient blood samples. This parameter may advantageously be measured simultaneously from a single sample with one or more measures of HIV viral load. In some embodiments, the tests described herein may further measure one or more blood parameters associated with other pathological situations in addition to, or alternatively to, HIV infection.

In certain embodiments, an analyte detection system may be used to diagnose tuberculosis (TB). In some embodiments, an analyte detection system may be used to detect reductions in systemic CD3$^{+}$ and CD4$^{+}$ cells that typically occur in TB patients. This parameter may be measured alone or in combination with the detection of one or more soluble proteins typically elevated in TB patients (such as IFN-$\gamma$), the mycobacterial proteins ESAT-6, CFP-10, or T cell populations that are reactive to ESAT-6 and CFP-10. Such applications may be particularly suited to certain point-of-care settings and/or in resource scarce countries where HIV and TB comorbidity are common.

In some embodiments, an analyte detection system as described herein may be used to diagnose viral infections in addition to HIV. Blood samples from both Epstein-Barr virus (EBV) and cytomegalovirus (CMV) infected patients exhibit increases in percentages of total T cells, suppressor T cells and activated HLA-DR$^{+}$ T cells when compared with healthy, uninfected people. Additionally, as seen in HIV-infected patients, individuals infected with EBV and/or CMV typically display significantly decreased levels CD4[+] T cells as well as a decrease in the ratio of CD4[+]/CD8[+] T cells. Blood samples from individuals infected with EBV may also exhibit elevated levels of NK cells.

The analyte detection systems described herein may, in some embodiments, be adapted to readily, reproducibly, and cost effectively diagnose a variety of maladies endemic to geographic and/or economically disadvantaged regions. An example of such an application is point-of-care diagnosis of malaria in geographic areas such as, for example, Africa, Latin America, the Middle East, South and Southeast Asia, and China. Currently, reliable diagnosis of malaria is time consuming, labor intensive, and typically involves identifying erythrocytes harboring *Plasmodium* parasites. Identification of such cells is typically made by microscopic examination of uncoagulated Giemsa-stained blood samples, possibly in combination with one or more serological and/or molecular diagnostic tests (e.g., polymerase chain reaction), all of which require highly specialized equipment. In some embodiments, analyte detection systems described herein may be sued to detect one or more *Plasmodium*-specific antigens that include, but are not limited to, panmalarial antigen (PMA), histidine-rich protein 2 (HRP2) and parasite lactate dehydrogenase (pLDH) in a blood sample. In some embodiments, the analyte detection systems presently described may be used to monitor one or more physiological parameters associated with malaria. For example, a portion of the hemoglobin from *Plasmodium*-parasitized erythrocytes forms lipidized pigment granules generally referred to as "hemozoin." Phagocytosed hemozoin impairs monocyte/macrophage and hence immune function, at least in part, by reducing the surface expression of MCH class II, CD11c and CD54 in phagocytes. Additionally, low peripheral blood monocyte counts may be associated with patients with severe and complicated malaria. Analyte detection systems described herein may be used to detect and monitor the presence and/or quantities of these physiological parameters associated with malaria.

In some embodiments, analyte detection systems described herein may be used to diagnose Good's syndrome, an immunodeficiency disorder secondary to thymoma and characterized by deficiencies of cell-mediated immunity and T-cell lymphopenia.

In some embodiments, an analyte detection system may be used to identify certain biological markers associated with increased susceptibility to various pathological conditions (e.g., cardiovascular disease, atherosclerosis, inflammation, and/or certain types of cancer). Inflammation has been identified as an underlying cause of atherosclerosis, a condition associated with the deposition of lipids on the lining of arteries that may progressively lead to serious vascular complications such as myocardial infarction (MI) and/or stroke. By measuring the concentration of certain proteins associated with inflammation (e.g., CRP) either alone or in conjunction with cellular profiles (e.g., WBC count), the presently described analyte detection systems may be used to screen individuals at risk for heart attack, atherosclerosis, or other vascular diseases. Likewise, MI patients with elevated CRP levels or WBC counts are at higher risk for subsequent cardiovascular events. Diagnostic and prognostic tests that provide measurements for these two important biological parameters associated with inflammation and vascular disease may provide powerful diagnostic and prognostic insight, allowing healthcare providers to make timely and appropriate therapeutic interventions. For example, it is recognized by practitioners of the art that individuals having elevated WBC counts and blood CRP levels have a greater risk for heart disease than individuals having WBC counts and CRP levels within normal range.

A low peripheral monocyte count in individuals with high cholesterol is generally predictive of increased risk for developing atherosclerosis. The presently described analyte detection systems may be readily and advantageously adapted to measure monocyte counts (CD13[+]CD14[+]CD45RA) associated with cardiac risk factors. Monocyte counts are also an important physiological parameter in subjects with hypercholesterolemia. Analyte detection systems described herein may also be used to measure the amounts of other cardiac risk factors such as troponin I and/or TNF-$\alpha$.

Low blood CD8[+] cells and high circulating monocytes have been associated with progressive encephalopathy (PE) (Sanchez-Ramon et al., Pediatrics, 111(2):E168-75). PE is one of the most common complications of HIV infection in children. As antiretroviral drugs become more readily available, the number of children with PE has increased, thus it is desired to evaluate risk factors for PE. CD8 labeled cells may be identified using an analyte detection system to monitor the progress of PE.

An analyte detection system for use in diagnostic and prognostic applications to specific pathologies, such as for example, those described above, may further allow a user of the system to readily identify characteristics in a sample that are associated with the malady. The analyte detection system may include, for example, various binding agents (such as specific antibodies) that bind to cell surface markers (e.g., CD markers or other disease-associated molecules) or any other analyte suspected to be present in a sample that allows rapid characterization of the sample. In some embodiments, one or more antibodies (e.g., monoclonal and/or polyclonal antibodies) that specifically recognize and bind to macromolecules expressed on the surface of cells (e.g., CD or other cell surface markers) may be used in an analyte detection system.

While certain specific examples of monoclonal or polyclonal antibodies are set forth above, it will be readily understood by ordinary practitioners of the art that the presently described analyte detection systems may be used, without limitation, in conjunction with any type of antibody that recognizes any antigen including, but not limited to, commercially available antibodies or antibodies generated specifically for the purpose of performing the tests described herein. Monoclonal and polyclonal antibody design, production and characterization are well-developed arts, and the methods used therein are widely known to ordinary practitioners of the art (see, e.g., "Antibodies: A Laboratory Manual," E. Howell and D. Lane, Cold Spring Harbor Laboratory, 1988). For example, a polyclonal antibody is prepared by immunizing an animal with an immunologically active composition including at least a portion of the macromolecule to which the desired antibody will be raised and collecting antiserum from that immunized animal. A wide range of animal species may be used for the production of antiserum. Examples of animals used for production of polyclonal anti-sera include rabbits, mice, rats, hamsters, horses, chickens, and guinea pigs.

A monoclonal antibody specific for a particular macromolecule can be readily prepared through use of well-known techniques such as those exemplified in US4,196,265, to Koprowski et al.. Typically, the technique involves first immunizing a suitable animal with a selected antigen (e.g., at least a portion of the macromolecule against which the desired antibody is to be raised) in a manner sufficient to provide an immune response. Rodents such as mice and rats are preferred species for the generation of monoclonal antibodies, although rabbits are also used. An appropriate time after the animal is immunized, spleen cells from the animal are harvested and fused, in culture, with an immortalized myeloma cell line.

The fused spleen/myeloma cells (referred to as "hybridomas") are cultured in a selective culture medium that preferentially allows the survival of fused splenocytes. After the fused cells are separated from the mixture of non-fused parental cells, populations of B cell hybridomas are cultured by serial dilution into single-clones in microtiter plates, followed by testing the individual clonal supernatants for reactivity with the immunogen. The selected clones may then be propagated indefinitely to provide the monoclonal antibody of interest. In some embodiments, a microsieve-based detection system for use in performing WBC counts on a blood sample may use one or more polyclonal or monoclonal antibodies that specifically recognize various cell types that constitute WBCs to visualize specific blood cells. Antibodies suitable for this purpose include, but are not limited to: anti-CD3; anti-CD4; anti-CD8; anti-CD16; anti-CD56; and/or anti-CD19 antibodies to specifically recognize: T cells; T helper cells and monocytes/macrophages; cytotoxic T cells; neutrophils, NK cells and macrophages; NK cells; and B cells, respectively.

If desired, dimers or multimers binding agents may be employed, such as dimeric polypeptides, and the detectable label may be joined *via* a biologically releasable bond, such as a selectively cleavable linker or amino acid sequence. Amino acids such as selectively cleavable linkers, synthetic linkers, or other amino acid sequences may be used to separate binding domains or the binding agent and the detectable label.

Cross-linking reagents are used to form molecular bridges that tie together functional groups of two different molecules, e.g., a stablizing and coagulating agent. To link two different proteins in a step-wise manner, hetero-bifunctional cross-linkers can be used that eliminate unwanted homopolymer formation.

The SMPT cross-linking reagent, as with many other known cross-linking reagents, lends the ability to cross-link functional groups such as the SH of cysteine or primary amines (*e.g.*, the epsilon amino group of lysine). Another possible type of cross-linker includes the hetero-bifunctional photoreactive phenylazides containing a cleavable disulfide bond such as sulfosuccinimidyl-2-(p-azido salicylamido) ethyl-1,3'-dithiopropionate. The N-hydroxy-succinimidyl group reacts with primary amino groups and the phenylazide (upon photolysis) reacts non-selectively with any amino acid residue.

In addition to hindered cross-linkers, non-hindered linkers also can be employed in accordance herewith. Other useful cross-linkers, not considered to contain or generate a protected disulfide, include SATA, SPDP and 2-iminothiolane (Wawrzynczak & Thorpe, 1987). The use of such cross-linkers is well understood in the art.

In some embodiments, a microsieve-based detection system is used to assess both CD4 cell count and CD4 cells as a percentage of total lymphocytes from a blood sample for diagnosis, staging, and/or monitoring of infections and/or diseases. For example, samples having CD4 counts below 200 cells per microliter may indicate specific drug therapy intervention. In certain embodiments, comparing CD4 cell counts to CD8, CD3, and/or CD19 cell counts may be used to assess the ratio CD4[+] T helper cells with respect to cytotoxic T cells, total circulating T cells, B cells, or combinations thereof.

In some embodiments, a sample, such as blood or diluted blood, is applied and/or transported to a microsieve of a microsieve-based detection system. The microsieve may retain portions of the sample, while allowing other portions of the sample to pass through. For example, the microsieve may be adapted to retain white blood cells, while allowing other portions of the sample, such as water or red blood cells, to pass through.

A combination of detectable labels may be applied and/or transported to the microsieve to allow a total number and/or different types of white blood cells (e.g., lymphocytes generally or T cells, NK cells, and/or B cells) to be identified. One or more detectable labels may be added to the matter collected on a surface of the detection system. For example, detectable labels may allow the detection of anti-CD3, anti-CD4, anti-CD8, anti-CD16, anti-CD56 and anti-CD19 antibodies bound to their respective CD markers on the surface of target cells. In some embodiments, anti-CD2, anti-CD4, and anti-CD19 antibodies may be coupled to the detectable label directly. In some embodiments, the detectable label may be coupled to a second macromolecule that specifically binds to and recognizes the antibody bound to the CD marker.

In some embodiments, a first detectable label may be used to label CD4[+] cells present in a mixed population of cells. Additional, distinct detectable labels may then be used to label the NK cells, B cells, and/or other T cells in the mixed population. For example, a mixed population of cells in a sample may be labeled with anti-CD4, anti-CD3, anti-CD56, and anti-CD19 antibodies to detect CD4$^+$ T helper cells, total T cells, NK cells, and B cells respectively.

In some embodiments, fluorescent dyes (e.g., ALEXA FLUOR® dyes from Invitrogen Corporation; Carlsbad, CA) may be coupled to antibodies to form fluorophore-labeled antibodies. Use of fluorophore-labeled antibodies to visualize cells may facilitate detection of the analyte. One or more fluorescent dyes may be used to label one or more cell surface markers to facilitate assessment of a desired marker percentage relative to other markers (e.g., a percentage of CD4$^+$ lymphocytes relative to other lymphocytes). An image of the cells labeled by the first detectable label may be provided and one or more additional images of cells labeled by the additional detectable labels may be provided. The images may be compared and/or combined to determine the total number of lymphocytes and/or a number of a specific type of lymphocyte in or on the microsieve. A detector optically coupled to at least a portion of the microsieve may provide the images. An analyzer may automatically compare the images during use. For example, ALEXA FLUOR® 488, which fluoresces green when exposed to light having a wavelength in a range surrounding the absorbtion maximum of about 495 nm, may be used to visualize anti-CD3 antibodies bound to the surface of all T cells present in a sample. ALEXA FLUOR® 647, which fluoresces red when exposed to light having a wavelength in a range surrounding the absorbtion maximum of about of 650 nm, may be used to visualize anti-CD4 bound to the surface of T helper cells and monocytes. In this way, at least three populations of cells (all T cells label red, T helper cells label red and green, and monocytes which label green) may be readily and simultaneously identified in a single sample.

Detectable labels may be colorimetric (includes visible and UV spectrum, including fluorescent), luminescent, enzymatic, or positron emitting (including radioactive). The label may be detected directly or indirectly. Radioactive labels include $^{125}$I, $^{32}$P, $^{33}$P, and $^{35}$S. Examples of enzymatic labels include alkaline phosphatase, luciferase, horseradish peroxidase, and β-galactosidase. Labels can also be proteins with luminescent properties, e.g., green fluorescent protein, red fluorescent protein, blue fluorescent protein, yellow fluorescent protein and phicoerythrin.

The colorimetric and fluorescent labels contemplated for use as detecatable labels include, but are not limited to, ALEXA FLUOR® dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; fluorescent energy transfer dyes, such as thiazole orange-ethidium heterodimer; and TOTAB.

Specific examples of dyes include, but are not limited to, those identified above and the following: ALEXA FLUOR® 350, ALEXA FLUOR® 405, ALEXA FLUOR® 430, ALEXA FLUOR® 488, ALEXA FLUOR® 500. ALEXA FLUOR® 514, ALEXA FLUOR® 532, ALEXA FLUOR® 546, ALEXA FLUOR® 555, ALEXA FLUOR® 568, ALEXA FLUOR® 594, ALEXA FLUOR® 610, ALEXA FLUOR® 633, ALEXA FLUOR® 647, ALEXA FLUOR® 660, ALEXA FLUOR® 680, ALEXA FLUOR® 700, and, ALEXA FLUOR® 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Specific examples of fluorescently labeled ribonucleotides are available from Molecular Probes, and these include, ALEXA FLUOR® 488-5-UTP, Fluorescein-12-UTP, BODIPY FL-14-UTP, BODIPY TMR-14-UTP, Tetramethylrhodamine-6-UTP, ALEXA FLUOR® 546-14-UTP, Texas Red-5-UTP, and BODIPY TR-14-UTP. Other fluorescent ribonucleotides are available from Amersham Biosciences, such as Cy3-UTP and Cy5-UTP.

Examples of fluorescently labeled deoxyribonucleotides include Dinitrophenyl (DNP)-11-dUTP, Cascade Blue-7-dUTP, ALEXA FLUOR® 488-5-dUTP, Fluorescein-12-dUTP, Oregon Green 488-5-dUTP, BODIPY FL-14-dUTP, Rhodamine Green-5-dUTP, ALEXA FLUOR® 532-5-dUTP, BODIPY TMR-14-dUTP, Tetramethylrhodamine-6-dUTP, ALEXA FLUOR® 546-14-dUTP, ALEXA FLUOR® 568-5-dUTP, Texas Red-12-dUTP, Texas Red-5-dUTP, BODIPY TR-14-dUTP, ALEXA FLUOR® 594-5-dUTP, BODIPY 630/650-14-dUTP, BODIPY 650/665-14-dUTP; ALEXA FLUOR® 488-7-OBEA-dCTP, ALEXA FLUOR® 546-16-OBEA-dCTP, ALEXA FLUOR® 594-7-OBEA-dCTP, ALEXA FLUOR® 647-12-OBEA-dCTP.

It is contemplated that analytes may be labeled with two different labels. Furthermore, fluorescence resonance energy transfer (FRET) may be employed in methods of the invention (e.g., Klostermeier *et al.*, 2002; Emptage, 2001; Didenko, 2001).

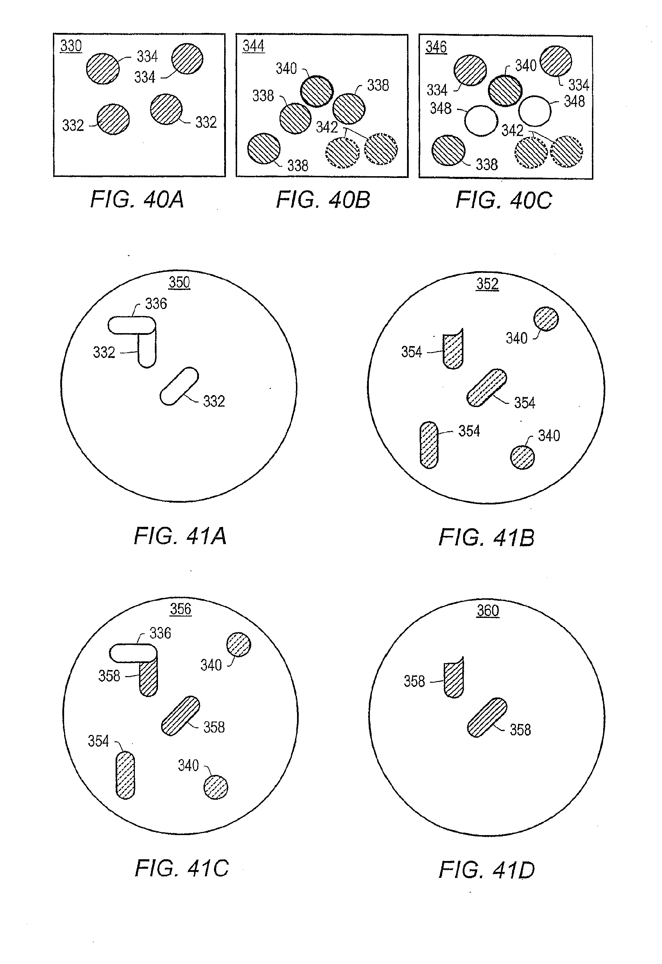

In some embodiments, two fluorophores and two light sources are used to determine types of white blood cells. The analyte detection systems depicted in FIGS. 36-39 may be used, for example, to determine type of white blood cells. FIGS. 40A- 40C depict representations of images collected using two fluorophores and two light sources. For example, a green fluorophore (e.g., ALEXA FLUOR® 488) may be coupled to anti-CD4 antibodies of a sample. A red fluorophore (e.g., ALEXA FLUOR® 647) may be coupled to the anti-CD56 antibodies, anti-CD3 antibodies, and anti-CD19 antibodies added to the sample. As discussed above and shown in Tables I and II, CD4 is expressed on the surface of T helper cells and monocytes, CD19 is expressed on the surface of B cells, CD56 is expressed on the surface of NK cells, and CD3 is expressed on T cells. Analysis of the samples captured on a microsieve using two wavelengths of light may allow differentiation of the types of WBCs captured.

FIG. 40A depicts a representation of image 330 of green cells 332, 334 obtained by exciting the green fluorophore detectable label with a light source, analyzing the signal generated by the excitation, and producing an image of the cells. Green cells 332, 334 represent CD4$^+$ cells.

FIG. 40B depicts a representation of an image of red cells obtained by exciting the red fluorophore, analyzing the signal produced from excitation, and producing an image of red cells. Red cells 338, 340, and 342, visible in image 344, represent cells expressing CD3, CD19, and CD56, respectively.

Cells that are visible in images 330 and 344 represent CD4$^+$ lymphocytes. Thus, monocytes (e.g., cells that only emit green light) may be identified. Combining image 330 and image 344 creates image 346 that includes green cells 334, red cells 338, 340, 342, and cells 348 that are labeled both green and red, as shown in FIG. 40C. Green cells 334 are representative of CD4$^+$CD3$^-$CD19$^-$ cells. CD56- cells 348 that appear in both images are representative of CD4$^+$CD3$^+$ T helper cells.

A total number of T helper cells (cells that express CD4 and CD3 and label both red and green), a total number of lymphocytes (cells that express CD3, CD19 or CD56 and label red), a total number of CD4 cells (cells that label green), and a ratio of (CD4$^+$ + CD3$^+$) cells to a total number of lymphocytes may all be determined from the combination of images 330, 344, 346. A total number of lymphocytes may be obtained from the combined image, as depicted in image 346, since the cells may be identified and isolated (e.g., cells that only emit green light or only emit red light).

An absolute number of CD4$^+$ T helper cells is the total number of cells 348 that are appear in both images. A ratio of CD4$^+$ T helper cells to the total number of cells may be calculated by dividing the number of cells 348 (CD4$^+$CD3$^+$) by red cells 338, 340, 342 (CD3$^+$, CD16$^+$, CD56$^+$, or CD19$^+$).

The ratio of T helper cells to total lymphocytes may be important in determining the progression of diseases, such as HIV, and in the treatment and monitoring of other diseases. Although green and red fluorophores were described, fluorophores of any color may be used without limitation.

In some embodiments, use of one or more detectable labels allows identification of lymphocytes retained on a microsieve of a microsieve-based detection system. The lymphocytes may contain cell surface markers CD4, CD3, and CD 19. Identification of CD4 and CD3 and on the surface of cells identifies T helper cells. FIGS. 41A through 41D represent images of cells expressing CD4, CD3, and CD19 markers in the presence of two excitation sources.

FIG. 41A depicts an image of cells obtained by excitation of a green fluorophore attached to cells expressing CD4. An excitation source may excite green fluorophores and a detector may analyze the signal produced during excitation and produce image 350 of green cells 332, 336.

FIG. 41B depicts an image of cells obtained by excitation of a red fluorophore attached to cells expressing CD3 or CD19. An excitation source excites red fluorophores bound to the cells and a detector analyzes the signal produced during excitation and produces image 352 of cells 340 containing CD19 and cells 354 containing CD3.

Image 350 may be combined with image 352 to produce image 356 in which green cells 336, red cells 354, 340 and cells labeled with both red and green 358 are visible. The total number of lymphocytes may be obtained from the combined image of cells labeled red, green or both, as depicted in FIG. 41C. The total number of T helper cells present on the microsieve is identifiable by determining the number of cells expressing both CD3 and CD4.

In some embodiments, a filter allows a desired wavelength of light to pass from the detection system to the detector. Using a filter may facilitate identification of one or more types of white blood cells and/or other types of matter.

While a system to identify T cell populations based on differential labeling of CD3, CD4, and CD19 markers on cells is described above, it is understood that any combination of CD markers may be used to identify one or more types of white blood cells and/or total lymphocytes in a sample.

In some embodiments, all cells except a lymphocyte of interest may be labeled. A white light image of the microsieve may be provided. One or more additional images may be provided in which cells labeled with one or more detectable labels are visible. The number of a specific lymphocyte population may be obtained by assessing the number of cells appearing in the first image (e.g., the white light image) but not appearing in the additional images (e.g., images in which only labeled cells appear). For example, a sample containing lymphocytes may be retained on a microsieve of an analyte detection system. A first image at a selected wavelength of light of the retained cells is taken. One or more detectable labels may be applied to the retained cells. At least one of the detectable labels attaches to part of the retained cells, but not CD4$^+$ cells. A second image at one or more wavelengths different from the wavelength for the first image is taken. Such "negative selection" or "substraction" strategies may be employed to determine the number of cells that are depicted in the first image but are not depicted in the second image, for instance, to give the number of CD4$^+$ lymphocytes. Such strategies may be particularly suited to applications where additional functional analyses are performed on the cell of interest. For example, it is known in the art that contacting certain CD markers (e.g., CD3, CD19) with certain antibodies (commonly referred to as "cross-linking antibodies") causes profound changes in cellular physiology. Therefore, the negative selection strategy outlined above may be useful when additional biological/functional analyses are to be performed on a particular cell type.

In some embodiments, it is contemplated that cells expressing one CD antigen may be labelled with one color while cells expressing a different CD antigen are labeled with a second (different) color. For instance, cells expressing CD4 may be labeled red and cells expressing CD45 may be labeled green.

In certain embodiments, cells labeled because of a certain surface marker may label brighter than cells labelled because of another surface marker. For example, labeled CD45 cells may appear brighter than labeled CD4$^+$ cells. A percentage of CD4 to total lymphocytes may be determined from the ratio of CD4$^+$ cells to brighter labeled CD45 cells.

It may be desirable to label various cell subtypes differentially to allow discrimination between various cell types even when the cells are labeled with the same antibodies having the same color tag. For example, the CD4$^+$ monocyte population may be differentiated from the CD4$^+$ lymphocyte population. Monocytes may exhibit a weaker label with CD4 antibodies, which allows monocytes to be distinguished from CD4 T cells, which are characterized by a strong label with CD antibodies. Low and high intensity CD4$^+$ cells may be extracted from images of the detection system obtained by a detector. Weakly labeled CD4$^+$ cells may then be labeled with a CD 14 label that identifies weakly labeled CD4$^+$ cells as monocytes. It may be desirable to simultaneously obtain a CD4 percentage and monocyte count from a sample based on differential labeling between subtypes.

Similar principles may be applied to other subsets of the lymphocyte population. A difference in the labeling of NK cells, B cells, and T cells due to the number of surface markers, antibody affinity, or antibody performance may identify a CD8$^+$ or other population. CD8$^+$ monitoring and/or a ratio of CD4$^+$ to CD8$^+$ cells may be important in providing information about the progression of certain diseases such as, for example, HIV progression and AIDS.

Differences in surface marker concentrations on cells may provide a tool for discrimination between cells. In some diseases, cell morphology may be correlated with disease states. Images from assay screening may provide information about the assay and cell morphology and may provide additional information about the disease. For example, the malaria antibody may be localized on a part of the cell to allow a difference in intensity across a cell to be observed. This difference in intensity may provide information about the health of the patient.

Different subpopulations of cells may accept the same label but emit light at different intensities. Therefore, the subpopulations may be differentiated. The antibody binding capacity for various surface antigens may be measured using methods generally known to ordinary practitioners of the art. For example, CD4$^+$ T cells bind about 50,000 antibody molecules. Protocols for assay development and image analysis can be defined based on the relative amount of antibody molecules that various cells can bind. Exposure times may be adjusted to further separate populations. For example, a total T-cell population may be identified with an anti-CD3 antibody. Even though CD3$^+$ cells are labeled with the same color as NK cells and B cells, the populations can be determined based on the differential labeling characterizing these cells. As the CD3$^+$ population becomes separated from the rest of the cell count (e.g., by increasing exposure time when taking the image), the percentage of CD8$^+$ cells may be determined by subtracting the number of CD4$^+$ cells and CD3$^+$ cells from the total CD3$^+$ cell count. In some embodiments, when cells are labeled with anti-CD8 antibody, there exists a strong intensity differential to discriminate CD8$^+$ cells from other cells such as NK cells and B cells. The strong intensity may accentuate the differential seen in a single color containing CD8$^+$ cytotoxic T cells, NK cells, and B cells. A ratio of CD8$^+$ cells may be calculated by dividing the total number of CD3$^+$ cells minus the total number of CD4$^+$ cells and CD3$^+$ cells by the total number of CD3$^+$ cells.

An analyte detection kit may include at least one cartridge designed for performing a predetermined analysis, a sample collection device and disinfectant wipes. In some embodiments, the cartridge, wipes, sample collection devices are individually obtained. In certain embodiments, the cartridge is checked for viability prior to use. In some embodiments, a portion of a human may be wiped with one of the disinfectant wipes and a blood sample may be obtained with the sample collection device. A portion of the collected sample may be deposited on or in a collection region of the cartridge. For example, a finger may be pricked with a lancet and a drop of blood transferred to the cartridge using disposable tubing, a pipette, or a fluid bulb. In some embodiments, the sample may be deposited directly onto a microsieve of a microsieve-based detection system. After the sample is introduced into a collection region of a cartridge, the collection region may be capped or sealed with, for example, an adhesive strip, a rubber plug, or a cover.

In some embodiments, one or more reagents may be provided to the sample. For example, anti-coagulant and/or fixative may be added to the blood sample. Fixatives include, but are not limited to, formaldehyde, paraformaldehyde, ethanol, sodium azide, colchicines, EDTA, EGTA, CYTO-CHEX® (Streck, Inc., Omaha, NE), and CYTO-CHEX ® BCT. In some embodiments, a reagent may be provided to the sample. The reagent may be mixed with the sample during or after collection of the sample. Alternatively, a reagent may be added to a sample after the sample is introduced into a cartridge. In certain embodiments, a reagent may be provided to the sample by, for example, one or more pumps, fluid packages, and/or reagent regions coupled to, positioned in, and/or positioned on a cartridge.

The cartridge may be positioned, automatically or manually, in a housing of the analyte detection system. The cartridge may substantially contain all fluids used for the analysis.

In some embodiments, a check of the cartridge may be performed. For example, the cartridge includes one or more particles having the desired analyte to be determined. An image of the particles may be obtained by one of the detectors. Analysis of the image is performed to determine if the known analyte can be detected. If the known analyte is detected, the cartridge is deemed suitable for use. If the known analyte is not detected, the cartridge may be disposed of and a new cartridge obtained. In some embodiments, the new cartridge is obtained from the kit or a supply of cartridges.

At least a portion of the sample may be provided to a metered volume portion of the cartridge. In some embodiments, the sample may be drawn by capillary action into the metered volume portion. In certain embodiments, the sample may be delivered by a fluid delivery system disposed in or coupled to the cartridge. After the sample has filled the metered volume portion, a portion of the sample may travel toward an overflow reservoir. In some embodiments, the sample may not be measured.

A fluid delivery system that includes a reagent may be actuated. Flow of fluid from the fluid delivery system may push a metered volume of sample from the metered volume portion towards a detection region that includes one or more detection systems (e.g., a particle-based detection system and/or a microsieve-based detection system). The reagent and sample may combine during passage of the sample toward the one or more detection regions to form a sample/reagent mixture. A portion of the sample/reagent mixture flows through or is collected in the detection region. The remaining portion of sample/reagent mixture may flow over or through the detection region to a waste region of the cartridge.

In some embodiments, the fluid delivery system is not necessary to push the sample towards the detection region. Capillary forces may transport the sample towards the detection region. In some embodiments, capillary forces that transport the sample are enhanced with hydrophilic materials (e.g., plastic or glass) or chemicals to coat a channel for aqueous samples. Certain portion of channels may include hydrophilic materials positioned proximate the collection region, in the metered volume chamber, and/or proximate the overflow reservoir to direct flow of aqueous samples through a cartridge.

In some embodiments, the sample may be drawn into a channel via negative pressure in the channel. For example, suction created by a passive valve or a negative pressure source may create negative pressure in a portion of a channel and draw fluids towards the detection region. In some embodiments, valves may be used to direct the flow of fluid and/or sample through the cartridge.

One or more additional fluid delivery systems may be actuated to release one or more additional fluids (e.g., additional PBS, water, or other buffers). One or more of the additional fluids may flow over or through one or more reagent regions (e.g., a reagent pad or a channel containing reagents). One or more reagents (e.g., one or more antibodies and/or a detectable label) in or on the reagent regions may be reconstituted by the additional fluids. The reconstituted reagents may be transported to the detection region of the cartridge. Transport of the reconstituted reagents may be accomplished by continued actuation of the fluid delivery systems or through other methods described herein. The reconstituted reagents may label and/or wash a portion of the sample collected in one or more detection regions of the cartridge (e.g., wash WBCs retained on a microsieve).

Portions of a sample and/or fluids may be provided to a detection region in a cartridge sequentially, successively, or substantially simultaneously. In some embodiments, a portion of the sample moves towards a detection region as a portion of the fluid from the second fluid delivery system flows towards a reagent region. Fluid from the second fluid delivery system may reconstitute and/or collect one or more reagents from the reagent region and deliver the reagents to the detection region after the sample has passed through the detection region. The collected reagents may then be added to an analytes that have been collected by the detection region.

Valves (e.g., pinch valves) and/or vents may be use to regulate flow of the sample. For example, a valve proximate the collection region may inhibit additional sample from flowing towards the detection region. In some embodiments, one or more changes in elevation of a channel may inhibit the sample form entering other channels.

In some embodiments, a reagent (e.g., a detectable label and/or one or more antibodies) may be directly added to the matter on a microsieve of a microsieve-based detection system. The sample may then be washed with fluid remaining in the first fluid delivery system or with the fluid from one or more of the other fluid delivery systems.

In some embodiments, only one fluid delivery system is used. For example, one or more syringes may be at least partially coupled to, positioned in, or positioned on the cartridge. Each syringe may contain one or more fluids to be used during the analysis. The syringes may be actuated and the fluids delivered sequentially, successively, or substantially simultaneously to the collection region, the reagent regions, and/or the detection region.

In some embodiments, analytes collected on a microsieve of a microsieve-based detection system may be viewed through a viewing chamber of the microsieve-based detection system. Light sources may be activated and light may be directed towards the microsieve-based detection system. Light may enter the microsieve-based detection system through a viewing chamber and/or a top layer of the microsieve-based detection system. A detector may collect a signal produced from interaction of light with one or more analytes in the detection region. In some embodiments, the detector may be optically aligned with the viewing chamber of the microsieve to allow the microsieve and/or detection region to be viewed by detector.

The detector processes the produced signal to produce images representative of the analytes collected by the detection system. Images may be obtained concurrently or simultaneously. Images may be analyzed and the analytes in the sample assessed.

The cartridge may then be removed from the analyzer and discarded. The above-described method may then be repeated for the next sample. In certain embodiments, portions of the analyzer may be disinfected between samples. In some embodiments, the cartridge is self-contained such that all fluids remain in the cartridge and the analyzer may not need to be disinfected.

Interaction of a sample with light produces a signal that is received by the detector. The detector may produce images from the signal. Images may be analyzed by an analyzer (e.g., automatically with a computer or manually by a human) to determine the analytes present in the sample.

A third fluid delivery system may be activated to allow a wash solution to flow through or over the detection region. The detection region may be washed repeatedly to clear the detection region and prepare for additional use.

The first fluid delivery system may be actuated, or an additional fluid delivery system may be used, to push a second portion of sample towards the microsieve. The analysis may be repeated to determine different and/or duplicate sample analysis.

The procedure may be repeated as necessary to obtain the needed data. Additional samples may also be obtained and used. In some embodiments, one or more microsieves may be used in a microsieve-based detection system. After all analyses have been completed, the cartridge may be ejected and properly discarded.

In some embodiments, an analyte detection system may be used to test for two or more analytes. The first and second analytes may include a wide range of cellular and/or chemical/biochemical components. Chemical/biochemical components may include, but are not limited to, electrolytes, proteins, nucleic acids (e.g., DNA and/or RNA), steroids and other drugs. In certain embodiments, an analyte detection system may be designed to test for indications of cancer (e.g., types of cancerous cells and/or levels of related biochemicals) as well as one or more diseases. For example, an analyte detection system may be designed to test for cervical cancer and sexually transmitted diseases.

In some embodiments, one or more cellular components of blood and/or one or more proteins may be assessed concurrently in an analyte detection system including particle- and/or microsieve-based detection systems coupled to one or more fluid flow systems. The proteins may include protein cardiac biomarkers. Protein cardiac biomarker targets may include, but are not limited to, proteins related to risk assessment, prognosis, and/or diagnosis. Protein cardiac biomarker targets related to necrosis, thrombosis, plaque rupture, endothelial dysfunction, inflammation, neurohormone activation, ischemia, arrhythmias, and/or other conditions may be assessed. Protein cardiac biomarker targets assessed by particle-based detection systems may include, but are not limited to, cardiac troponin T (cTNT), cardiac troponin I (cTNI), myoglobin (MYO), fatty acid binding protein (FABP), myeloperoxidase (MPO), plasminogen activator inhibitor-1 (PAI-1), tissue factor, soluble CD40 ligand (sCD40L), von Willebrand factor (vWF), D-dimer, matrix metalloproteins (MMPs), pregnancy associated plasma protein (PAPP), placental growth factor (PIGF), soluble intercellular adhesion molecules (sICAM), P-selectin, CRP, high sensitivity C-reactive protein (hs-CRP), oxidized low-density lipoprotein (ox-LDL), monocyte chemotactic protein-1 (MCP-1), interleukin-18 (IL-18), IL-6, TNF-$\alpha$, B-type natriuretic peptide (BNP), norepinephrine (NE), ischemia modified albumin (IMA), free fatty acids (uFFA), and combinations thereof.

The cellular components may include cellular cardiac biomarkers. Cellular cardiac biomarkers may include, but are not limited to, white blood cells, circulating endothelial cells (CFCs), endothelial progenitor cells (EPCs), platelets, and/or combinations or subsets thereof. In some embodiments, for example, a white blood cell subset may include lymphocytes.

Tests targeting CRP and WBCs are widely available in clinical settings; they are typically administered separately on different instruments. These tests may require large sample volumes, additional sample preparation steps, and longer assay times. In addition, the clinical instruments and methodologies currently used to complete these tests are not suitable for point-of-care testing, such as in a doctor's office, in an emergency room, or in an ambulance. The diagnostic and prognostic value of these biomarkers may be enhanced if these two tests could be administered concurrently on the same instrument in a convenient, accurate, and highly accessible manner.

In some embodiments, an analyte detection system is used to analyze two or more analytes in a fluid and/or sample. A first analyte may be cellular matter and a second analyte may be a one or more protein components. For example, the first analyte may be WBCs and the second analyte may be CRP. A sample (e.g., whole blood) may be obtained using the methods described herein or other sampling techniques known in the art. A portion of the sample may be provided to a collection region of a multi-functional cartridge.

At least a portion of the sample may be provided to a metered volume portion of the cartridge. In some embodiments, the sample may be drawn by capillary action into the metered volume portion. In certain embodiments, the sample may be delivered to a metered volume portion using a fluid delivery system. As the sample fills the metered volume portion, an excess portion of the sample may travel toward an overflow reservoir. The metered portion of the sample may be advanced toward one or more regions including, but not limited to, a particle-based detection system, a microsieve-based detection system, a cell-lysing chamber, a processing chamber, a polymerase chain reaction chamber, or combinations of these regions. In some embodiments, a metered volume portion of the cartridge may not be necessary.

Portions of the sample may be provided to detection systems in the cartridge sequentially, successively, or substantially simultaneously through pathways (e.g., channels) described previously. In some embodiments, a portion of the sample may be provided to a microsieve-based detection system, passed through the microsieve-based detection system, and the remaining sample is provided to a particle-based detection system. In some embodiments, a portion of the sample may be provided to a particle-based detection system before a portion of the sample is provided to a microsieve-based detection system. In certain embodiments, portions of the sample may be provided to a particle-based detection system and a microsieve-based detection system via separate pathways (e.g. channels) substantially simultaneously. In some embodiments, a sample from a single collection region may be provided to two or more pathways. In certain embodiments, samples may be provided to two or more collection regions and processed independently. After the collection region is filled, the collection region may be capped or sealed with a cover. At least a portion of the sample may be delivered to a microsieve-based detection system by methods including, but not limited to, activation of a fluid delivery system.

In some embodiments where the cartridge is designed for analysis of blood samples, one or more microsieves may be used to achieve separation of various whole blood components. For example, after the whole blood sample is provided to the microsieve, WBCs may remain on the surface of the microsieve, while other components of the blood sample (e.g., RBCs and/or plasma) move through the microsieve toward a waste reservoir or along one or more paths for further analysis. Cellular components (e.g., WBCs) on the surface of the microsieve may be washed or otherwise treated or assessed (e.g., counted). In some embodiments, one or more reagents (e.g., one or more WBC-specific antibodies labeled with an indicator molecule) may be provided to the microsieve by one or more fluid delivery systems. In certain embodiments, reagents provided to a sample may be filtered, reconstituted, or otherwise processed in a portion of the cartridge. The portion of the blood sample that passes through the microsieve may be directed toward an additional microsieve for filtering. For example, a second microsieve may remove RBCs from the blood sample. In some embodiments, RBCs may be further processed (e.g., lysed or recovered) and evaluated, such as by polymerase chain reaction (PCR), hematocrit count/calculation, and/or other tests.

In some embodiments, a portion of the blood sample that is substantially free of particulate (e.g., cellular) components may be directed toward a particle-based detection system for further analysis. For example, plasma may be directed toward a particle-based detection system that includes particles designed to detect specific proteins in the plasma. For example, particles designed to detect CRP may include CRP-capturing antibodies coupled to the particles. In some embodiments, one or more reagents may be delivered to the particle-based detection system by mechanisms including, but not limited to, fluid packages, reagent pads, or mini-pumps. In certain embodiments, a reagent delivered to a particle-based detection system may include one or more labeled antibodies. The amount and/or identity of the analytes may be assessed using an analyte detection system. In some embodiments, the cartridge may be positioned, manually or automatically, to allow an analyte detection system to analyze a microsieve-based detection system. The cartridge may then be repositioned, manually or automatically, in the analyte detection system to allow analytes in the particle-based detection system to be assessed.

## Example

An analyte detection system was used for the concurrent measurement of both CRP and WBCs. The analyte detection system included a multi-functional cartridge. The cartridge included a particle-based detection system and a microsieve-based detection system. The microsieve-based detection system was configured to capture and detect blood cells, while the particle-based detection system was configured to interact with blood proteins. The detection systems were each coupled to a fluid delivery system. The two detection systems shared a common computer. The computer controlled fluid delivery systems and optical components. The fluid delivery systems provided fluids for the analysis. The optical components assisted in microscopic evaluation of signals collected from the two detection systems.

The particle-based detection system of the cartridge was used to perform a CRP-specific immunoassay. The particle-based detection system included porous agarose microparticles positioned in a micro-etched array (3 x 3 array) of wells on a silicon wafer microchip. Three particles, coated with antibodies irrelevant to CRP, were used as negative controls. The other six particles were dedicated to CRP capture and detection. Rabbit CRP-specific antibodies were coupled to the particle to capture the CRP antigen. This level of particle redundancy increased the statistical significance and, hence, the precision and accuracy of the CRP measurements. ALEXA FLUOR® 488 labeled antibodies were employed to visualize the particle-captured protein.

A portion of the blood sample was introduced to the particle-based detection system, and the particles were washed with PBS. Low internal volumes of each particle (about 2 nL to about 30 nL per bead) used in conjunction with high effective flow rates (1-5 mL/min) allowed for the completion of highly stringent washes (>5000 effective washes per minute). The wash efficiently reduced nonspecific binding of antigens and detecting antibody reagents to the particles.

After washing, an image of the particle array was acquired in the following manner. Using standard epi-illumination geometry, white light from a 100-W mercury lamp was collimated, passed through a filter to select the excitation wavelengths centered at 480 nm with a 40 nm spectral bandwidth, reflected by a dichroic mirror (505 nm long pass mirror), and focused onto the particle array using a 4x microscope objective (NA of about 0.13). The fluorescence from the particles was collected by the microscope objective, transmitted through the dichroic mirror, passed through an emission filter centered at 535 nm with a 50 nm spectral width and detected by a CCD camera. The image was digitally processed and analyzed, and the signal intensity converted for each particle into a quantitative CRP measurement with the aid of a calibration curve. The time required to process the sample was approximately 12 minutes.

The particle-based detection region was washed one or more times with PBS and another image was acquired. Each assay of the sample was followed by a wash with PBS.

The particle-based CRP assay generally exhibited a detection range of at least 1 ng/ML up to 10,000 ng/mL. With the appropriate choice of assay conditions, use of particles coated with varying concentrations of binding agent (i.e., antibody), and/or use of sample dilution, the detection range for CRP was estimated to be expandable up to 100,000 ng/mL.

The above-described particle-based CRP assay was validated against a commercial high sensitivity-CRP enzyme limited immunosorbent assay (ELISA). CRP values from 9 human blood samples evaluated in parallel by ELISA and the particle-based method were in determined to be in agreement with each other.

A portion anti-coagulated blood sample was fixed with 4% paraformaldehyde, and then incubated for 5 minutes with an ALEXA FLUOR® 488 labeled anti-CD45 antibody specific for WBCs. Coagulation of blood may be inhibited by adding an anti-coagulating agent to the blood sample (e.g., heparin or ethylenediaminetetraacetic acid (EDTA)). The mixture was diluted with PBS and introduced to a membrane of the microsieve-based detection system with the use of an external peristaltic pump equipped with an injection valve. The membrane was a supported 13 mm track-etched polycarbonate membrane. Image acquisition was performed as described above for the particle-based detection system. Analysis of the scanning electron micrographs of the filtered whole blood revealed that RBCs, with roughly the same diameter as the WBCs, deformed and passed through the 3.0 micrometer pores of the membrane while WBCs were captured on the membrane. After removal of the RBCs, the WBCs were labeled with anti-CD45 antibody. Two populations of cells were observed. One population of cells was brighter than the second population of cells captured on the membrane.

To evaluate the linearity and analytical range of the membrane WBC assay, increasing volumes of a CD45-labeled whole blood suspension were delivered to the microsieve-based detection system. Following a rinse with PBS, images of the WBCs on the membrane were captured at 3 different fields of view (FOV) on the membrane. A pixel analysis algorithm, as described in U.S. Patent Application No. 10/522,499, was applied to identify and count individual WBC based on size, shape, and fluorescence intensity thresholding within the image J environment. From the images, it was determined that the WBC counts increased in a linear fashion with an increasing volume of blood delivered to the flow cell. The coefficient of variation (CV) of the counts measured in different FOVs (intra-assay precision) was found to be within the range of 5% to 15%, and was dependent on the volume of blood delivered on the membrane. Optimal precision with the above-described cell structure was achieved for volumes of blood between 0.81 μL and 14.3 μL.

To evaluate the inter-assay precision of the WBC assay, the equivalent of 2.1 μL of labeled whole blood was delivered to the microsieve-based detection system. For healthy donors with 5000 to 11,000 WBCs/μL, this volume of blood includes 10,500 to 23,100 WBCs. With the optical instrumentation described above, one FOV represented an area of 0.60 mm$^2$. Given that the total surface area of the membrane utilized for cell capture is 78.54 mm$^2$, the current membrane element was estimated to yield about 130 FOVs. Consequently, while the entire sample volume yields 10,500 to 23,100 FOVs, the single FOV collected a fluorescence signature of about 80 to about 176 cells, assuming that the cells were evenly distributed across the entire membrane.

Images from 5 non-overlapping FOVs were captured to get the preliminary mean WBC count. The preliminary count was converted to an absolute count after application of a scaling factor that incorporated the volume of blood delivered to the flow cell, as well as the number of FOVs covering the microsieve-based detection system onto which WBCs are captured. The experiment was repeated 5 times using different microsieve-based detection systems of the same configuration. The inter-assay coefficient of variation of the counts from one microsieve-based detection system to another microsieve-based detection system was determined to be 4.3%.

Additionally, the WBC counts achieved by the membrane counting method were in agreement (95%) with those determined by flow cytometry. Flow cytometry requires a larger blood sample size (100 μL) and an additional processing step to lyse the red blood cells. The excellent agreement between flow cytometry and microsieve-based detection indicates that the assumption of even cell distribution on the membrane of the microsieve-based detection system was accurate.

As shown by this example, an analyte detection system that includes a particle-based detection system and a microsieve-based detection system allows for enhanced CRP detection levels in whole blood and for separation, isolation, and detection of white blood cells from whole blood.

List of Reference Numbers in Drawings

| Name | Old Number | New Number | |
|------|-----------|-----------|---|
| Cartridge | 100 | 100 | 100 |
| Collection region | 50 | 102 | 102 |
| Cover | | 104 | 104 |
| Fluid channel | | 106 | 106 |
| Detection region | 60 | 108 | 108 |
| Top layer | 10 | 110 | 110 |
| Channel layer | 40 | 112 | 112 |
| Sample layer | 30 | 114 | 114 |
| Reservoir layer | 80 | 116 | 116 |
| Support (Bottom seal) layer | 90 | 118 | 118 |
| Opening | 20 | 120 | 120 |
| Reagent region | 70 | 122 | 122 |
| reservoir | | 124 | 124 |
| Channel to overflow | | 125 | 125 |
| Elevated channel 2 | | 126 | 126 |
| Channel 3 | | 128 | 128 |
| Channel 4 | | 130 | 130 |
| overflow region | 650 | 132 | 132 |
| Waste region | 670 | 134 | 134 |
| Connectors | 690 | 136 | 136 |
| Meter volume portion | 60 | 138 | 138 |
| Vent | | 140 | 140 |
| Viewing window | 332 | 142 | 142 |
| Cap of waste region | 710 | 144 | 144 |
| Fluid delivery system | | 150 | 150 |
| Fluid package | 350 | 152 | 152 |
| Fluid pack reservoir | 440 | 154 | 154 |
| Valves | | 156 | 156 |
| Pinch valve | 775 | 158 | 158 |
| Pinch valve layer 1 | 780 | 160 | 160 |
| Pinch valve layer 2 | 790 | 162 | 162 |
| Pinch valve layer 3 | 800 | 164 | 164 |
| Pinch valve channel | 810 | 166 | 166 |
| Actuators | 750 | 168 | 168 |
| Actuator | | 169 | 169 |
| Slider structure | 760 | 170 | 170 |

(continued)

| Name | Old Number | New Number | |
|---|---|---|---|
| Slider track | 770 | 172 | 172 |
| Adhesive layer | 360 | 174 | 174 |
| Fluid | 370 | 176 | 176 |
| Gas | 380 | 178 | 178 |
| Wall of fluid package | 390 | 180 | 180 |
| Channel | | 182 | |
| Projection | 410 | 182 | 182 |
| Top of reservoir | 420 | 184 | 184 |
| Laminate layer | 490 | 186 | 186 |
| Support | | 188 | 188 |
| Support layer | 460 | 189 | 189 |
| Channel layer | 470 | 190 | 190 |
| Channel cover (Coupling) | 475 | 192 | 192 |
| Top layer | | 194 | 194 |
| Coupling layer Opening | | 196 | 196 |
| Laminate layer opening | 495 | 198 | 198 |
| baffles | | 200 | 200 |
| seal | 480 | 202 | 202 |
| Opening in reservoir | | 203 | 203 |
| Fluid package rigid walls | | 204 | 204 |
| Flange | 500 | 205 | 205 |
| Fluid package gasket | | 206 | 206 |
| Burst point | | 208 | 208 |
| Fluid bulb | 510 | 210 | 210 |
| Body | | 211 | 211 |
| Fluid bulb stem | 520 | 212 | 212 |
| Fluid bulb tip | 530 | 214 | 214 |
| Connector | 570 | 216 | 216 |
| Syringe | 580 | 217<br>218<br>219 | 217<br>218<br>219 |
| Microsieve-based detection system | | 220 | 220 |
| Particle-based detection system | 610 | 222 | 222 |
| Membrane | 140 | 226 | 226 |
| Support | 150 | 228 | 228 |
| Housing | 110 | 230 | 230 |
| Bottom spacer | 120 | 232 | 232 |
| Bottom member | 130 | 234 | 234 |

(continued)

| Name | Old Number | New Number | |
|---|---|---|---|
| Indentations | | 236 | 236 |
| Indentation to receive fluid | | 238 | 238 |
| Outlet | 160 | 240 | 240 |
| Reagent pads | 640 | 240 | |
| Gasket | 170 | 242 | 242 |
| Top member | 190 | 244 | 244 |
| Inlet | 180 | 246 | 246 |
| Top spacer | 200 | 248 | 248 |
| Fastening member | 210 | 250 | 250 |
| top layer | 334 | 252 | 252 |
| middle layer | 338 | 254 | 254 |
| Bottom layer | 346 | 256 | 256 |
| Opening in top layer | 336 | 258 | 258 |
| Opening in middle layer | 340 | 260 | 260 |
| Opening in bottom layer | 344 | 262 | 262 |
| Support outer area | | 264 | 264 |
| Support open area | | 266 | 266 |
| Support open area pores | | 268 | 268 |
| Open area top portion | | 270 | 270 |
| Open area bottom portion | | 272 | 272 |
| Open area middle portion | | 274 | 274 |
| Analyte housing | | 280 | 280 |
| Housing | | 281 | 281 |
| Optical platform | | 282 | 282 |
| Detector | 850 | 284 | 284 |
| First light source | 820 | 286 | 286 |
| Second light source | 890 | 288 | 288 |
| First Lens | 830 | 290 | 290 |
| Second lens | 870 | 292 | 292 |
| third lens | 900 | 294 | 294 |
| Fourth lens | 910 | 296 | 296 |
| First filter | 850 | 298 | 298 |
| Second filter | 880 | 300 | 300 |
| Emission filter | 860 | 302 | 302 |
| Light | | 304 | 304 |
| Emitted light | | 306 | 306 |
| Second light | | 308 | 308 |
| Second emitted light | | 310 | 310 |

(continued)

| Name | Old Number | New Number | |
|------|-----------|-----------|-----|
| First dichroic mirror | 920 | 312 | 312 |
| Second dichroic mirror | | 314 | 314 |
| Second detector | | 316 | 316 |
| Detector lens | 960 | 318 | 318 |
| Second emission filter | 950 | 320 | 320 |
| Shutter | 970 | 322 | 322 |
| Filter holder | 990 | 324 | 324 |
| Actuator | 980 | 326 | 326 |
| First image | | 330 | 330 |
| Green cells (CD4) | | 332 | 332 |
| Green cells cd3- | | 334 | 334 |
| Green cells cd19- | | 336 | 336 |
| Red cells cd3+ | | 338 | 338 |
| Red cells cd19+ | | 340 | 340 |
| Red cells cd56+ | | 342 | 342 |
| Second image | | 344 | 344 |
| Third image | | 346 | 346 |
| Yellow cells | | 348 | 348 |
| Fig. 40 first image (fourth image) | | 350 | 350 |
| Fifth image | | 352 | 352 |
| red cells in second image of fig. 40-cd2 | | 354 | 354 |
| Sixth image | | 356 | 356 |
| Yellow cells c2, c4 | | 358 | 358 |
| Seventh image | | 360 | 360 |

## EMBODIMENTS

The following are preferred embodiments (emb.'s) of the present invention:

1. A system comprising a cartridge comprising:

a collection region;
a detection region configured to interact with at least a portion of a sample deposited in the collection region to allow detection of an analyte; and
a fluid delivery system configured to facilitate transport of fluid through at least a portion of the cartridge during use, the fluid delivery system comprises one or more gas packages configured to release gas to transport the fluid through the cartridge;

wherein the collection region, the detection region, and the fluid delivery system are coupled to, at least partially positioned in, or at least partially positioned on the cartridge.
2. The system of emb. 0, wherein the detection region is configured to allow imaging of at least a portion of the sample in the detection region.
3. The system of emb. 0, wherein the detection region is configured to allow detection of a signal produced by at least a portion of the sample in the detection region.

4. The system of emb. 0, wherein the detection region is configured to allow detection of a signal produced by an interaction of at least a portion of the sample with one or more components of the detection region.

5. The system of emb. 0, wherein the det. region comprises a particle-based detection system.

6. A system comprising a cartridge comprising:

a microsieve-based detection system coupled to, at least partially positioned in, or at least partially positioned on the cartridge, the microsieve-based detection system comprising a microsieve, wherein the microsieve is configured to at least partially retain one or more white blood cells in or on the microsieve when one or more blood samples are applied to the microsieve during use; and one or more detectable labels contained in or on the cartridge, wherein one or more of the detectable labels are configured to label one or more white blood cells retained in or on the microsieve during use, wherein at least one of the white blood cells comprises CD4$^+$ cells.

7. A method comprising applying a blood sample to a microsieve, wherein the microsieve is coupled to, at least partially positioned in, or at least partially positioned on a cartridge, and wherein one or more white blood cells in the blood sample are at least partially retained on or in the microsieve; and applying one or more detectable labels to label one or more of the retained white blood cells.

8. The method of emb. 7, wherein one or more of the detectable labels comprise an antibody coupled to a fluorophore.

9. The method of emb. 7, wherein one or more of the detectable labels comprise one or more dyes.

10. The method of emb. 7, wherein one or more of the labeled white blood cells have a fluorescence that is different than the fluorescence of an unlabeled white blood cell.

11. The method of emb. 7, wherein one or more of the labeled white blood cells have an absorbance that is different than the absorbance of an unlabeled white blood cell.

12. The method of emb. 7, further comprising obtaining an image of at least a portion of the microsieve.

13. The method of emb. 7, further comprising applying the sample to a collection region coupled to or at least partially positioned in or on the cartridge, wherein the collection region is coupled to the microsieve.

14. The method of emb. 13, further comprising transporting the sample from the collection region to the microsieve.

15. The method of emb. 7, wherein applying one or more detectable labels comprises activating at least one fluid delivery system to release at least one detectable label retained in the fluid delivery system, wherein the fluid delivery system is coupled to, at least partially positioned in, or at least partially positioned on the cartridge.

16. The method of emb. 7, further comprising assessing a total number of lymphocytes in the white blood cells by detecting the labeled lymphocytes.

17. A system comprising a cartridge comprising:

a collection region;

a detection region configured to interact with at least a portion of a sample deposited in the collection region to allow detection of an analyte; and

a fluid delivery system comprising one or more fluid packages, wherein the fluid delivery system is configured to facilitate transport of fluid through at least a portion of the cartridge during use;

wherein the collection region, the detection region, and the fluid delivery system are coupled to, at least partially positioned in, or at least partially positioned on the cartridge.

18. A method of detecting analytes in a fluid and/or a sample comprising applying a sample on or to a collection region coupled to, at least partially positioned in, or at least partially positioned on a cartridge; flowing fluid from a fluid delivery system to facilitate transport of at least a portion of the sample from the collection region towards a detection region, wherein the fluid delivery system and the detection region are coupled to, are at least partially positioned in, or are at least partially positioned on the cartridge; and obtaining one or more images of at least a portion of the detection region after the sample reaches the detection region.

19. The method of emb. 18, wherein the fluid delivery system comprises one or more fluid packages, and wherein flowing fluid from the fluid delivery system comprises applying pressure to a surface of at least one fluid package of the fluid delivery system.

20. The method of emb. 19, wherein pressure is applied using one or more actuators.

21. The method of emb. 18, further comprising transporting at least a portion of the sample through the detection region to a waste reservoir coupled to, at least partially positioned in, or at least partially positioned on the cartridge.

22. The method of emb. 18, wherein the det. region comprises a microsieve-based det. system.

23. The method of emb. 22, further comprising washing a surface of a microsieve of the microsieve-based detection system.

24. The method of emb. 18, wherein obtaining one or more of the images comprises capturing one or more of the images with one or more detectors optically coupled to the detection region.

25. The method of emb. 18, wherein the detection region comprises a microsieve-based detection system and a particle-based detection system, the method further comprising: transporting at least a portion of the sample to a microsieve of the microsieve-based detection system, wherein the microsieve at least partially retains a portion of the sample; and transporting at least a portion of the non-retained sample to the particle-based detection system.

26. The method of emb. 18, wherein the sample comprises blood.

27. A system comprising a cartridge comprising:

a collection region;
a detection region configured to interact with at least a portion of a sample deposited in the collection region to allow detection of an analyte;
one or more channels configured to allow fluid to flow through at least a portion of the cartridge; and

a fluid delivery system comprising one or more fluid packages, wherein at least one of the fluid packages is configured to create a partial vacuum, when opened, in one or more of the channels during use, and wherein the collection region, the detection region, and the fluid delivery system are coupled to, at least partially positioned in, or at least partially positioned on cartridge, wherein one or more of the channels couples the fluid delivery system to the collection region and to the detection region such that the partial vacuum created in at least one of the channels facilitates transport of at least a portion of the sample from the collection region to the detection region.

28. The system of emb. 27, wherein at least one of the fluid packages is configured to open when a seal between the fluid package and at least one of the channels is ruptured or removed during use.

29. The system of emb. 27, wherein at least one of the fluid packages is configured to open when heat is applied to the fluid package during use.

30. The system of emb. 27, wherein at least one of the fluid packages is configured to open when an electromagnetic signal is applied to the fluid package during use.

31. The system of emb. 27, further comprising one or more actuators configured to apply pressure to one or more of the fluid packages, wherein application of pressure to at least one of the fluid packages causes the fluid package to rupture, creating a partial vacuum in one or more channels during use.

32. The system of emb. 31, further comprising one or more slider structures, wherein one or more of the actuators are coupled to one or more of the slider structures, and wherein one or more of the slider structures are configured to position one or more of the actuators proximate to at least one of the fluid packages during use.

33. The system of emb. 27, wherein one or more of the channels couple the collection region to the detection region.

34. The system of emb. 27, wherein one or more of the channels couple one or more of the fluid packages to the collection region.

35. The system of emb. 27, wherein one or more of the channels couple one or more of the fluid packages to the detection region.

36. The system of emb. 27, further comprising one or more detectors optically coupled to the detection region, wherein one or more of the detectors are configured to view at least a portion of the detection region.

37. The system of emb. 27, wherein the detection region is configured to allow imaging of at least a portion of the sample in the detection region.

38. The system of emb. 27, wherein the detection region is configured to allow detection of a signal produced by at least a portion of the sample in the detection region.

39. The system of emb. 27, wherein the detection region is configured to allow detection of a signal produced by an interaction of at least a portion of the sample with one or more components of the detection region.

40. The system of emb. 27, wherein the detection region is configured to allow detection of absorption of one or more wavelengths of light by the sample in the detection region.

41. The system of emb. 27, wherein the detection region is configured to allow detection of fluorescence from the sample in the detection region.

42. The system of emb. 27, wherein the det. region comprises a particle-based detection system.

43. The system of emb. 27, wherein the coll. region comprises one or more absorbent pads.

44. The system of emb. 27, wherein the collection region comprises one or more reservoirs.

45. A method of detecting analytes in a sample comprising applying a sample on or to a collection region, wherein the collection region is coupled to, at least partially positioned in, or at least partially positioned on a cartridge; opening one or more fluid packages to create a partial vacuum that facilitates transport of at least a portion of the sample from the collection region to a detection region, wherein the detection region is coupled to, at least partially positioned in, or at least partially positioned on the cartridge; and obtaining one or more images of at least a portion of the detection region.

46. The method of emb. 45, wherein one or more channels couple the collection region to the detection region, and wherein opening one or more of the fluid packages creates a partial vacuum in one or more of the channels.

47. The method of emb. 45, wherein opening one or more fluid packages comprises applying pressure to one or more of the fluid packages.

48. The method of emb. 47, wherein pressure is applied using one or more actuators.

49. The method of emb. 45, further comprising transporting at least a portion of the sample through the detection region to a waste reservoir coupled to, at least partially positioned on, or at least partially positioned in the cartridge.

50. The method of emb. 45, wherein the detection region comprises a microsieve-based detection system.

51. The method of emb. 50, further comprising washing a surface of a microsieve of the microsieve-based detection system.

52. The method of emb. 45, wherein the det. region comprises a particle-based det. system.

53. The method of emb. 45, wherein obtaining one or more of the images comprises capturing one or more of images with one or more detectors optically coupled to the detection region.

54. The method of emb. 45, wherein the detection region comprises a microsieve-based detection system and a particle-based detection system, the method further comprising: transporting at least a portion of the sample to a microsieve of the microsieve-based detection system, wherein the microsieve at least partially retains a portion of the sample; and transporting at least a portion of the non-retained sample to the particle-based detection system.

55. The method of emb. 45, wherein the sample comprises blood.

56. A system comprising a cartridge comprising:

a collection region;
a detection region configured to interact with at least a portion of a sample deposited in the collection region to allow detection of an analyte; and

one or more viewing windows optically coupled to the detection region, wherein one or more of the viewing windows are configured to allow at least a portion of the detection region to be viewed by one or more detectors, wherein the cartridge further comprises a fluid delivery system, wherein the fluid delivery system is coupled to the collection region and the detection region such that fluid from the fluid delivery system facilitates transport of at least a portion of the sample from the collection region to the detection region during use.

57. The system of emb. 56, wherein the fluid delivery system comprises one or more fluid packages configured to open by application of pressure.

58. The system of emb. 57, further comprising one or more actuators configured to apply pressure to one or more of the fluid packages during use.

59. The system of emb. 58, further comprising one or more slider structures, wherein one or more of the actuators are coupled to one or more of the slider structures, and wherein one or more of the slider structures are configured to position one or more of the actuators proximate to one or more of the fluid packages during use.

60. The system of emb. 56, wherein the fluid deliv. system comprises one or more gas packages configured to release gas to transport the sample from the collection region to the det. region.

61. The system of emb. 56, wherein the cartridge further comprises one or more channels coupling the det. region to the coll. region, wherein one or more of the channels are configured to facilitate transport of at least a portion of the sample from the coll. region to the det. region. The system of emb. 56, wherein one or more of the viewing windows are configured to allow detection by one or more of the detectors of absorption of one or more wavelengths of light by a sample in the detection region or of fluorescence from a sample in the detection region. The system of emb. 56, wherein the detection region comprises a particle-based detection system.

62. The system of emb. 56, wherein the coll. region comprises one or more absorbent pads.

63. The system of emb. 56, wherein the collection region comprises one or more reservoirs.

64. A method comprising applying a sample on or to a collection region coupled to, at least partially positioned in, or at least partially positioned on a cartridge; flowing at least a portion of the sample from the collection region to a detection region coupled to, at least partially positioned in or at least partially positioned on the cartridge via one or more channels; obtaining one or more images of at least a portion of the detection region through one or more viewing windows optically coupled to the detection region.

65. The method of emb. 64, further comprising flowing fluid from a fluid delivery system coupled to, at least partially positioned in, or at least partially positioned on the cartridge to facilitate transport of the sample from the collection region to the detection region.

66. The method of emb. 65, wherein the fluid delivery system comprises one or more fluid packages, and wherein flowing fluid from the fluid delivery system comprises applying pressure to a surface of one or more fluid packages of the fluid delivery system.

67. The method of emb. 66, wherein pressure is applied using one or more actuators.

68. The method of emb. 64, further comprising transporting at least a portion of the sample through the detection region to a waste reservoir coupled to, at least partially positioned on, or at least partially positioned in the cartridge.

69. The method of emb. 64, wherein obtaining one or more of the images comprises capturing one or more images of at least a portion of the detection region with one or more detectors optically coupled to the detection region.

70. The method of emb. 64, wherein the det. region comprises a microsieve-based det. system.

71. The method of emb. 70, further comprising washing a surface of a microsieve of the microsieve-based detection system

72. The method of emb. 64, wherein the det. region comprises a particle-based det. system.

73. The method of emb. 64, wherein the det. region comprises a microsieve-based detection system and a particle-based detection system, the method further comprising transporting at least a portion of the sample to a microsieve of the microsieve-based detection system, wherein the microsieve at least partially retains a portion of the sample; and transporting at least a portion of the non-retained sample to the particle-based detection system.

74. The method of emb. 65, wherein the sample comprises blood.

75. A system comprising a cartridge comprising:

a collection region; a detection region configured to interact with at least a portion of a sample deposited in the collection region to allow detection of an analyte; a fluid delivery system comprising one or more channels, wherein the fluid delivery system is configured to facilitate transport of fluid through at least a portion of the cartridge during use; and one or more pinch valves coupled to one or more of the channels; wherein the collection region, the detection region, and the fluid delivery system are coupled to, at least partially positioned in, or at least partially positioned on the cartridge, the system further comprising one or more actuators configured to apply pressure to one or more of the pinch valves during use, wherein application of pressure to one or more of the pinch valves causes at least a portion of one or more of the channels to be at least partially blocked.

76. The system of emb. 75, wherein the fluid delivery system comprises one or more fluid packages, and wherein fluid from one or more of the fluid packages facilitates transport of at least a portion of a sample from the collection region to the detection region during use.

77. The system of emb. 76, wherein one or more of the fluid packages are configured to open when pressure is applied to the fluid package.

78. The system of emb. 76, further comprising one or more actuators configured to apply pressure to one or more of the fluid packages to open the fluid package during use.

79. The system of emb. 78, further comprising one or more slider structures, wherein one or more of the actuators are coupled to one or more of the slider structures, and wherein one or more of the slider structures are configured to position one or more of the actuators proximate to one or more of the fluid packages during use.

80. The system of emb. 75, wherein the fluid delivery system comprises one or more gas packages configured to release gas to transport fluid through the cartridge.

81. The system of emb. 75, wherein one or more of the channels couple the collection region to the detection region.

82. The system of emb. 75, wherein one or more of the channels couple the fluid delivery system to the collection region.

83. The system of emb. 75, wherein one or more of the channels couple the fluid delivery system to the detection region.

84. The system of emb. 75, further comprising one or more detectors optically coupled to the detection region, wherein one or more of the detectors is configured to view at least a portion of the detection region.

85. The system of emb. 75, wherein the detection region is configured to allow imaging of at least a portion of a sample in the detection region.

86. The system of emb. 75, wherein the detection region is configured to allow detection of a signal produced by at least a portion of a sample in the detection region.

87. The system of emb. 75, wherein the det. region is configured to allow det. of a signal produced by an interaction of at least a portion of the sample with one or more components of the detection region.

88. The system of emb. 75, wherein the detection region is configured to allow detection of absorption of one or more wavelengths of light by a sample in the detection region.

89. The system of emb. 75, wherein the detection region is configured to allow detection of fluorescence from a sample in the detection region.

90. The system of emb. 75, wherein the det. region comprises a particle-based det. system.

91. The system of emb. 75, wherein the detection region comprises a microsieve-based detection system and a particle-based detection system.

92. The system of emb. 75, wherein the coll. region comprises one or more absorbent pads.

93. The system of emb. 75, wherein the collection region comprises one or more reservoirs.

94. A method of detecting analytes in a fluid comprising applying a sample on or to a collection region coupled to, at least partially positioned in, or at least partially positioned on a cartridge; transporting fluid from a fluid delivery system through one or more channels to facilitate transport of at least a portion of the sample from the collection region to a detection region, wherein the fluid delivery system and the detection region are coupled to, at least partially positioned in, or at least partially positioned on the cartridge; applying pressure to one or more pinch valves coupled to one or more of the channels to control fluid flow through one or more of the channels; and obtaining one or more images of a detection region after the sample reaches the detection region, wherein the fluid delivery system comprises one or more fluid packages, wherein transporting the fluid through one or more channels comprises releasing fluid from one or more of the fluid packages.

95. The method of emb. 94, further comprising applying pressure to one or more of the fluid packages to release fluid from the fluid packages.

96. The method of emb. 95, wherein pressure is applied using one or more actuators.

97. The method of emb. 94, further comprising transporting at least a portion of the sample through the detection region to a waste reservoir coupled to, at least partially positioned in, or at least partially positioned on the cartridge.

98. The method of emb. 94, wherein the det. region comprises a microsieve-based det. system.

99. The method of emb. 98, further comprising washing a surface of a microsieve of the microsieve-based detection system

100. The method of emb. 94, wherein the det. region comprises a particle-based det. system.

101. The method of emb. 94, wherein obtaining one or more of the images comprises capturing one or more of the images with one or more detectors optically coupled to the detection region.

102. The method of emb. 94, wherein the det. region comprises a microsieve-based det. system and a particle-based det. system, the method further comprising: transporting at least a portion of the sample to a microsieve of the microsieve-based det. system, wherein the microsieve at least partially retains a portion of the sample; and transporting at least a portion of the non-retained sample to the particle-based det. system.

103. The method of emb. 94, wherein the sample comprises blood.

104. A system comprising a cartridge comprising:

a collection region;
a detection region configured to interact with at least a portion of a sample deposited in the collection region to allow detection of an analyte; and
a fluid delivery system comprising one or more syringes configured to facilitate transport of fluid through at least a portion of the cartridge during use;

wherein the collection region, the detection region, and the fluid delivery system are coupled to, at least partially positioned in, or at least partially positioned on the cartridge, wherein the fluid delivery system is coupled to the collection region and the detection region such that fluid from one or more of the syringes facilitates transport of at least a portion of the sample from the collection region to the detection region during use.

105. The system of emb. 104, further comprising one or more actuators configured to apply pressure to one or more of the syringes, wherein application of pressure to one or more of the syringes causes release of fluid from one or more of the syringes during use.

106. The system of emb. 104, further comprising one or more slider structures, wherein one or more of the actuators are coupled to one or more of the slider structures, and wherein one or more of the slider structures are configured to position one or more of the actuators proximate to one or more of the syringes during use.

107. The system of emb. 104, further comprising one or more channels, wherein one or more of the channels couple the collection region to the detection region.

108. The system of emb. 104, further comprising one or more channels, wherein one or more of the channels couple one or more of the syringes to the collection region.

109. The system of emb. 104, further comprising one or more channels, wherein one or more of the channels couple one or more of the syringes to the detection region.

110. The system of emb. 104, further comprising one or more detectors optically coupled to the detection region, wherein one or more of the detectors are configured to view at least a portion of the detection region.

111. The system of emb. 104, wherein the detection region is configured to allow imaging of at least a portion of the sample in the detection region.

112. The system of emb. 104, wherein the detection region is configured to allow detection of a signal produced by at least a portion of the sample in the detection region.

113. The system of emb. 104, wherein the detection region is configured to allow detection of a signal produced by an interaction of at least a portion of the sample with one or more components of the detection region.

114. The system of emb. 104, wherein the detection region is configured to allow detection of absorption of one or more wavelengths of light by the sample in the detection region. The system of emb. 104, wherein the detection region is configured to allow detection of fluorescence from the sample in the detection region.

115. The system of emb. 104, wherein the det. region comprises a particle-based det. system.

116. The system of emb. 104, wherein the coll. region comprises one or more absorbent pads.

117. The system of emb. 104, wherein the coll. region comprises one or more reservoirs.

118. A method comprising applying a sample on or to a collection region coupled to, at least partially positioned in, or at least partially positioned on a cartridge; flowing fluid from one or more syringes coupled to, at least partially positioned in, or at least partially positioned on the cartridge to facilitate transport of the sample from the collection region to a detection region positioned in or on the cartridge; and obtaining one or more images of the detection region after the sample reaches the detection region.

119. The method of emb. 118, further comprising applying pressure to one or more of the syringes to cause fluid to flow from one or more syringes.

120. The method of emb. 119, wherein pressure is applied using one or more actuators.

121. The method of emb. 118, further comprising transporting at least a portion of the sample through the detection region to a waste reservoir coupled to, at least partially positioned in, or at least partially positioned on the cartridge.

122. The method of emb. 118, wherein obtaining one or more of the images comprises capturing one or more of the images with one or more detectors optically coupled to the detection region.

123. The method of emb. 118, wherein the detection region comprises a microsieve-based detection system.

124. The method of emb. 123, further comprising washing a surface of a microsieve of the microsieve-based detection system.

125. The method of emb. 118, wherein the detection region comprises a particle-based detection system.

126. The method of emb. 118, wherein the detection region comprises a microsieve-based detection system and a particle-based detection system, the method further comprising: transporting at least a portion of the sample to a microsieve of the microsieve-based detection system, wherein the microsieve at least partially retains a portion of the sample; and transporting at least a portion of the non-retained sample to the particle-based det. system.

127. The method of emb. 118, wherein the sample comprises blood.

128. A system comprising a cartridge comprising:

a collection region;

a detection region configured to interact with at least a portion of a sample deposited in the collection region to allow detection of an analyte; and

one or more channels configured to allow fluid to flow through at least a portion of the cartridge, wherein a shape or an elevation of at least a portion of one or more of the channels is configured to selectively direct fluids flowing in or through one or more of the channels during use;

wherein the collection region and the detection region are coupled to or at least partially positioned in or on the cartridge, wherein it is preferred that a change in elevation of one or more of the channels partially inhibits fluids from flowing into a portion of the channel.

129. The system of emb. 128, wherein a change in a diameter of one or more of the channels partially inhibits fluids from flowing into a portion of the channel.

130. The system of emb. 128, wherein a shape or an elevation of at least a portion of one or more of the channels is configured to partially inhibit fluids flowing through one or more of the channels during use.

131. The system of emb. 128, wherein a shape or an elevation of at least a portion of one or more of the channels is configured to facilitate flowing fluids through one or more of the channels during use.

132. The system of emb. 128, wherein the cartridge comprises two or more layers coupled together, and wherein one or more of the channels are positioned in more than one layer of the cartridge.

133. The system of emb. 128, wherein one or more of the channels couple the collection region to the detection region.

134. The system of emb. 128, wherein one or more of the channels are part of a fluid delivery system, wherein the fluid delivery system is configured to facilitate transport of fluid through at least a portion of the cartridge during use.

135. The system of emb. 134, wherein the fluid delivery system is coupled to the collection region and the detection region such that the fluid delivery system facilitates transport of at least a portion of a sample from the collection region to the detection region during use.

136. The system of emb. 134, wherein the fluid delivery system comprises one or more fluid packages.

137. The system of emb. 136, wherein one or more fluid packages are configured to open when pressure is applied to the fluid package.

138. The system of emb. 137, further comprising one or more actuators configured to apply pressure to one or more of the fluid packages during use.

139. The system of emb. 138, further comprising one or more slider structures, wherein one or more of the actuators are coupled to one or more of the slider structures, and wherein one or more of the slider structures are configured to position one or more of the actuators proximate to one or more of the fluid packages during use.

140. The system of emb. 134, wherein the fluid delivery system comprises one or more gas packages configured to release gas to transport fluid through the cartridge during use.

141. The system of emb. 128, further comprising one or more detectors optically coupled to the detection region, wherein one or more of the detectors are configured to view at least a portion of the detection region.

142. The system of emb. 128, wherein the detection region is configured to allow imaging of at least a portion of the sample after the sample reaches the detection region.

143. The system of emb. 128, wherein the detection region is configured to allow detection of a signal produced by at least a portion of the sample in the detection region.

144. The system of emb. 128, wherein the detection region is configured to allow detection of a signal produced by an interaction of at least a portion of the sample with a component of the detection region.

145. The system of emb. 128, wherein the detection region is configured to allow detection of absorption of one or more wavelengths of light by the sample in the detection region.

The system of emb. 128, wherein the detection region is configured to allow detection of fluorescence from the sample in the detection region.

The system of emb. 128, wherein the det. region comprises a particle- or microsieve-based det. system, or wherein the det. region comprises a microsieve coupled to or at least partially positioned in or on the cartridge and one or more particles in one or more cavities in or on the cartridge.

The system of emb. 128, wherein the coll. region comprises one or more absorbent pads.

146. The system of emb. 128, wherein the coll. region comprises one or more reservoirs.

147. A method comprising applying a sample on or to a coll. region coupled to, at least partially positioned in, or at least partially positioned on a cartridge; and directing at least a portion of the sample through one or more channels from the collection region to a det. region, wherein the det. region is coupled to, at least partially positioned in, or at least partially positioned on the cartridge, and wherein a shape or an elevation of at least a portion of one or more of the channels selectively directs the sample through one or more of the channels.

148. The method of emb. 147, wherein one or more of the channels are part of a fluid delivery system, and wherein the fluid delivery system comprises one or more fluid packages, the method further comprising releasing fluid from one or more fluid packages into one or more of the channels.

149. The method of emb. 148, further comprising applying pressure to one or more of the fluid packages to release fluid from one or more of the fluid packages.

150. The method of emb. 149, wherein pressure is applied using one or more actuators.

151. The method of emb. 147, further comprising capturing one or more images of the detection region with one or more detectors optically coupled to the detection region.

152. The method of emb. 147, further comprising transporting at least a portion of the sample through the detection region to a waste reservoir coupled to, at least partially positioned in, or at least partially positioned on the cartridge.

153. The method of emb. 147, wherein the detection region comprises a microsieve-based detection system.

154. The method of emb. 153, further comprising washing a surface of a microsieve of the microsieve-based detection system.

155. The method of emb. 147, wherein the detection region comprises a particle-based detection system.

156. The method of emb. 147, wherein the detection region comprises a microsieve-based detection system and a particle-based detection system, the method further comprising transporting at least a portion of the sample to a microsieve of the microsieve-based detection system, wherein the microsieve at least partially retains a portion of the sample; and transporting at least a portion of the non-retained sample to the particle-based det. system.

157. The method of emb. 147, wherein the sample comprises blood.

158. A system comprising a cartridge comprising:

a collection region; a detection region configured to interact with at least a portion of a sample deposited in the collection region to allow detection of an analyte; and one or more channels configured to allow fluid to flow through at least a portion of the cartridge, wherein a surface of, or a coating on, at least a portion of one or more of the channels is configured to selectively direct fluids flowing through one or more of the channels during use; and

wherein the collection region and the detection region are coupled to, at least partially positioned in, or at least partially positioned on the cartridge, wherein a surface of at least a portion of one or more of the channels is substantially hydrophobic.

159. The system of emb. 158, wherein a surface of at least a portion of one or more of the channels is substantially hydrophilic.

160. The system of emb. 158, wherein a coating on at least a portion of one or more of the channels is substantially hydrophobic.

161. The system of emb. 158, wherein a coating on at least a portion of one or more of the channels is substantially hydrophilic.

162. The system of emb. 158, wherein one or more of the channels couple the collection region to the detection region.

163. The system of emb. 158, wherein one or more of the channels are part of a fluid delivery system, wherein the fluid delivery system is configured to facilitate transport of fluid through at least a portion of the cartridge during use.

164. The system of emb. 163, wherein the fluid delivery system is coupled to the collection region and the detection region such that fluid delivery system facilitates transport of at least a portion of a sample from the collection region to the detection region during use.

165. The system of emb. 163, wherein the fluid delivery system comprises one or more fluid packages.

166. The system of emb. 165, wherein one or more of the fluid packages are configured to open when pressure is applied to the fluid package.

167. The system of emb. 166, further comprising one or more actuators configured to apply pressure to one or more of the fluid packages during use.

168. The system of emb. 167, further comprising one or more slider structures, wherein one or more of the actuators are coupled to one or more of the slider structures, and wherein one or more of the slider structures are configured to position one or more of the actuators proximate to one or more of the fluid packages.

169. The system of emb. 163, wherein the fluid delivery system comprises one or more gas packages configured to release gas to transport fluid through the cartridge during use.

170. The system of emb. 158, further comprising one or more detectors optically coupled to the detection region, wherein one or more of the detectors are configured to view at least a portion of the detection region.

171. The system of emb. 158, wherein the detection region is configured to allow imaging of at least a portion of a sample in the detection region.

172. The system of emb. 158, wherein the detection region is configured to allow detection of a signal produced by at least a portion of a sample in the detection region.

173. The system of emb. 158, wherein the det. region is configured to allow detection of a signal produced by an interaction of at least a portion of a sample with a component of the det. region.

174. The system of emb. 158, wherein the detection region is configured to allow detection of absorption of one or more wavelengths of light by a sample in the detection region.

The system of emb. 158, wherein the detection region is configured to allow detection of fluorescence from a sample in the detection region.

The system of emb. 158, wherein the detection region comprises a particle-based det. system.

175. The system of emb. 158, wherein the coll. region comprises one or more absorbent pads.

176. The system of emb. 158, wherein the coll. region comprises one or more reservoirs.

177. A method comprising applying a sample on or to a collection region coupled to, at least partially positioned in, or at least partially positioned on a cartridge; directing at least a portion of the sample through one or more channels from the collection region to a detection region, wherein the detection region is coupled to, at least partially positioned in, or at least partially positioned on the cartridge, wherein a surface of, or a coating on, at least a portion of one or more of the channels selectively directs the sample through one or more of the channels.

178. The method of emb. 177, wherein one or more of the channels are part of a fluid deliv. system, and wherein the fluid deliv. system comprises one or more fluid packages, the method further comprising releasing fluid from one or more fluid packages into one or more of the channels.

179. The method of emb. 178, further comprising applying pressure to one or more of the fluid packages to release fluid into one or more of the channels.

180. The method of emb. 179, wherein pressure is applied using an actuator.

181. The method of emb. 177, further comprising transporting at least a portion of the fluid through the detection region to a waste reservoir coupled to, at least partially positioned in, or at least partially positioned on the cartridge.

182. The method of emb. 177, further comprising capturing one or more of the images of the sample with one or more detectors optically coupled to the detection region.

183. The method of emb. 177, wherein the detection region comprises a microsieve-based detection system.

184. The method of emb. 183, further comprising washing a surface of a microsieve of the microsieve-based detection system

185. The method of emb. 177, wherein the detection region comprises a particle-based detection system.

186. The method of emb. 177, wherein the detection region comprises a microsieve-based detection system and a particle-based detection system, the method further comprising transporting at least a portion of the sample to a microsieve of the microsieve-based detection system, wherein the microsieve at least partially retains a portion of the sample; and transporting at least a portion of the non-retained sample to the particle-based detection system.

187. The method of emb. 177, wherein the sample comprises blood.

188. A system comprising a cartridge comprising:

a collection region; a detection region configured to interact with at least a portion of a sample deposited in the collection region to allow detection of an analyte; one or more channels configured to allow fluid to flow through at least a portion of the cartridge; and a body comprising a plurality of layers coupled together,

wherein the collection region and the detection region are coupled to, at least partially positioned in, or at least partially positioned on the body, wherein one or more of the channels are positioned in more than one layer of the body such that one or more of the channels change elevation in the body.

189. The system of emb. 188, wherein the collection region is positioned in a different layer than the detection region.

190. The system of emb. 188, wherein the body comprises:

one or more channel layers comprising at least a portion of one or more of the channels; a sample layer comprising the collection region and the detection region; and
a reservoir layer coupled to the sample layer, wherein the reservoir layer collects one or more fluids passing through the sample layer; and

wherein one or more of the channels couple the collection region to the detection region.

191. The system of emb. 188, further comprising a fluid delivery system coupled to one or more of the channels, wherein the fluid delivery system is configured to facilitate transfer of fluids through one or more of the channels during use.

192. The system of emb. 190, wherein the fluid delivery systems is coupled to the coll. region and the det. region such that fluid from the fluid delivery system facilitates transport of at least a portion of a sample from the coll. region to the det. region during use.

193. The system of emb. 190, wherein the fluid delivery system comprises one or more fluid packages, and wherein fluid from one or more of the fluid packages facilitates transport of fluids through one or more of the channels during use.

194. The system of emb. 192, wherein one or more of the fluid packages are configured to open when pressure is applied to the fluid package.

195. The system of emb. 193, further comprising one or more actuators configured to apply pressure to one or more of the fluid packages.

196. The system of emb. 194, further comprising one or more slider structures, wherein one or more of the actuators are coupled to one or more of the slider structures, and wherein one or more of the slider structures are configured to position one or more of the actuators proximate to one or more of the fluid packages.

197. The system of emb. 190, wherein the fluid delivery system comprises one or more gas packages configured to release gas to facilitate transport of fluids through one or more of the channels during use.

198. The system of emb. 188, wherein one or more of the channels couple the collection region to the detection region.

199. The system of emb. 188, further comprising one or more detectors optically coupled to the detection region, wherein one or more of the detectors are configured to view at least a portion of the detection region.

200. The system of emb. 188, wherein the detection region is configured to allow imaging of at least a portion of a sample in the detection region.

201. The system of emb. 188, wherein the detection region is configured to allow detection of a signal produced by at least a portion of the sample in the detection region.

202. The system of emb. 188, wherein the detection region is configured to allow detection of a signal produced by an interaction of at least a portion of a sample with a component of the detection region.

203. The system of emb. 188, wherein the detection region is configured to allow detection of absorption of one or more wavelengths of light by a sample in the detection region.

The system of emb. 188, wherein the detection region is configured to allow detection of fluorescence from a sample in the detection region.

The system of emb. 188, wherein the det. region comprises a particle-based detection system.

204. The system of emb. 188, wherein the coll. region comprises one or more absorbent pads.

205. The system of emb. 188, wherein the coll. region comprises one or more reservoirs.

206. A system comprising a cartridge comprising:

a coll. region; a cover removably positionable over the coll. region; and a det. region configured to interact with at least a portion of a sample deposited in the coll. region to
allow detection of an analyte;

wherein the coll. region and the det. region are coupled to, at least partially positioned in, or at least partially positioned on the cartridge, the system optionally further comprising a fluid delivery system coupled to the coll. region and the det. region, wherein the fluid delivery systems is configured to facilitate transport of fluid through at least a portion of the cartridge.

207. The system of emb. 206, wherein the fluid delivery system comprises one or more fluid packages.

208. The system of emb. 207, further comprising one or more actuators configured to apply pressure to one or more of the fluid packages, wherein one or more of the fluid packages are configured to open when pressure is applied to the fluid package.

209. The system of emb. 208, further comprising one or more slider structures, wherein one or more of the actuators are coupled to one or more of the slider structures, and wherein one or more of the slider structures are configured to position one or more of the actuators proximate to one or more of the fluid packages.

210. The system of emb. 206, wherein the fluid deliv. system comprises one or more gas packages configured to release gas to facilitate transport of fluid through at least a portion of the cartridge.

211. The system of emb. 206, further comprising one or more channels that couple the collection region to the detection region.

212. The system of emb. 206, further comprising one or more detectors optically coupled to the detection region, wherein one or more of the detectors are configured to view at least a portion of the detection region.

213. The system of emb. 206, wherein the detection region is configured to allow imaging of at least a portion of the sample in the detection region.

214. The system of emb. 206, wherein the detection region is configured to allow detection of a signal produced by at least a portion of the sample in the detection region.

215. The system of emb. 206, wherein the det. region is configured to allow det. of a signal produced by an interaction of at least a portion of the sample with a component of the det. region.

216. The system of emb. 206, wherein the detection region is configured to allow detection of absorption of one or more wavelengths of light by the sample in the detection region.

The system of emb. 206, wherein the detection region is configured to allow detection of fluorescence from the sample in the detection region.

The system of emb. 206, wherein the det. region comprises a microsieve- or particle-based det. system, or comprises a microsieve coupled to or at least partially positioned in or on the cartridge and one or more particles in one or more cavities in or on the cartridge.

217. The system of emb. 206, wherein the coll. region comprises one or more absorbent pads.

218. The system of emb. 206, wherein the coll. region comprises one or more reservoirs.

219. The system of emb. 206, wherein the sample comprises blood.

220. A method comprising applying a sample on or to a collection region coupled to, at least partially positioned in, or at least partially positioned on a cartridge; positioning a cover over the collection region; transporting at least a portion of the sample from the collection region towards a detection region, wherein the detection region is coupled to, at least partially positioned in, or at least partially positioned on the cartridge; and obtaining an image of at least a portion of the sample in or on the detection region of the cartridge.

221. The method of emb. 220, wherein positioning the cover comprises sliding the cover over the collection region.

222. The method of emb. 220, further comprising sealing the collection region with the cover.

223. The method of emb. 220, wherein the cover is removable from the cartridge, or is positioned over the collection region after application of the sample to or on the coll. region or over the coll. region after at least a portion of the sample is transported to the detection region.

224. The method of emb. 220, further comprising flowing fluid to transport at least a portion of the sample from the collection region towards the detection region or releasing fluids from a fluid delivery system coupled to the collection region to transport at least a portion of the sample from the collection region towards the detection region.

225. The method of emb. 224, wherein the fluid delivery system comprises one or more fluid packages, and wherein releasing fluids from the fluid delivery system comprises applying pressure to one or more of the fluid packages to cause release of fluid from the fluid package.

226. The method of emb. 225, wherein pressure is applied using one or more actuators.

227. The method of emb. 220, further comprising transporting at least a portion of the sample through the detection region to a waste reservoir coupled to, at least partially positioned on, or at least partially positioned in the cartridge.

228. The method of emb. 220, wherein obtaining one or more of the images comprises capturing one or more of the images with one or more detectors optically coupled to the detection region.

229. The method of emb. 220, wherein the det. region comprises a microsieve-based det. system.

230. The method of emb. 229, further comprising washing a surface of a microsieve of the microsieve-based detection system.

231. The method of emb. 220, wherein the detection region comprises a particle-based detection system.

232. The method of emb. 220, wherein the detection region comprises a microsieve-based detection system and a particle-based detection system, the method further comprising: transporting at least a portion of the sample to a microsieve of the microsieve-based detection system, wherein the microsieve at least partially retains a portion of the sample; and transporting at least a portion of the non-retained sample to the particle-based det. system.

233. The method of emb. 220, wherein the sample comprises blood.

234. A system comprising a cartridge comprising:

a collection region;

a detection region configured to interact with at least a portion of a sample deposited in the collection region to allow detection of an analyte; one or more reagent regions coupled to the det. region, wherein one or more of the reagent regions are configured such that one or more reagents from one or more of the reagent regions are added to a fluid flowing through one or more reagent regions during use;

wherein the collection region, the detection region and one or more of the reagent regions are coupled to, at least partially positioned in, or at least partially positioned on the cartridge, wherein one or more of the reagent regions comprises one or more reagent pads.

235. The system of emb. 234, wherein one or more of the reagent regions are coupled to the collection region.

236. The system of emb. 234, wherein one or more of the reagent regions are coupled to the collection region such that at least a portion of one or more of the reagents from the reagent region are added to a portion of the sample during use or such that a portion of the sample from the collection region releases one or more reagents from one or more of the reagent regions as the sample passes through one or more of the reagent regions during use.

237. The system of emb. 234, further comprising a fluid deliv. system coupled to, at least partially positioned in, or at least partially positioned on the cartridge, wherein the fluid deliv. system is configured facilitate transport of fluid from the collection region to the detection region.

238. The system of emb. 234, wherein the fluid delivery system comprises one or more fluid packages, and wherein fluid from one or more of the fluid packages facilitates transport of at least a portion of the sample from the coll. region to the detection region during use.

239. The system of emb. 238, wherein one or more of the fluid packages are configured to open when pressure is applied to the fluid package.

240. The system of emb. 239, further comprising one or more actuators configured to apply pressure to one or more of the fluid packages to open the fluid package during use.

241. The system of emb. 240, further comprising one or more slider structures, wherein one or more of the actuators are coupled to one or more of the slider structures, and wherein one or more of the slider structures are configured to position one or more of the actuators proximate to one or more of the fluid packages during use.

242. The system of emb. 238, wherein the fluid delivery system comprises one or more gas packages configured to release gas to facilitate transport of fluid through at least a portion of the cartridge.

243. The system of emb. 234, further comprising one or more detectors optically coupled to the detection region, wherein one or more of the detectors are configured to view at least a portion of the detection region.

244. The system of emb. 234, wherein the detection region is configured to allow imaging of at least a portion of the sample in the detection region.

245. The system of emb. 234, wherein the detection region is configured to allow detection of a signal produced by at least a portion of the sample in the detection region.

246. The system of emb. 234, wherein the detection region is configured to allow detection of a signal produced by an interaction of at least a portion of the sample with a component of the detection region.

247. The system of emb. 234, wherein the detection region is configured to allow detection of absorption of one or more wavelengths of light by the sample in the detection region.

The system of emb. 234, wherein the detection region is configured to allow detection of fluorescence from the sample in the detection region.

The system of emb. 234, wherein the det. region comprises a particle-based detection system.

248. The system of emb. 234, wherein the coll. region comprises one or more absorbent pads.

249. The system of emb. 234, wherein the coll. region comprises one or more reservoirs.

250. A system comprising a cartridge comprising:

a collection region; a detection region configured to interact with at least a portion of a sample deposited in the collection region to allow detection of an analyte; and one or more channels configured to allow fluid to flow through at least a portion of the cartridge, wherein one or more of the channels comprise one or more reagents positioned in the one or more channels such that one or more reagents are added to a fluid passing through one or more of the channel during use;

wherein the collection region and the detection region are coupled to, at least partially positioned in, or at least partially positioned on the cartridge.

251. A method comprising applying a sample on or to a collection region coupled to, at least partially positioned in,

or at least partially positioned on a cartridge; transporting at least a portion of the sample from the collection region towards a detection region, wherein the detection region is coupled to, at least partially positioned in, or at least partially positioned on the cartridge; flowing fluids or sample through one or more reagent regions coupled to, at least partially positioned in, or at least partially positioned on the cartridge such that one or more reagents are added from the reagent region to the fluid or sample; and obtaining an image of at least a portion of the sample in or on the detection region of the cartridge.

252. The method of emb. 251, further comprising releasing fluids from a fluid delivery system coupled to the collection region to transport at least a portion of the sample from the collection region towards the detection region.

253. The method of emb. 252, wherein the fluid delivery system comprises one or more fluid packages, and wherein releasing fluids from the fluid deliv. system comprises applying pressure to one or more of the fluid packages to cause release of fluid from the fluid package.

254. The method of emb. 253, wherein pressure is applied using one or more actuators.

255. The method of emb. 251, further comprising transporting at least a portion of the sample through the detection region to a waste reservoir coupled to, at least partially positioned in, or at least partially positioned on the cartridge.

256. The method of emb. 251, further comprising capturing one or more images of a portion of the detection region with one or more detectors optically coupled to the detection region.

257. The method of emb. 251, wherein the detection region comprises a microsieve-based detection system.

258. The method of emb. 256, further comprising washing a surface of a microsieve of the microsieve-based detection system.

259. The method of emb. 251, wherein the detection region comprises a particle-based detection system.

260. The method of emb. 251, wherein the detection region comprises a microsieve-based detection system and a particle-based detection system, the method further comprising transporting at least a portion of the sample to a microsieve of the microsieve-based detection system, wherein the microsieve at least partially retains a portion of the sample; and transporting at least a portion of the non-retained sample to the particle-based detection system.

261. The method of emb. 251, wherein the sample comprises blood.

262. A method comprising applying at least a portion of a sample to a detection region coupled to, at least partially positioned in, or at least partially positioned on a cartridge, wherein the det. region interacts with at least a portion of the sample to allow detection of an analyte; and flowing fluids or sample through one or more channels in or on the cartridge such that one or more reagents disposed in one or more of the channels are added to the sample.

263. A system comprising a cartridge comprising:

a collection region; a detection region configured to interact with at least a portion of a sample deposited in the collection region to allow detection of an analyte;

one or more channels configured to allow fluid to flow through at least a portion of the cartridge; and one or more vents coupled to one or more of the channels, wherein one or more of the vents are configured to allow gas to enter or exit one or more of the channels during use;

wherein the collection region and the detection region are coupled to or at least partially positioned in or on the cartridge, the system optionally further comprising a fluid deliv. system coupled to or at least partially positioned in or on the cartridge, wherein the fluid deliv.

systems is configured to facilitate transport of fluid through one or more of the channels. The system of emb. 263, wherein the fluid delivery system comprises one or more fluid packages, and wherein fluid from one or more of the fluid packages is released into one or more of the channels during use.

264. The system of emb. 263, wherein one or more of the fluid packages are configured to open when pressure is applied to the fluid package.

265. The system of emb. 264, further comprising one or more actuators configured to apply pressure to one or more of the fluid packages to open the fluid package during use.

266. The system of emb. 265, further comprising one or more slider structures, wherein one or more of the actuators are coupled to one or more of the slider structures, and wherein one or more of the slider structures are configured to position one or more of the actuators proximate to one or more of the fluid packages.

267. The system of optional emb. 263, wherein the fluid delivery system comprises one or more gas packages configured to release gas to facilitate transport of fluid through at least a portion of one or more of the channels.

268. The system of emb. 263, further comprising one or more detectors optically coupled to the detection region, wherein one or more of the detectors are configured to view at least a portion of the detection region.

269. The system of emb. 263, wherein the detection region is configured to allow imaging of at least a portion of a sample in the detection region or detection of a signal produced by at least a portion of a sample in the detection region. The system of emb. 263, wherein the detection region is configured to allow detection of a signal produced by an interaction of at least a portion of a sample with a component of the detection region, of absorption of one or more wavelengths of light by a sample in the detection region, or of fluorescence from a sample in the det. region or comprises a particle-based detection system.

270. The system of emb. 263, wherein the collection region comprises one or more absorbent pads or one or more reservoirs.

271. The system of emb. 263, wherein the det. region comprises a microsieve-based detection system.

272. The system of emb. 263, wherein the detection region comprises:a microsieve coupled to or at least partially positioned in or on the cartridge and one or more particles in one or more cavities in or on the cartridge.

273. A method comprising applying a sample on or to a coll. region coupled to, at least partially positioned in, or at least partially positioned on a cartridge; transporting the sample through one or more channels in or on the cartridge, wherein the one or more channels are coupled to the coll. region; and releasing gas from one or more of the channels through vents coupled to one or more of the channels as the sample is transported through one or more of the channels.

274. The method of emb. 273, further comprising releasing fluids from a fluid deliv. system coupled to one or more channels to transport at least a portion of the sample through one or more channels.

275. The method of emb. 274, wherein the fluid delivery system comprises one or more fluid packages, and wherein releasing fluids from the fluid delivery system comprises applying pressure to one or more of the fluid packages to cause release of fluid from the fluid package.

276. The method of emb. 275, wherein pressure is applied using one or more actuators.

277. The method of emb. 273, further comprising transporting the sample to a detection region coupled to, at least partially positioned in or on the cartridge.

278. The method of emb. 277, further comprising transporting at least a portion of the fluid through the detection region to a waste reservoir coupled to, at least partially positioned in, or at least partially positioned on the cartridge or capturing one or more of the images of a portion of the detection region with one or more detectors optically coupled to the cartridge.

279. The method of emb. 277, wherein the detection region comprises a microsieve-based detection system.

280. The method of emb. 279, further comprising washing a surface of a microsieve of the microsieve-based detection system.

281. The method of emb. 277, wherein the detection region comprises a particle-based detection system.

282. The method of emb. 277, wherein the detection region comprises a microsieve-based detection system and a particle-based detection system, the method further comprising transporting at least a portion of the sample to a microsieve of the microsieve-based detection system, wherein the microsieve at least partially retains a portion of the sample; and transporting at least a portion of the non-retained sample to the particle-based detection system.

283. The method of emb. 273, wherein the sample comprises blood.

284. A system comprising a cartridge comprising:

a collection region; a detection region configured to interact with at least a portion of a sample deposited in the collection region to allow detection of an analyte; and an
overflow reservoir coupled to the collection region to collect excess sample or fluid;

wherein the collection region, the detection region and the overflow reservoir are coupled to or at least partially positioned in or on the cartridge, the system optionally further comprising a sample collection device coupled to the collection region.

285. The system of emb. 284, wherein the overflow reservoir is configured to collect excess sample from the collection region or is coupled to the collection region such that a predetermined volume of the sample is contained in the collection region and excess sample flows to the overflow reservoir.

286. The system of emb. 284, further comprising a fluid delivery system coupled to, at least partially positioned in, or at least partially positioned on the cartridge, wherein the fluid delivery system is configured facilitate transport of fluid from the collection region to the det. region.

287. The system of emb. 286, wherein the fluid delivery system comprises one or more fluid packages.

288. The system of emb. 287, further comprising one or more actuators configured to apply pressure to one or more fluid packages, wherein the one or more fluid packages are configured to open when pressure is applied to the fluid package or one or more slider structures, wherein one or more of the actuators are coupled to one or more of the slider structures, and wherein one or more of the slider structures are configured to position one or more of the actuators proximate to one or more of the fluid packages.

289. The system of emb. 286, wherein the fluid delivery system comprises one or more gas packages configured to release gas that transports fluid through at least a portion of the cartridge.

290. The system of emb. 284, wherein one or more channels couple the collection region to the detection region.

291. The system of emb. 284, further comprising one or more detectors optically coupled to the detection region, wherein one or more of the detectors are configured to view at least a portion of the detection region.

292. The system of emb. 284, wherein the detection region is configured to allow imaging of at least a portion of a sample in the det. region, to allow detection of a signal produced by at least a portion of a sample in the detection region, or to allow detection of a signal produced by an interaction of at least a portion of a sample with a component of the det. region.

293. The system of emb. 284, wherein the detection region is configured to allow detection of absorption of one or more wavelengths of light by a sample in the detection region or of fluorescence from a sample in the detection region.

294. The system of emb. 284, wherein the det. region comprises a microsieve- or particle-based det. system or comprises a microsieve coupled to or at least partially positioned in or on the cartridge and one or more particles in one or more cavities in or on the cartridge.

295. The system of emb. 284, wherein the collection region comprises one or more absorbent pads or one or more reservoirs.

296. A method comprising applying a sample on or to a collection region coupled to, at least partially positioned in, or at least partially positioned on a cartridge; transporting the sample through one or more channels in or on the cartridge, wherein the one or more channels are coupled to the coll. region; and directing a portion of the sample to an overflow reservoir coupled to, at least partially positioned in, or at least partially positioned on the cartridge; transporting the sample to a det. region coupled to or at least partially positioned in or on the cartridge; obtaining an image of at least a portion of the sample in or on the detection region of the cartridge.

297. The method of emb. 296, further comprising releasing fluids from a fluid delivery system coupled to one or more of the channels to transport at least a portion of the sample through one or more channels.

298. The method of emb. 297, wherein the fluid delivery system comprises one or more fluid packages, and wherein releasing fluids from the fluid delivery system comprises applying pressure to one or more of the fluid packages to cause release of fluid from the fluid package.

299. The method of emb. 298, wherein pressure is applied using an actuator.

300. The method of emb. 296, further comprising transporting at least a portion of the sample through the detection region to a waste reservoir coupled to or at least partially positioned in or on the cartridge or capturing one or more of the images of a portion of the detection region with one or more detectors optically coupled to the cartridge.

301. The method of emb. 296, wherein the detection region comprises a microsieve-based detection system.

302. The method of emb. 301, further comprising washing a surface of a microsieve of the microsieve-based detection system.

303. The method of emb. 296, wherein the detection region comprises a particle-based detection system.

304. The method of emb. 296, wherein the detection region comprises a microsieve-based detection system and a particle-based detection system, the method further comprising:

transporting at least a portion of the sample to a microsieve of the microsieve-based detection system, wherein the microsieve at least partially retains a portion of the sample; and transporting at least a portion of the non-retained sample to the particle-based det. system.

305. The method of emb. 296, wherein the sample comprises blood.

306. A system comprising a cartridge comprising:

a body comprising one or more openings configured to receive one or more components used to facilitate analyte detection; one or more channels coupling one or more of the openings together; a collection region configured to be placed at least partially in one or more of the openings, wherein the collection region is configured to receive a sample; and

one or more detection systems configured to be placed at least partially in one or more of the openings, wherein one or more of the detection regions are configured to interact with at least a portion of a sample deposited in the collection region to allow detection of an analyte, wherein at least one opening is configured to receive a fluid delivery system, wherein the fluid delivery system is configured to release fluids into one or more of the channels during use.

307. The system of emb. 306, further comprising a fluid delivery system configured to be at least partially positioned in one or more of the openings, wherein the fluid delivery system is configured to release fluids into one or more of the channels during use.

308. The system of emb. 307, wherein the fluid delivery system comprises one or more fluid packages.

309. The system of emb. 308, further comprising one or more actuators configured to apply pressure to one or more of the fluid packages, wherein the one or more of the fluid packages are configured to open when pressure is applied to the fluid package.

310. The system of emb. 310, further comprising one or more slider structures, wherein one or more of the actuators are coupled to one or more of the slider structures, and wherein one or more of the slider structures are configured to position one or more of the actuators proximate to one or more of the fluid packages.

The system of emb. 310, wherein the fluid delivery system comprises one or more gas packages configured to release gas that transports fluid through one or more of the channels.

311. The system of emb. 306, wherein one or more of the detection systems is a particle-based detection system.

312. The system of emb. 306, wherein one or more of the detection systems is:

a microsieve, wherein the microsieve is coupled to, at least partially positioned in, or at least partially positioned on the cartridge; and

one or more particles in one or more cavities in or on the cartridge.

313. The system of emb. 306, wherein the collection region comprises one or more absorbent pads or comprises one or more reservoirs.

314. A system comprising a cartridge comprising:

a body comprising one or more openings configured to receive one or more components used to facilitate analyte detection;

one or more channels coupling one or more of the openings together; and

a fluid delivery system configured to be positioned in one or more of the openings, wherein the fluid delivery system is configured to release fluids into one or more of the channels during use.

315. A method comprising obtaining a body comprising:

one or more openings configured to receive one or more components used to facilitate analyte detection;

one or more channels coupling one or more of the openings together;

positioning a collection region at least partially in one or more of the openings, wherein the collection region is configured to receive a sample during use; and

positioning one or more det. systems in one or more of the openings, wherein one or more of the det. regions are configured to interact with at least a portion of a sample deposited in the coll. region to allow detection of an analyte during use.

316. The method of emb. 315, wherein the collection region comprises an absorbent pad.

317. The method of emb. 315, wherein the collection region comprises a reservoir.

318. The method of emb. 315, wherein at least one of the detection systems comprises a microsieve-based detection system or a particle-based detection system.

319. The method of emb. 315, wherein at least one of the detection systems comprises:

a microsieve, wherein the microsieve is coupled to, at least partially positioned in, or at least partially positioned on the body; and

one or more particles in one or more cavities in or on the body.

320. The method of emb. 315, further comprising positioning a fluid delivery system at least partially in one or more of the openings in a body, wherein the fluid delivery system is configured to release fluids into one or more of the channels during use.

321. A system comprising a cartridge comprising:

a collection region;

a detection region configured to interact with at least a portion of a sample deposited in the collection region to allow detection of an analyte;

a cartridge control system coupled to the detection region, wherein the cartridge control system comprises one or more control analytes, wherein the detection region is further configured to interact with at least a portion of the control analytes to allow detection of the control analyte; and

wherein the collection region, the detection region, and the cartridge control system are coupled to or at least partially positioned in or on the cartridge, wherein the detection region comprises:

one or more analyte detection systems, wherein one or more of the analyte detection systems are configured to interact with at least a portion of a sample deposited in the collection region to allow detection of an analyte; and

one or more control detection systems, wherein one or more of the control detection systems are configured to interact with one or more of the control analytes to allow detection of one or more of the control analytes.

322. The system of emb. 321, wherein the control analyte is configured to produce a result that is within a selected range when analyzed in or by the detection region or that is substantially similar to an expected result from a selected analyte when analyzed in or by the detection region.

323. The system of emb. 321, further comprising a fluid delivery system configured to transfer fluids through at least a portion of the cartridge during use.

324. The system of emb. 323, wherein the fluid delivery system is configured to facilitate transport of at least a portion of a sample from the collection region to the detection region during use or of one or more of the control analytes from one or more of the cartridge control systems to the detection region during use.

325. The system of emb. 321, further comprising one or more channels coupling the collection region to the detection region or coupling the cartridge control system to the detection region or one or more detectors optically coupled to the detection region, wherein the detector is configured to view at least a portion of the detection region.

326. The system of emb. 321, wherein the detection region is configured to allow imaging of at least a portion of a sample or a control analyte in the detection region or detection of a signal produced by at least a portion of a sample or a control analyte in the detection region.

327. The system of emb. 321, wherein the detection region is configured to allow detection of a signal produced by an interaction of at least a portion of a sample or a control analyte with a component of the detection region or of absorption of one or more wavelengths of light by a sample or a control analyte in the detection region.

328. The system of emb. 321, wherein the detection region is configured to allow detection of fluorescence from a sample or a control analyte in the detection region, comprises a particle-based detection system, or comprises one or more absorbent pads.

329. The system of emb. 321, wherein the coll. region comprises one or more reservoirs.

330. A method of detecting analytes in a fluid comprising transporting one or more control analytes to a det. region, wherein the detection region is coupled to, at least partially positioned in, or at least partially positioned on a cartridge; assessing whether the cartridge is functioning within a selected range by observing a signal produced by the detection region; and applying a sample on or to a coll. region coupled to, at least partially positioned in, or at least partially positioned on the cartridge if the cartridge is determined to be working properly.

331. The method of emb. 330, further comprising obtaining one or more images of at least a portion of the detection region after one or more of the control analytes reach the detection region; removing at least a portion of one or more of the control analytes from the detection region; or transporting at least a portion of the sample from the collection region to the detection region and optionally obtaining one or more images of at least a portion of the detection region after at least a portion of the sample enters the detection region.

332. The method of emb. 330, wherein one or more of the control analytes are transported to a control detection system in the detection region.

333. The method of emb. 332, further comprising providing one or more images of at least a portion of the control detection system after one or more of the control analytes reach the control detection system.

334. A system comprising a collection region;
a detection system comprising a microsieve and an anti-reflective material optically coupled to the microsieve, wherein the detection system is configured to interact with at least a portion of a sample deposited in the collection region to allow detection of an analyte, and wherein the anti-reflective material is configured to inhibit reflection of light applied to the sample on the microsieve such that an image of at least a portion of the sample in or on the microsieve is improved with respect to an image taken of the sample in the absence of the anti-reflective material, wherein the collection region and the detection system are coupled to, at least partially positioned in, or at least partially positioned on a cartridge.

335. The system of emb. 334, further comprising a fluid deliv. system coupled to the coll. region and the det. system, wherein the fluid delivery system is configured to facilitate transport of at least a portion of the sample from the coll. region to the detection system.

336. The system of emb. 335, wherein the fluid deliv. system comprises one or more fluid packages.

337. The system of emb. 336, wherein at least one of the fluid packages is configured to open when pressure is applied to the fluid package.

338. The system of emb. 337, further comprising one or more actuators configured to apply pressure to one or more of the fluid packages to open the fluid package.

339. The system of emb. 338, further comprising one or more slider structures, wherein one or more of the actuators are coupled to one or more of the slider structures, and wherein one or more of the slider structures are configured to position one or more of the actuators proximate to one or more of the fluid packages.

340. The system of emb. 335, wherein the fluid deliv. system comprises one or more gas packages configured to release gas that transports fluid through at least a portion of the cartridge.

341. The system of emb. 334, wherein one or more channels couple the collection region to the detection system.

342. The system of emb. 334, further comprising one or more detectors optically coupled to the detect. system, wherein one or more of the detectors are configured to view at least a portion of the detection system.

343. The system of emb. 334, wherein the detection system is configured to allow imaging of at least a portion of the sample in the detection system or detection of a signal produced by at least a portion of the sample in the detection system or of absorption of one or more wavelengths of light by the sample in the detection system.

344. The system of emb. 334, wherein the detection system is configured to allow detection of fluorescence from the sample in the detection system.

345. The system of emb. 334, wherein the collection region comprises one or more absorbent pads or one or more reservoirs.

346. A system comprising a collection region; a detection system comprising a microsieve and a hydrophilic material coupled to the microsieve, wherein the detection system is configured to interact with at least a portion of a sample deposited in the collection region to allow detection of an analyte, and wherein the hydrophilic material is configured to enhance the flow of aqueous fluids through the microsieve, wherein the hydrophilic material attracts fluid flowing through the microsieve.

347. The system of emb. 346, further comprising a support coupled to the microsieve, wherein the support is coated with the hydrophilic material.

348. The system of emb. 346, wherein the collection region and the detection system are coupled to, at least partially positioned in, or at least partially positioned on a cartridge.

349. The system of emb. 348, wherein the fluid delivery system comprises one or more fluid packages.

350. The system of emb. 349, wherein at least one of the fluid packages is configured to open when pressure is applied to the fluid package.

351. The system of emb. 350, further comprising one or more actuators configured to apply pressure to one or more of the fluid packages to open the fluid package.

352. The system of emb. 351, further comprising one or more slider structures, wherein one or more of the actuators are coupled to one or more of the slider structures, and wherein one or more of the slider structures are configured to position one or more of the actuators proximate to one or more of the fluid packages.

353. The system of emb. 348, wherein the fluid delivery system comprises one or more gas packages configured to release gas that transports fluid through at least a portion of the cartridge.

354. The system of emb. 346, wherein one or more channels couple the collection region to the detection system.

355. The system of emb. 346, further comprising one or more detectors optically coupled to the detection system, wherein one or more of the detectors are configured to view at least a portion of the detection system.

356. The system of emb. 346, wherein the detection system is configured to allow imaging of at least a portion of the sample in the detection system or detection of a signal produced by at least a portion of the sample in the detection system or of absorption of one or more wavelengths of light by the sample in the detection system.

357. The system of emb. 346, wherein the detection system is configured to allow detection of fluorescence from the sample in the detection system.

358. The system of emb. 356, wherein the collection region comprises one or more absorbent pads or one or more reservoirs.

359. A method of obtaining a measurement of CD4$^+$ cells comprising applying a fluid sample comprising a mixed population of cells to a microsieve such that at least some of the cells in the fluid sample are retained by the microsieve, wherein at least a portion of the mixed population of cells are white blood cells; applying a first detectable label to the cells retained on the microsieve, wherein the first detectable label is adapted to identify CD4$^+$ cells, wherein the CD4$^+$ cells are detectable at a first wavelength of light; applying one or more additional detectable labels to the cells retained on the microsieve, wherein the additional detectable labels are adapted to identify one or more of T cells, NK cells, B cells, or combinations thereof that are present on the microsieve, such that the cells identified by one or more of the additional detectable labels are detectable at one or more wavelengths of light that are different from the first wavelength of light; obtaining a first image of the retained cells at the first wavelength of light; obtaining a second image of the retained cells at one or more wavelengths of light that are different from the first wavelength of light; and obtaining a measurement of CD4$^+$ cells, wherein obtaining a first image comprises capturing a first image with a detector, and wherein obtaining a second image comprises capturing a second image with a detector.

360. The method of emb. 359, wherein the first fluorophore and the second fluorophore are the same fluorophore.

361. A method of obtaining a ratio of CD4$^+$ cells relative to other cells in a sample comprising applying a fluid sample containing a mixed population of cells to a microsieve such that at least the microsieve retains some of the cells in the fluid sample, wherein the mixed population of cells comprises white blood cells; applying a first detectable label to the matter retained on the microsieve, wherein the first detectable label identifies CD4$^+$ cells retained on the microsieve such that the CD4$^+$ cells are detectable at a first wavelength of light; applying one or more additional detectable labels to the matter retained on the microsieve, wherein the additional detectable labels identify any T cells, NK cells, B cells, or combinations thereof, that are present on the microsieve, such that the cells identified by one or more of the additional detectable labels are detectable at one or more wavelengths of light that are different from the first wavelength of light; obtaining a first image of the retained cells at the first wavelength of light; obtaining one or more images of the retained cells at one or more wavelengths of light that are different from the first wavelength of light; and assessing a ratio of CD4$^+$ cells in the sample by comparing the number of labeled cells that are depicted in both the first image and one or more of the second images, to the number of labeled cells that are depicted in one or more of the second images.

362. The method of emb. 361 wherein obtaining a first image comprises capturing a first image with a detector, and wherein obtaining a second image comprises capturing a second image with a detector.

363. The method of emb. 361 wherein one or more of the detectable labels comprise antibodies coupled to a fluorophore.

364. The method of emb. 361 wherein the first detectable label comprises anti-CD4 antibodies coupled to a fluorophore, wherein at least one of the additional detectable labels comprises anti-CD2 antibodies coupled to a fluorophore, wherein at least one of the additional detectable labels comprises anti-CD3 antibodies coupled to a fluorophore, wherein at least one of the additional detectable labels comprises anti-CD56 antibodies coupled to a fluorophore, wherein at least one of the additional detectable labels comprises anti-CD19 antibodies coupled to a fluorophore, or wherein the additional detectable labels comprise anti-CD3 antibodies coupled to a first fluorophore; anti-CD56 antibodies coupled to a second fluorophore; and anti-CD19 antibodies coupled to a third fluorophore, wherein the first fluorophore, the second fluorophore, and the third fluorophore are optionally the same fluorophore.

365. The method of emb. 361 wherein the additional detectable labels comprise anti-CD2 antibodies coupled to a first fluorophore; and anti-CD19 antibodies coupled to a second fluorophore, in particular wherein the first fluorophore and the second fluorophore are the same fluorophore.

366. The method of emb. 361, further comprising transporting at least a portion of the cells retained on the microsieve to a detection system comprising at least one particle in a cavity, wherein the particle interacts with at least a portion of the sample.

367. The method of emb. 366, wherein the particle interacts with at least a portion of the matter to allow detection of an analyte.

368. A method of counting CD4$^+$ cells comprising applying a fluid sample to a microsieve such that at least the microsieve retains some matter in the fluid sample, wherein the fluid sample comprises white blood cells; obtaining a first image of the matter retained on the microsieve; applying one or more detectable labels to the matter retained on the microsieve, wherein the detectable labels label at least a portion of the matter retained on the microsieve, but does not identify CD4$^+$ cells, and wherein the labeled matter is detectable at one or more wavelengths of light; obtaining a second image of matter retained on the microsieve at one or more wavelengths of light; obtaining a number of CD4$^+$ cells by assessing the number of cells that are depicted in the first image but are not depicted in the second image.

369. The method of emb. 368, wherein the first image comprises an image of all cells retained by the microsieve.

370. The method of emb. 368, wherein the cells depicted in the second image comprises labeled cells.

371. The method of emb. 368, wherein providing a first image comprises capturing the first image with a detector, and wherein providing a second image comprises capturing the second image with a detector.

372. The method of emb. 368, wherein at least one detectable label is adapted to label white blood cells other than CD4$^+$ cells.

373. The method of emb. 368, wherein at least one detectable label is adapted to label cells retained other than white blood cells and CD4$^+$ cells.

374. The method of emb. 368, further comprising providing a third image of cells retained at one or more additional wavelengths of light; and assessing a number of lymphocytes by assessing the sum of a number of cells that are in the third image and a number of cells that are in the first image and not the second image.

375. The method of emb. 374, further comprising assessing a ratio of CD4$^+$ cells to total lymphocytes.

376. A system comprising a cartridge comprising:

> a collection region; and
> a detection region configured to interact with at least a portion of a sample deposited in the collection region to allow detection of an analyte;

wherein the collection region and the detection region are coupled to, at least partially positioned in, or at least partially positioned on a body of the cartridge, the system further comprising a fluid delivery system configured to facilitate transport of fluid through at least a portion of the cartridge during use.

377. The system of emb. 376, further comprising a fluid delivery system coupled to the coll. region and the detection region such that fluid from the fluid delivery system facilitates transport of at least a portion of the sample from the collection region to the detection region during use.

378. The system of emb. 377, wherein the fluid delivery system comprises one or more fluid packages or one or more fluid packages configured to open by application of pressureor to be opened by application of heat.

379. The system of emb. 387, wherein the fluid delivery system comprises one or more fluid packages configured to be opened by an electromagnetic signal or by application of pressure, the system further comprising one or more actuators configured to apply pressure to one or more fluid packages of the fluid delivery system during use.

380. The system of emb. 379, further comprising one or more slider structures, wherein one or more of the actuators are coupled to one or more of the slider structures, and wherein one or more of the slider structures are configured to position one or more of the actuators proximate to one or more of the fluid packages during use.

381. The system of emb. 377, wherein the fluid delivery system comprises one or more gas packages configured to release gas to transport the fluid through the cartridge, one or more fluid packages, wherein the fluid package comprises a wall configured to rupture upon application of pressure, or one or more syringes coupled to or at least partially positioned in or on the cartridge.

382. The system of emb. 377, wherein the fluid delivery system comprises one or more fluid bulbs coupled to or at least partially positioned in or on the cartridge or one or more fluid packages and one or more channels, wherein one or more of the fluid packages are configured to create a partial vacuum, when opened, in one or more of the channels during use

383. The system of emb. 377, further comprising one or more channels, wherein one or more of the channels couple the fluid deliv. system to the coll. region or one or more channels, wherein one or more of the channels couple the fluid delivery system to the det. region.

384. The system of emb. 377, wherein the fluid delivery system comprises one or more channels and one or more valves positioned in channels, wherein one or more of the valves are capable of controlling fluid or gas flow through the cartridge.

385. The system of emb. 384, wherein one or more of the valves are pinch valves.

386. The system of emb. 377, wherein the fluid deliv. system comprises one or more channels and one or more vents coupled to one or more of the channels, wherein one or more of the vents are configured to allow gas to escape from one or more of the channels when fluids pass through the channel or is coupled to one or more reagent regions coupled to, at least partially positioned in, or at least partially positioned on the cartridge, wherein one or more of the reagent regions are configured to at least partially release one or more reagents when contacted with a fluid from the fluid delivery system during use.

387. The system of emb. 386, wherein one or more of the reagent regions comprises one or more reagents that are reconstituted upon contact with a fluid.

388. The system of emb. 386, wherein one or more of the reagent regions comprise one or more reagents entrained in a dissolvable material, wherein a fluid passing over one or more of the reagent pads dissolves at least a portion of the dissolvable material, releasing at least a portion of the entrained reagent into the fluid.

389. The system of emb. 377, wherein the fluid delivery system comprises one or more channels, and wherein a portion of one or more of the channels is coated with one or more reagents, and wherein one or more of the reagents within the channel are added to fluid passing through the channel during use.

390. The system of emb. 376, further comprising one or more channels, wherein one or more of the channels couple the collection region to the detection region, one or more channels, wherein at least a portion of one or more of the channels comprise a hydrophilic coating, or one or more channels, wherein at least a portion of one or more of the channels comprise a hydrophobic coating.

391. The system of emb. 376, wherein the collection region comprises a metered volume portion configured to deliver a metered volume sample to the detection region.

392. The system of emb. 376, further comprising one or more reservoirs coupled to, at least partially positioned in, or at least partially positioned on the cartridge, wherein one or more of the reservoirs are configured to collect fluids during use.

393. The system of emb. 392, wherein one or more of the reservoirs are removable from the cartridge.

394. The system of emb. 376, further comprising one or more detectors optically coupled to the detection region, wherein one or more of the detectors are configured to view at least a portion of the detection region.

395. The system of emb. 376, wherein the detection region is configured to allow imaging of at least a portion of the sample in the detection region or detection of a signal produced by at least a portion of the sample in the detection region or by an interaction of at least a portion of the sample with one or more components of the detection region.

396. The system of emb. 376, wherein the detection region is configured to allow detection of absorption of one or more wavelengths of light by the sample in the detection region or of fluorescence from the sample in the detection region.

397. The system of emb. 376, wherein the det. region comprises a particle-based det. system.

398. The system of emb. 376, wherein the body comprises two or more layers coupled together.

399. The system of emb. 376, wherein the collection region comprises one or more absorbent pads or one or more reservoirs.

400. The system of emb. 376, wherein the collection region comprises a portion of a channel that is configured to hold a predetermined amount of the sample.

401. The system of emb. 376, further comprising a cover removably positionable over the collection region, one or more cartridge control systems comprising one or more control analytes, wherein one or more of the cartridge control systems are coupled to the detection region, or one or more light sources.

402. The system of emb. 376, wherein the body is substantially planar.

403. The system of emb. 376, wherein the body has a height of at most 30 mm, a width of at most 100 mm, or a length of at most 300 mm.

404. The system of emb. 376, wherein the cartridge is capable of being held by a human hand or is portable.

405. A method of analyzing a blood sample, comprising introducing the blood sample into an analyte det. apparatus, the analyte det. apparatus comprising a microsieve-based det. system and a particle-based det. system; passing the blood sample to the microsieve-based det. system, wherein at least some of the cell. components in the blood sample are collected by a microsieve of the microsieve-based det. system; passing the blood sample to the particle-based det. system, wherein the particle-based det. system is configured to interact with one or more proteins in the blood sample; assessing the amount and/or identity of at least a portion of the cell. components collected by the microsieve; and assessing the amount and/or identity of proteins that interact with the particle-based det. system.

406. The method of emb. 405, wherein one or more of the proteins include one or more protein cardiac biomarkers.

407. The method of emb. 405, wherein the microsieve-based detection system and the particle-based detection system are coupled to, at least partially positioned in, or at least partially positioned on a cartridge.

408. The apparatus of emb. 407, wherein one or more analytes in the blood sample comprise cellular components of the blood sample, one or more cellular cardiac biomarkers, one or more proteins, one or more nucleic acids, or one or more protein cardiac biomarkers.

409. The apparatus of emb. 407, wherein the microsieve-based detection system and the particle-based detection system are coupled to, at least partially positioned in, or at least partially positioned on a cartridge or wherein the microsieve-based detection system is coupled to the particle-based detection system.

410. A system, comprising a cartridge comprising:

a microsieve-based det. system configured to interact with at least a portion of one or more analytes in a sample to allow detection of one or more analytes, wherein the microsieve-based det. system comprises a microsieve, and wherein at least a portion the sample is retained by the microsieve as the sample passes through the microsieve; and
a particle-based det. system comprising one or more particles, wherein at least a portion of the particles are configured to interact with one or more of the analytes in the sample.

411. A system comprising a cartridge comprising:

a collection region;
a detection region configured to interact with at least a portion of a sample deposited in the collection region to allow detection of an analyte;
one or more channels configured to allow fluid to flow through at least a portion of the cartridge; and
a fluid delivery system comprising one or more fluid packages, wherein at least one of the fluid packages is configured to create a partial vacuum, when opened, in one or
more of the channels, and

wherein the collection region, the detection region, and the fluid delivery system are coupled to or at least partially positioned in or on the cartridge;
wherein any one of the following features applies:

the detection region comprises a microsieve-based detection system and
a microsieve coupled to or at least partially positioned in or on the cartridge and one or more cavities comprising one or more particles.

412. A system comprising a cartridge comprising:

a collection region;
a detection region configured to interact with at least a portion of a sample deposited in the collection region to allow detection of an analyte; and
one or more viewing windows optically coupled to the detection region, wherein one or more of the viewing windows are configured to allow at least a portion of the

detection region to be viewed by one or more detectors,

wherein the detection region comprises any one or more of the following:

a microsieve-based detection system and
a microsieve coupled to or at least partially positioned in or on the cartridge and one or more cavities comprising one or more particles.

413. A system comprising a cartridge comprising:

a collection region;
a detection region configured to interact with at least a portion of a sample deposited in the collection region to allow detection of an analyte; and
a fluid delivery system comprising one or more syringes configured to facilitate
transport of fluid through at least a portion of the cartridge;

wherein the collection region, the detection region, and the fluid delivery system are coupled to or at least partially positioned in or on the cartridge, and
wherein the detection region comprises any one of the following:

a microsieve-based detection system and
a microsieve coupled to or at least partially positioned in or on the cartridge and one or more particles in one or more cavities in or on the cartridge.

414. A system comprising a cartridge comprising:

a collection region;
a detection region configured to interact with at least a portion of a sample deposited in the collection region to allow detection of an analyte; and
one or more channels configured to allow fluid to flow through at least a portion of the cartridge, wherein a surface of, or a coating on, at least a portion of one or more of the channels is configured to selectively direct fluids flowing through one or more of
the channels during use;

wherein the collection region and the detection region are coupled to or at least partially positioned in or on the cartridge; and
wherein the detection region comprises any one of the following:

a microsieve-based detection system; and
a microsieve coupled to or at least partially positioned in or on the cartridge and one or more particles in one or more cavities in or on the cartridge.

415. A system comprising a cartridge comprising:

a collection region;
a detection region configured to interact with at least a portion of a sample deposited in the collection region to allow detection of an analyte;
one or more channels configured to allow fluid to flow through at least a portion of the cartridge; and
a body comprising a plurality of layers coupled together, wherein the collection region and the detection region are coupled to or at least partially positioned in or on the body; and

wherein the detection region comprises any one or more of the following:

a microsieve-based detection system and
a microsieve coupled to or at least partially positioned in or on the body and one or more particles in one or more cavities in or on the body.

416. A system comprising a cartridge comprising:

a collection region;

a detection region configured to interact with at least a portion of a sample deposited in the collection region to allow detection of an analyte;

one or more reagent regions coupled to the detection region, wherein one or more of the reagent regions are configured such that one or more reagents from one or more of the reagent regions are added to a fluid flowing through one or more reagent regions

during use;

wherein the collection region, the detection region and one or more of the reagent regions are coupled to, at least partially positioned in or on the cartridge; and

wherein the detection region comprises any one of the following:

a microsieve-based detection system and

a microsieve coupled to or at least partially positioned in or on the cartridge and one or more particles in one or more cavities in or on the cartridge.

417. A system comprising a cartridge comprising:

a body comprising one or more openings configured to receive one or more components used to facilitate analyte detection;

one or more channels coupling one or more of the openings together;

a collection region configured to be placed at least partially in one or more of the openings, wherein the collection region is configured to receive a sample; and

one or more detection systems configured to be placed at least partially in one or more of the openings, wherein one or more of the detection regions are configured to interact with at least a portion of the sample deposited in the collection region to

allow detection of an analyte; and

wherein one or more of the detection systems is any one or more of the following:

a microsieve-based detection system; and

a microsieve coupled to or at least partially positioned in or on the cartridge and one or more particles in one or more cavities in or on the cartridge.

418. A system comprising a cartridge comprising:

a collection region; and

a detection region configured to interact with at least a portion of a sample deposited in the collection region to allow detection of an analyte;

wherein the collection region and the detection region are coupled to or at least partially positioned in or on a body of the cartridge; and

wherein the detection region comprises any one of the following:

a microsieve-based detection system, in particular a microsieve-based detection system comprising a microsieve and a support configured to inhibit sagging of the microsieve, wherein the support is preferably composed of, or coated with, a hydrophilic material or an anti-reflective material, or is configured to allow a predetermined flow rate of fluid through the microsieve;

a microsieve coupled to or at least partially positioned in or on the cartridge and one or more particles in one or more cavities in or on the cartridge.

**Claims**

1. A method of obtaining a measurement of CD4$^+$ cells, the method comprising the following steps:

(a) applying a fluid sample comprising a mixed population of cells to a microsieve such that at least some of the cells in the fluid sample are retained by the microsieve, wherein at least a portion of the mixed population of cells are white blood cells;

(b) applying a first detectable label to the cells retained on the microsieve, wherein the first detectable label is adapted to identify CD4$^+$ cells, wherein the CD4$^+$ cells are detectable at a first wavelength of light;

(c) applying one or more additional detectable labels to the cells retained on the microsieve, wherein the additional detectable labels are adapted to identify one or more of T cells, NK cells, B cells, or combinations thereof that are present on the microsieve, such that the cells identified by one or more of the additional detectable labels are detectable at one or more wavelengths of light that are different from the first wavelength of light;

(d) obtaining a first image of the retained cells at the first wavelength of light;

(e) obtaining a second image of the retained cells at one or more wavelengths of light that are different from the first wavelength of light; and

(f) obtaining a measurement of CD4$^+$ cells.

2. The method of claim 1, wherein any one of the following requirements are met:

- one or more of the detectable labels comprise antibodies coupled to a fluorophore,
- the first detectable label comprises anti-CD4 antibodies coupled to a fluorophore, and
- at least one of the additional detectable labels comprises (i) anti-CD2 antibodies, (ii) anti-CD3 antibodies, (iii) anti-CD56 antibodies, or (iv) anti-CD 19 antibodies coupled to a fluorophore.

3. The method of claim 1, wherein any one of the following requirements are met:

- the additional detectable labels comprise: anti-CD3 antibodies coupled to a first fluorophore; anti-CD56 antibodies coupled to a second fluorophore; and anti-CD19 antibodies coupled to a third fluorophore, the first, the second, and the third fluorophore in particular being the same fluorophore, and
- the additional detectable labels comprise (i) anti-CD2 antibodies coupled to a first fluorophore and (ii) anti-CD 19 antibodies coupled to a second fluorophore, in such case the method preferably further comprising the step of transporting at least a portion of the matter retained on the microsieve to a detection system comprising at least one particle in a cavity, wherein the particle interacts with at least a portion of the sample to allow detection of an analyte.

4. A method of detecting analytes in a fluid, the method comprising the steps of:

(a) applying a sample on or to a collection region coupled to or at least partially positioned in or on a cartridge;

(b) transporting fluid from a fluid delivery system through one or more channels to facilitate transport of at least a portion of the sample from the collection region to a detection region, wherein the fluid delivery system and the detection region are coupled to or at least partially positioned in or on the cartridge;

(c) applying pressure to one or more pinch valves coupled to one or more of the channels to control fluid flow through one or more of the channels; and

(d) obtaining one or more images of a detection region after the sample reaches the detection region.

5. A system comprising:

a cartridge comprising:

a collection region;
a detection region configured to interact with at least a portion of a sample deposited in the collection region to allow detection of an analyte;
a fluid delivery system comprising one or more channels, wherein the fluid delivery system is configured to facilitate transport of fluid through at least a portion of the cartridge; and
one or more pinch valves coupled to one or more of the channels;

wherein the collection region, the detection region, and the fluid delivery system are coupled to or at least partially positioned in or on the cartridge, and
wherein the detection region comprises a microsieve-based detection system.

6. A system comprising:

a cartridge comprising:

a collection region;
a detection region configured to interact with at least a portion of a sample deposited in the collection region to allow detection of an analyte; and
one or more channels configured to allow fluid to flow through at least a portion of the cartridge, wherein a shape or an elevation of at least a portion of one or more of the channels is configured to selectively direct fluids flowing in or through one or
more of the channels;

wherein the collection region and the detection region are coupled to or at least partially positioned in or on the cartridge.

7. A system comprising:

a cartridge comprising:

a collection region;
a cover removably positionable over the collection region; and
a detection region configured to interact with at least a portion of a sample deposited
in the collection region to allow detection of an analyte;

wherein the collection region and the detection region are coupled to or at least partially positioned in or on the cartridge.

8. A system comprising:

a cartridge comprising:

a collection region;
a detection region configured to interact with at least a portion of a sample deposited in the collection region to allow detection of an analyte;
one or more channels configured to allow fluid to flow through at least a portion of the cartridge; and
one or more vents coupled to one or more of the channels, wherein one or more of the vents are configured to allow gas to enter or exit one or more of the channels;

wherein the collection region and the detection region are coupled to or at least partially positioned in or on the cartridge.

9. A system comprising:

a cartridge comprising:

a collection region;
a detection region configured to interact with at least a portion of a sample deposited in the collection region to allow detection of an analyte; and
an overflow reservoir coupled to the collection region to collect excess sample or fluid; and

wherein the collection region, the detection region, and the overflow reservoir are coupled to or at least partially positioned in or on the cartridge.

10. A system comprising:

a cartridge comprising:

a body comprising one or more openings configured to receive one or more components used to facilitate analyte detection; and
one or more channels coupling one or more of the openings together; and
one or more detection systems configured to be at least partially placed in one or
more of the openings,

wherein one or more of the detections systems are configured to interact with at least a portion of a sample to allow detection of an analyte.

11. A system comprising:

a cartridge comprising:

a collection region;
a detection region configured to interact with at least a portion of a sample deposited in the collection region to allow detection of an analyte;
a cartridge control system coupled to the detection region,

wherein the cartridge control system comprises one or more control analytes,
wherein the detection region is further configured to interact with at least a portion of the control analytes to allow detection of the control analyte;
wherein the collection region, the detection region, and the cartridge control system are coupled to or at least partially positioned in or on the cartridge; and
wherein any one of the following two features applies:

- the detection region comprises a microsieve-based detection system;
- the detection region comprises:

a microsieve, wherein the microsieve is coupled to or at least partially positioned in or on the cartridge and one or more particles in one or more cavities in or on the cartridge.

12. An apparatus for analyzing a blood sample, the apparatus comprising:

a microsieve-based detection system comprising a microsieve, wherein one or more analytes in the blood sample are collected by the microsieve as at least a portion of the blood sample passes through the microsieve; and
a particle-based detection system comprising one or more particles, wherein at least a portion of the particles are configured to interact with one or more analytes in the blood sample.

13. A system comprising:

a cartridge comprising:

a microsieve-based detection system coupled to or at least partially positioned in or on the cartridge, the microsieve-based detection system comprising a microsieve, wherein the microsieve is configured to at least partially retain one or more white blood cells in or on the microsieve when one or more blood samples are applied to the microsieve; and
one or more detectable labels contained in or on the cartridge, wherein one or more of the detectable labels are configured to label one or more white blood cells retained in or on the microsieve; and

wherein the system optionally further comprises any one of the following:

one or more light sources configured to direct light toward at least a portion of the microsieve;
one or more detectors configured to collect light emitted and/or reflected from at least a portion of the microsieve;
an optical platform, the optical platform comprising one or more light sources optically coupled to at least a portion of the microsieve and one or more detectors optically coupled to at least a portion of the microsieve, wherein the cartridge is positionable in the optical platform;
a housing, wherein the housing comprises a cartridge positioning system configured to receive and automatically position the cartridge in the housing;
one or more detectors and a viewing window in or on the cartridge, wherein one or more of the detectors are configured to view at least a portion of the microsieve through the viewing window; and
a collection region coupled to the microsieve, wherein the collection region is configured to receive a sample or a fluid; or.

wherein the cartridge further comprises a window optically coupled to the microsieve.

**14.** A system comprising:

a collection region;
a detection system comprising a microsieve and an anti-reflective material optically coupled to the microsieve, wherein the detection system is configured to interact with at least a portion of a sample deposited in the collection region to allow detection of an analyte, and wherein the anti-reflective material is configured to inhibit reflection of light applied to the sample on the microsieve such that an image of at least a portion of the sample in or on the microsieve is improved with respect to an image taken of the sample in the absence of the anti-reflective material; and

wherein the system optionally further comprises any one of the following:

a support coupled to the microsieve, wherein the support is made of;
is coupled to; and
is coated with the anti-reflective material.

**15.** A system comprising:

a collection region;
a detection system comprising a microsieve and a hydrophilic material coupled to the microsieve, wherein the detection system is configured to interact with at least a portion of a sample deposited in the collection region to allow detection of an analyte, and wherein the hydrophilic material is configured to enhance the flow of aqueous fluids through the microsieve; and

wherein optionally any one of the following features applies:

the hydrophilic material is downstream from the microsieve;
the hydrophilic material attracts fluid flowing through the microsieve;
the system further comprises a support coupled to the microsieve, wherein the support (a) is made of,(b) is coupled to, or (c) is coated with the hydrophilic material;
the system further comprises a fluid delivery system coupled to the collection region and the detection system, wherein the fluid delivery system is configured to facilitate transport of at least a portion of the sample from the collection region to the microsieve.

FIG. 1

FIG. 2

FIG. 3

FIG. 4

FIG. 5

FIG. 6

FIG. 7

*FIG. 8*

*FIG. 9*

*FIG. 10*

*FIG. 11*

*FIG. 12*

FIG. 13

FIG. 14

72

FIG. 15

FIG. 16

FIG. 17

FIG. 18

FIG. 19

FIG. 20

FIG. 21

FIG. 22A

FIG. 22B

FIG. 23

FIG. 24

218

*FIG. 25A*

218

*FIG. 25B*

218

*FIG. 25C*

218

*FIG. 25D*

218

*FIG. 25E*

218

*FIG. 25F*

218

*FIG. 25G*

218

*FIG. 25H*

FIG. 26A

FIG. 26B

FIG. 27

FIG. 28

FIG. 29

FIG. 30

FIG. 31

FIG. 32

FIG. 33

FIG. 34

FIG. 35

81

FIG. 36

FIG. 37

FIG. 38

EP 2 508 867 A1

FIG. 39A

FIG. 39B

EP 2 508 867 A1

FIG. 40A

FIG. 40B

FIG. 40C

FIG. 41A

FIG. 41B

FIG. 41C

FIG. 41D

85

**European Patent Office**
Europäisches Patentamt
European Patent Office
Office européen des brevets

## EUROPEAN SEARCH REPORT

Application Number

EP 12 17 3753

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/072278 A1 (CHOU HOU-PU [US] ET AL) 15 April 2004 (2004-04-15) <br> * paragraphs [0352], [0354], [0353] * <br> * paragraphs [0700], [0708] * <br> * paragraphs [0527], [0747], [0774], [0750], [0794] - [0797], [0857] * <br> ----- | 1-15 | INV. <br> G01N15/06 <br> G01N33/00 <br> G01N33/48 <br> G01N35/00 <br> B01L3/00 |
|  |  |  | TECHNICAL FIELDS SEARCHED (IPC) <br><br> B01L |

The present search report has been drawn up for all claims

| Place of search | Date of completion of the search | Examiner |
|---|---|---|
| Munich | 27 August 2012 | Hoyal, Barnaby |

EPO FORM 1503 03.82 (P04C01)

**EP 2 508 867 A1**

**ANNEX TO THE EUROPEAN SEARCH REPORT
ON EUROPEAN PATENT APPLICATION NO.**

EP 12 17 3753

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2012

| Patent document cited in search report | Publication date | Patent family member(s) | Publication date |
|---|---|---|---|
| US 2004072278 A1 | 15-04-2004 | US 2004072278 A1<br>US 2004224380 A1 | 15-04-2004<br>11-11-2004 |

EPO FORM P0459

For more details about this annex : see Official Journal of the European Patent Office, No. 12/82

87

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020197622 A, McDevitt
- US 11022176 B, McDevitt
- US 11020443 B
- US 11020442 B
- US 11022365 B
- US 11021123 B
- US 11022219 B
- US 5096660 A, Lauks
- US 5837199 A, Dumschat
- US 6010463 A, Lauks
- US 6908770 B
- US 6680206 B
- US 6602702 B
- US 6589779 B
- US 6649403 B
- US 6713298 B
- US 20020160363 A
- US 20040029259 A
- US 20030064422 A
- US 20030186228 A
- US 20040053322 A
- US 20050136548 A
- US 20050164320 A
- US 20050214863 A
- US 09616731 B
- US 10522499 B
- US 10470646 B
- US 10522926 B
- US 10544864 B
- US 10544954 B
- US 20020160363 B
- US 20020064422 B
- US 20040053322 B
- US 20030186228 B
- US 20020197622 B
- US 20040029259 B
- US 20050136548 B
- US 20050214863 B
- US 4196265 A, Koprowski
- US 522499 A

**Non-patent literature cited in the description**

- **ROITT ; BROSTOFF ; MALE.** IMMUNOLOGY. Mosby/Times Mirror International Publication, 1998
- IMMUNOBIOLOGY: THE IMMUNE SYSTEM IN HEALTH AND DISEASE. Garland Publishing, Inc, 2001
- **SANCHEZ-RAMON et al.** *Pediatrics,* vol. 111 (2), 168-75
- **E. HOWELL ; D. LANE.** Antibodies: A Laboratory Manual. Cold Spring Harbor Laboratory, 1988